# EUROPEAN PATENT APPLICATION

(11) **EP 4 748 301 A1**
(43) Date of publication of application: **27.05.2026**
(21) Application number: 24884796.4
(22) Date of filing: 30.10.2024
(51) Int. Cl.: A61B 5/02, A61B 5/022, A61B 5/0225

(54) **BLOOD PRESSURE MEASUREMENT METHOD, WEARABLE DEVICE, AND STORAGE MEDIUM**

(30) Priority: 31.10.2023 CN 202311441143
(71) Applicant: HUAWEI TECHNOLOGIES CO., LTD., Shenzhen 518129 (CN)
(72) Inventor: JIA, Zheng, Shenzhen, Guangdong 518129 (CN); WU, Zhouzhen, Shenzhen, Guangdong 518129 (CN); ZHU, Yu, Shenzhen, Guangdong 518129 (CN); LIU, Dengkuan, Shenzhen, Guangdong 518129 (CN)
(74) Representative: Epping - Hermann - Fischer
(86) International application number: PCT/CN2024/128415
(87) International publication number: WO 2025/092794

(57) **Abstract**

A wearable device (100) is provided, including a motion sensor (203G) and a PPG module. A blood pressure monitoring method applied to the wearable device is provided, including: The wearable device (100) obtains motion data collected by the motion sensor (203G) and a first PPG signal collected by the PPG module; when one or both of the motion data and the first PPG signal meet a first condition, the wearable device (100) determines whether a user is in a sleep state; and when determining that the user is in a first sleep event, the wearable device (100) obtains a first blood pressure value. The first sleep event includes any one or more of the following: an OSA event, a REM event, an NREM sleep event, and an elevated central sympathetic nerve activity event. When a specific sleep event is detected, blood pressure starts to be measured, and when no specific sleep event is detected, the blood pressure stops being measured. This can reduce interference to sleep of the user, reduce power consumption of the wearable device (100), and improve user experience.

## Description

This application claims priority to Chinese Patent Application No. 202311441143.1, filed with the China National Intellectual Property Administration on October 31, 2023 and entitled "BLOOD PRESSURE MONITORING METHOD, WEARABLE DEVICE, AND STORAGE MEDIUM", which is incorporated herein by reference in its entirety.

### TECHNICAL FIELD

This application relates to the terminal field, and in particular, to a blood pressure monitoring method, a wearable device, and a storage medium.

### BACKGROUND

With improvement of living standards, people's health attracts increasing attention. Hypertension is a common cardiovascular disease, and regularly measuring blood pressure is one of important means to ensure health of hypertensive patients. Ambulatory blood pressure measurement is a technology of continuously measuring user blood pressure 24 hours, and a plurality of blood pressure measurement values within 24 hours may be obtained. Usually, measurement is performed once every 10 minutes to 15 minutes, and an average value of the plurality of blood pressure measurement values within 24 hours is used as a blood pressure value. Currently, there is a cuff-based blood pressure monitor for measuring ambulatory blood pressure of a user. However, to use the cuff-based blood pressure monitor, the user needs to carry the cuff-based blood pressure monitor 24 hours. It is inconvenient for the user to use the cuff-based blood pressure monitor.

To facilitate blood pressure measurement of the user, a wrist ambulatory blood pressure monitor is provided. The wrist ambulatory blood pressure monitor is comfortable to wear, and saves time and effort. The wrist ambulatory blood pressure monitor may monitor nocturnal blood pressure and daytime blood pressure of the user. Monitoring the nocturnal blood pressure is essential for prevention and intervention in a cardiovascular event. However, the user is in a sleep stage at night. The wrist ambulatory blood pressure monitor periodically measures user blood pressure, which may affect sleep quality of the user. How to reduce impact of nocturnal blood pressure measurement on the sleep quality of the user is to be further studied.

### SUMMARY

This application provides a blood pressure monitoring method, a wearable device, and a storage medium. The wearable device may enable a blood pressure monitoring function when detecting a specific sleep event at night, to reduce impact on sleep quality of a user when the wearable device measures blood pressure at night, thereby improving user experience.

According to a first aspect, this application provides a blood pressure monitoring method. The method is applied to a wearable device, the wearable device includes a motion sensor and a photoplethysmography PPG module, and the method includes: The wearable device obtains motion data collected by the motion sensor and a first PPG signal collected by the PPG module; when one or both of the motion data and the first PPG signal meet a first condition, the wearable device determines whether a user is in a sleep state; and when determining that the user is in a first sleep event, the wearable device measures user blood pressure, and obtains a first blood pressure value. The first sleep event includes any one or more of the following: an OSA event, a REM event, an NREM sleep event, and an elevated central sympathetic nerve activity event.

Optionally, the wearable device may determine the first sleep event based on the first PPG signal. When determining that the user is in the sleep state and determining that the user is in the first sleep event, the wearable device measures the user blood pressure, and obtains the first blood pressure value.

In some embodiments, the wearable device may determine, based on physiological data such as blood oxygen, a heart rate, and a respiratory rate collected by the PPG module, whether the OSA event occurs.

In some embodiments, the wearable device may determine, based on physiological data such as a heart rate and a respiratory rate collected by the PPG module, whether the user is in the REM sleep event.

In some embodiments, the wearable device may determine, based on physiological data such as a heart rate and a respiratory rate collected by the PPG module, whether the user is in the NREM sleep event.

In some embodiments, the elevated central sympathetic nerve activity event is related to a quantity of nocturnal awakenings, NREM sleep duration, and a REM sleep latency. The wearable device may monitor a quantity of awakenings of the user, NREM sleep event duration, a REM sleep event latency, and the like. When the quantity of awakenings of the user is greater than a preset quantity of times, the NREM sleep event duration is less than a first value, and duration of the REM sleep event latency is less than a second value, a central sympathetic nerve activity of the user at night is enhanced.

In some embodiments, whether the elevated central sympathetic nerve activity event occurs may be determined based on physiological data such as blood oxygen, a heart rate, and a respiratory rate collected by the PPG module preconfigured in the wearable device.

When the blood oxygen, the heart rate, and the respiratory rate meet a second condition, the wearable device determines that the OSA event occurs. When the heart rate and the respiratory rate meet a third condition, the wearable device determines that the REM event occurs. When the heart rate and the respiratory rate meet a fourth condition, the wearable device determines that the NREM event occurs. When the blood oxygen, the heart rate, and the respiratory rate meet a fifth condition, the wearable device determines that the elevated central sympathetic nerve activity event occurs. The second condition, the third condition, and the fourth condition are different from each other.

In some embodiments, the PPG module may alternatively be replaced with another component. In this application, only the PPG module is used as an example for description. This is not limited in this application.

In some embodiments, the motion sensor may alternatively be replaced with another component. In this application, only the motion sensor is used as an example for description. This is not limited in this application.

In some embodiments, in addition to the PPG module and the motion sensor, the wearable device may further determine, by collecting an ambient sound and a speaking sound of a person by a microphone, whether the user is asleep. Usually, a surrounding environment is quiet, and the user hardly speaks after falling asleep. When intensity of the ambient sound is less than preset intensity and/or the speaking sound of the person is less than the preset intensity, it may be determined that the user is in a sleep state.

In some embodiments, the wearable device may further determine, based on ambient luminance collected by an optical sensor, whether the user is asleep. Usually, ambient light when the user falls asleep is dark. When the ambient luminance collected by the optical sensor is less than preset luminance, it may be determined that the user is in the sleep state.

In some embodiments, the wearable device may further determine, based on a signal such as an EMG electromyography signal, an EEG electroencephalography signal, or a GSR galvanic skin response signal collected by an electrode, whether the user is asleep. When the EMG electromyography signal, the EEG electroencephalography signal, and the GSR galvanic skin response signal meet a preset condition, it may be determined that the user is in the sleep state. The foregoing one or more manners of determining whether the user is asleep may be used independently to determine whether the user is asleep, or two or more manners may be used together to determine whether the user is asleep. This is not limited in this application. According to the method, when a specific sleep event is detected, blood pressure starts to be measured, and when no specific sleep event is detected, the blood pressure stops being measured. This can reduce interference to sleep of the user, reduce power consumption of the wearable device, and improve user experience.

With reference to the first aspect, in a possible implementation, the OSA event, the REM event, and the elevated central sympathetic nerve activity event are sleep events that cause risen user blood pressure; and when the first sleep event includes any one or more of the OSA event, the REM event, and the elevated central sympathetic nerve activity event, before the wearable device obtains the motion data collected by the motion sensor and the first PPG signal collected by the PPG module, the method further includes: The wearable device receives a first user operation, where the first user operation indicates the wearable device to measure the user blood pressure when detecting the first sleep event; and when the user is in the sleep state and it is determined, based on the first PPG signal, that the user is in the first sleep event, that the wearable device measures the user blood pressure, and obtains the first blood pressure value specifically includes: in response to the first operation, when the user is in the sleep state and it is determined, based on the first PPG signal, that the user is in the first sleep event, the wearable device measures the user blood pressure, and obtains the first blood pressure value.

In this way, when the user learns that the user is a hypertensive user, the user may actively set that the wearable device automatically starts to measure the user blood pressure when detecting a sleep event that causes risen user blood pressure. In this way, the wearable device can be actively triggered to monitor blood pressure of the hypertensive user, and interference to sleep of the user can be reduced.

With reference to the first aspect, in a possible implementation, the NREM sleep event is a sleep event that causes dropped user blood pressure; and when the first sleep event includes the NREM sleep event, before the wearable device obtains the motion data collected by the motion sensor and the first PPG signal collected by the PPG module, the method further includes: The wearable device receives a second user operation, where the second user operation indicates the wearable device to measure the user blood pressure when detecting the first sleep event; and when the user is in the sleep state and it is determined, based on the first PPG signal, that the user is in the first sleep event, that the wearable device measures the user blood pressure, and obtains the first blood pressure value specifically includes: in response to the second operation, when the user is in the sleep state and it is determined, based on the first PPG signal, that the user is in the first sleep event, the wearable device measures the user blood pressure, and obtains the first blood pressure value.

In this way, when the user learns that the user is a hypotensive user, the user may actively set that the wearable device automatically starts to measure the user blood pressure when detecting a sleep event that causes dropped user blood pressure. In this way, the wearable device can be actively triggered to monitor blood pressure of the hypotensive user, and interference to sleep of the user can be reduced.

With reference to the first aspect, in a possible implementation, the OSA event, the REM event, and the elevated central sympathetic nerve activity event are sleep events that cause risen user blood pressure; and when the first sleep event includes any one or more of the OSA event, the REM event, and the elevated central sympathetic nerve activity event, before the wearable device obtains the motion data collected by the motion sensor and the PPG signal collected by the PPG module, the method further includes: The wearable device obtains user blood pressure measurement data within first duration; and the wearable device determines, based on the user blood pressure measurement data within the first duration, that the user is a hypertensive user; and when the user is in the sleep state and it is determined, based on the first PPG signal, that the user is in the first sleep event, that the wearable device measures the user blood pressure, and obtains the first blood pressure value specifically includes: in response to determining that the user is a hypertensive user, when detecting the first sleep event, the wearable device measures the user blood pressure, and obtains the first blood pressure value.

In this way, the wearable device can determine, based on historical blood pressure data, whether the user is a hypertensive user. After it is determined that the user is a hypertensive user, when a sleep event that causes risen user blood pressure is detected at night, the user blood pressure automatically starts to be measured. In this way, the wearable device can be automatically triggered to monitor blood pressure of the hypertensive user, and interference to sleep of the user can be reduced.

With reference to the first aspect, in a possible implementation, the NREM sleep event is a sleep event that causes dropped user blood pressure; and when the first sleep event includes the NREM sleep event, before the wearable device obtains the motion data collected by the motion sensor and the PPG signal collected by the PPG module, the method further includes: The wearable device obtains user blood pressure measurement data within first duration; and the wearable device determines, based on the user blood pressure measurement data within the first duration, that the user is a hypotensive user; and when the user is in the sleep state and it is determined, based on the first PPG signal, that the user is in the first sleep event, that the wearable device measures the user blood pressure, and obtains the first blood pressure value specifically includes: in response to determining that the user is a hypotensive user, when detecting the first sleep event, the wearable device measures the user blood pressure, and obtains the first blood pressure value.

In this way, the wearable device can determine, based on historical blood pressure data, whether the user is a hypotensive user. After it is determined that the user is a hypotensive user, when a sleep event that causes dropped user blood pressure is detected at night, the user blood pressure automatically starts to be measured. In this way, the wearable device can be automatically triggered to monitor blood pressure of the hypotensive user, and interference to sleep of the user can be reduced.

With reference to the first aspect, in a possible implementation, the wearable device further includes a blood pressure measurement device, the blood pressure measurement device includes an inflation component, an airbag, and a barometric pressure sensor, the airbag is connected to the inflation component and the barometric pressure sensor, and the PPG module includes a light source and a PPG sensor; before the wearable device obtains the motion data collected by the motion sensor and the PPG signal collected by the PPG module, the method further includes: The wearable device controls the blood pressure measurement device to collect N groups of blood pressure values, and controls the PPG module to collect N groups of PPG signals corresponding to the N groups of blood pressure values; and the wearable device generates a first target model based on the N groups of blood pressure values and the N groups of PPG signals, where an input into the first target model is a PPG signal, an output of the first target model is a blood pressure value, and N is a positive integer greater than or equal to 2; and that the wearable device measures the user blood pressure, and obtains the first blood pressure value specifically includes: the wearable device controls the PPG module to collect a second PPG signal; and the wearable device determines the first blood pressure value based on the second PPG signal and the first target model. According to the method, the wearable device may measure the user blood pressure at night based on the PPG signal collected by the PPG module, so that impact of nocturnal blood pressure measurement on sleep of the user can be further reduced.

With reference to the first aspect, in a possible implementation, the wearable device further includes a blood pressure measurement device, the blood pressure measurement device includes an inflation component, an airbag, and a barometric pressure sensor, and the airbag is connected to the inflation component and the barometric pressure sensor;
before the wearable device obtains the motion data collected by the motion sensor and the PPG signal collected by the PPG module, the method further includes: The wearable device collects N groups of airbag pressure and N groups of blood pressure values corresponding to the N groups of airbag pressure, where the N groups of airbag pressure include airbag pressure corresponding to a moment at which oscillation waves of barometric pressure in N groups of airbags reach a maximum value and airbag pressure corresponding to a moment at which an oscillation wave of barometric pressure in the airbag reaches a × Maximum value; and
the wearable device generates a second target model based on the N groups of airbag pressure and the N groups of blood pressure values corresponding to the N groups of airbag pressure, where an input into the second target model is a maximum value of the airbag pressure, an output of the first target model is a blood pressure value, and N is a positive integer greater than or equal to 2; and that the wearable device measures the user blood pressure, and obtains the first blood pressure value specifically includes: the wearable device controls the inflation component to input barometric pressure of first airbag pressure into the airbag, where the first airbag pressure is less than the airbag pressure corresponding to the moment at which the oscillation wave of the barometric pressure in the airbag reaches a × Maximum value; and the wearable device determines the first blood pressure value based on the first airbag pressure and the second target model.

According to the method, the wearable device may collect the user blood pressure at night in a micro-inflation/deflation manner by using the blood pressure measurement device, so that impact of nocturnal blood pressure measurement on sleep of the user can be further reduced.

With reference to the first aspect, in a possible implementation, before the wearable device obtains the motion data collected by the motion sensor and the PPG signal collected by the PPG module, the method further includes: The wearable device displays first prompt information when detecting that the wearable device switches from a non-worn state to a worn state, where the first prompt information is used to prompt the user to confirm whether the wearable device is worn by a local user; and the wearable device receives a first operation performed by the user on a first option in the first prompt information, and in response to the first operation, confirms that the wearable device is worn by the local user; and after the wearable device obtains the first blood pressure value, the method further includes: The wearable device stores the first blood pressure value in a first storage area, where the first storage area stores blood pressure measurement data of the local user.

In a possible implementation, the method further includes: The wearable device receives a second operation performed by the user on a second option in the first prompt information, and in response to the second operation, confirms that the wearable device is worn by a non-local user; and after the wearable device obtains the first blood pressure value, the method further includes: the wearable device stores the first blood pressure value in a second storage area, where the second storage area stores blood pressure measurement data of a non-local user, and the first storage area is different from the second storage area.

In this way, before starting to measure the blood pressure, the wearable device may prompt the user to choose whether the wearable device is worn by the local user. Therefore, blood pressure measurement data of different users can be prevented from being stored together, and accuracy of an analysis result of blood pressure measurement data of a single user is affected.

According to a second aspect, this application provides a wearable device. The wearable device includes a motion sensor, a PPG module, a memory, and a processor, the motion sensor, the PPG module, the memory, and the processor are coupled, the memory is configured to store a computer program, and when the processor invokes the computer program, the wearable device is enabled to perform the blood pressure monitoring method according to any possible implementation of any one of the foregoing aspects.

According to a third aspect, this application provides a computer-readable storage medium, including instructions. When the instructions are run on a wearable device, the wearable device is enabled to perform the blood pressure monitoring method according to any possible implementation of any one of the foregoing aspects.

According to a fourth aspect, this application provides a chip system. The chip system includes one or more processors, and the processor is configured to invoke computer instructions, to perform the blood pressure monitoring method according to any possible implementation of any one of the foregoing aspects.

According to a fifth aspect, this application provides a computer program product including instructions. When the computer program product runs on a wearable device, the wearable device is enabled to perform the blood pressure monitoring method according to any possible implementation of any one of the foregoing aspects.

For descriptions of beneficial effects of the second aspect to the fifth aspect, refer to the descriptions of the beneficial effects of the first aspect. Details are not described herein again in this application.

### BRIEF DESCRIPTION OF DRAWINGS

FIG. 1 is a diagram in which a user wears a wearable device 100;
FIG. 2 is a diagram of a composition structure of a wearable device 100;
FIG. 3 is an example diagram of a principle of an oscillometric method according to an embodiment of this application;
FIG. 4 is another example diagram of a principle of an oscillometric method according to an embodiment of this application;
FIG. 5A is a diagram of a hardware structure of a wearable device 100;
FIG. 5B is a diagram of a composition structure of an airbag, an air pump, and a pneumatic connection component according to an embodiment of this application;
FIG. 6A to FIG. 6E are diagrams in which a wearable device 100 receives a user operation and enables a nocturnal blood pressure measurement mode;
FIG. 6F to FIG. 6M are diagrams in which an electronic device 200 receives a user operation and enables a nocturnal blood pressure measurement mode;
FIG. 6N to FIG. 6O are diagrams in which a wearable device 100 automatically enables a nocturnal blood pressure measurement mode;
FIG. 6P to FIG. 6V are diagrams in which a wearable device 100 confirms a user identity;
FIG. 7A to FIG. 7D are diagrams in which a wearable device 100 measures user blood pressure;
FIG. 8A to FIG. 8D are diagrams of composition structures of several PPG modules;
FIG. 9A to FIG. 9I are diagrams in which a wearable device 100 displays a blood pressure measurement result;
FIG. 10A to FIG. 10E are diagrams in which after detecting abnormally high daytime blood pressure, a wearable device 100 prompts a user to record a recently executed activity event; and
FIG. 11A to FIG. 11E are diagrams in which after detecting abnormally low daytime blood pressure, a wearable device 100 prompts a user to record a recently executed activity event.

### DESCRIPTION OF EMBODIMENTS

The technical solutions according to embodiments of this application are clearly and completely described in the following with reference to the accompanying drawings. In the descriptions of embodiments of this application, "/" indicates or, unless otherwise specified. For example, A/B may indicate A or B. In this specification, "and/or" describes only an association relationship between associated objects, and indicates that three relationships may exist. For example, A and/or B may indicate the following three cases: Only A exists, both A and B exist, and only B exists. In addition, in the descriptions of embodiments of this application, "a plurality of" means two or more than two.

The following terms "first" and "second" are merely intended for a purpose of description, and shall not be understood as an indication or implication of relative importance or implicit indication of a quantity of indicated technical features. Therefore, a feature limited by "first" and "second" may explicitly or implicitly include one or more features. In the descriptions of embodiments of this application, unless otherwise specified, "a plurality of" means two or more.

A term "user interface (user interface, UI)" in the following embodiments of this application is a medium interface for interaction and information exchange between an application or an operating system and a user, and implements conversion between an internal form of information and a form acceptable to the user. The user interface is usually represented in a form of a graphical user interface (graphical user interface, GUI), and is a user interface that is related to a computer operation and that is displayed in a graphic manner. The user interface may be a visual interface element, for example, text, an icon, a button, a menu, a tab, a text box, a dialog box, a status bar, a navigation bar, or a widget, that is displayed on a display of a wearable device.

For a high-risk patient, nocturnal sleep monitoring is essential for effective prevention and intervention in a cardiovascular event. The nocturnal sleep monitoring may reflect a cardiovascular function and a risk level of a patient based on a plurality of physiological indicators, for example, including but not limited to respiration, a heart rate, sleep quality, blood pressure, and blood oxygen. The nocturnal sleep monitoring is essentially different from daytime monitoring, because hemodynamics, respiration, an autonomic nervous system, and the like of the patient have special changes in a sleep state, which may induce or aggravate the cardiovascular event. Compared with daytime blood pressure, nocturnal blood pressure is more closely related to a death risk of cardiovascular and cerebrovascular diseases. Therefore, nocturnal blood pressure of the patient may be monitored, to prevent occurrence of a critical situation such as the cardiovascular event. A user may measure user blood pressure by using a wrist ambulatory blood pressure monitor. The wrist ambulatory blood pressure monitor is worn on a wrist of the user, is easy to wear, and does not affect daily activities of the user. In addition, the wrist ambulatory blood pressure monitor facilitates measurement of ambulatory blood pressure of the user, to monitor the user blood pressure in real time.

With development of electronic technologies, functions of wearable devices are continuously enhanced. For example, the wearable device such as a band or a watch may provide a blood pressure measurement function. The user wears the wearable device on the wrist, so that not only the user blood pressure can be monitored in real time, but also other functions such as playing music, making/answering a call, sending a message, and viewing motion data can be implemented based on the wearable device.

After the user falls asleep at night, if the wearable device 100 continuously/aperiodically/periodically measures the user blood pressure, rest of the user may be disturbed, and sleep quality of the user is affected.

To monitor nocturnal abnormal blood pressure of the user without affecting sleep quality of a user, in a nocturnal blood pressure measurement method provided in this application, a wearable device 100 may monitor a sleep event of the user, and dynamically enable nocturnal blood pressure measurement, without continuously measuring user blood pressure. In this way, not only nocturnal abnormal blood pressure of the user can be monitored, but also impact on sleep quality of the user is reduced.

The sleep event includes a sleep event that may cause risen user blood pressure or a sleep event that may cause dropped user blood pressure. Abnormal user blood pressure may cause occurrence of a critical situation such as a cardiovascular event that affects life safety of the user.

The wearable device 100 may monitor a nocturnal sleep event, dynamically enable nocturnal blood pressure measurement, and monitor nocturnal blood pressure corresponding to the sleep event, to monitor whether the critical situation such as the cardiovascular event that affects the life safety of the user occurs.

The sleep event may include but is not limited to an obstructive sleep apnea (obstructive sleep apnea, OSA) event, a rapid eye movement (rapid eye movement, REM) sleep event, a non-REM sleep event, an elevated central sympathetic nerve activity event, and the like.

The following describes definitions of the foregoing different sleep events.

### 1. OSA event

The OSA event causes risen user blood pressure.

OSA is a common disease, and means that the user repeatedly experiences apnea or hypopnea due to upper airway obstruction in a sleep process, resulting in intermittent hypoxia and hypercapnia. The OSA event is related to diseases of a plurality of systems, and is an independent risk factor in a common disease such as a cardiovascular disease. At an end of apnea, intra-arterial blood pressure rises sharply as a cardiac output increases and a blood vessel contracts strongly. For example, the blood pressure may rise from normal 130/60 mmHg to 220/130 mmHg at the time of apnea.

Generally, an OSA patient mainly has symptoms including but not limited to the following symptoms: habitual snoring, daytime sleepiness, nocturnal asthma or awakening, insomnia, hypomnesis, and the like.

In some embodiments, whether the OSA occurs may be determined based on physiological data such as blood oxygen, a heart rate, and a respiratory rate collected by a PPG module preconfigured in the wearable device 100.

In other embodiments, whether the user has OSA during sleep may alternatively be determined based on an apnea-hypopnea index. For example, when the apnea-hypopnea index (apnea-hypopnea index, AHI) is greater than or equal to 5 times/hour, the OSA may be diagnosed.

### 2. REM sleep event

The REM sleep event may cause risen user blood pressure. For example, blood pressure of a hypertensive user may rise abnormally during the REM sleep event.

The REM sleep event is a stage of animal sleep. In a REM sleep stage, an eyeball moves quickly, and body muscles are relaxed. The REM sleep stage is similar to an awake state. The body is in a paralyzed state, but a brain activity is very active. The REM sleep event is related to dreaming. During the REM sleep event, a sympathetic nerve activity sometimes increases, and consequently, a heart rate and blood pressure of the user temporarily increase. In some embodiments, whether the user is in the REM sleep event may be determined based on physiological data such as a heart rate and a respiratory rate collected by a PPG module preconfigured in the wearable device 100.

### 3. NREM sleep event

The NREM sleep event may cause dropped user blood pressure. For example, blood pressure of a hypertensive user may drop abnormally during the REM sleep event.

NREM sleep may be sleep without rapid eye movement. In an NREM sleep stage, a brain activity drops to a lowest level, so that a human body can get complete comfort. The NREM sleep is different from REM sleep. Dreaming rarely occurs in the NREM sleep stage. The NREM sleep can be divided into four stages. A first stage occurs at the beginning of sleep. In this case, an eyeball moves slowly, and the user considers that the user is in an awake state. However, the user has a sleepy feeling. In a second stage, the user has entered an unconscious stage, and the eyeball no longer moves. A third stage is a transitional period between the second stage and the fourth stage.

The fourth stage may be a deep sleep stage. In this stage, the user has lowest awakening consciousness. The awakening consciousness of the user in the fourth stage is lower than awakening consciousness of the user in the third stage, the awakening consciousness of the user in the third stage is lower than awakening consciousness of the user in the second stage, and the awakening consciousness of the user in the second stage is lower than awakening consciousness of the user in the first stage.

In some embodiments, whether the NREM sleep event occurs may be determined based on physiological data such as a heart rate and a respiratory rate collected by a PPG module preconfigured in the wearable device 100.

### 4. Elevated central sympathetic nerve activity event

The elevated central sympathetic nerve activity event may cause risen user blood pressure. For example, blood pressure of a hypertensive user may rise abnormally during the elevated central sympathetic nerve activity event.

The elevated central sympathetic nerve activity event is related to a quantity of nocturnal awakenings, NREM sleep duration, and a REM sleep latency.

A REM sleep event latency may be duration from falling asleep to first occurrence of a REM sleep event.

The wearable device 100 may monitor a quantity of awakenings of the user, NREM sleep event duration, the REM sleep event latency, and the like. When the quantity of awakenings of the user is greater than a preset quantity of times, the NREM sleep event duration is less than a first value, and duration of the REM sleep event latency is less than a second value, a central sympathetic nerve activity of the user at night is enhanced. Consequently, user blood pressure is risen.

In some embodiments, whether the elevated central sympathetic nerve activity event occurs may be determined based on physiological data such as blood oxygen, a heart rate, and a respiratory rate collected by a PPG module preconfigured in the wearable device 100.

It can be learned from the foregoing descriptions that the OSA event, the REM sleep event, and the elevated central sympathetic nerve activity event may cause risen blood pressure of the hypertensive user, and the NREM sleep event may cause dropped blood pressure of the hypertensive user.

In addition to the foregoing sleep event, the wearable device 100 may further monitor another sleep event. In this application, the OSA event, the REM sleep event, the NREM sleep event, and the elevated central sympathetic nerve activity event are only used as examples for description, but constitute no limitation.

In addition to a PPG signal collected by the PPG module, the OSA event, the REM event, the NREM sleep event, and the elevated central sympathetic nerve activity event may be determined based on a signal collected by another component, for example, based on information such as an EMG electromyography signal, an EEG electroencephalography signal, and a GSR galvanic skin response signal collected by an electrode. This is not limited in this application.

In a nocturnal blood pressure monitoring method provided in this application, a wearable device 100 may monitor a nocturnal sleep event, and dynamically enable blood pressure monitoring, to reduce impact on sleep of a user.

The wearable device 100 may classify sleep events into two types based on impact of the sleep event on nocturnal blood pressure, including a sleep event that may cause risen blood pressure and a sleep event that may cause dropped blood pressure.

The sleep event that may cause risen blood pressure may include but is not limited to an OSA event, a REM sleep event, and an elevated central sympathetic nerve activity event.

The sleep event that may cause dropped blood pressure may include but is not limited to an NREM sleep event.

In some embodiments, different blood pressure measurement policies may be enabled for different populations with abnormal blood pressure.

For example, for a hypertensive population, the wearable device 100 may monitor the sleep event that causes risen blood pressure, and measure blood pressure after detecting the sleep event that causes risen blood pressure.

For another example, for a hypertensive population, the wearable device 100 may monitor the sleep event that causes dropped blood pressure, and measure blood pressure after detecting the sleep event that causes dropped blood pressure.

For another example, for a hypertensive population, the wearable device 100 may monitor both the sleep event that causes risen blood pressure and the sleep event that causes dropped blood pressure, and measure blood pressure after detecting the sleep event that causes risen blood pressure and the sleep event that causes dropped blood pressure.

In another embodiment, the user may alternatively set an occasion at which the wearable device 100 measures blood pressure. For example, the user may set that the wearable device 100 starts to measure blood pressure when detecting the sleep event that causes risen blood pressure. For another example, the user may set that the wearable device 100 starts to measure blood pressure when detecting the sleep event that causes dropped blood pressure. For another example, the user may set that the wearable device 100 starts to measure blood pressure when detecting the sleep event that causes risen blood pressure and the sleep event that causes dropped blood pressure. The following describes a wearable device 100, used to measure blood pressure, provided in this application.

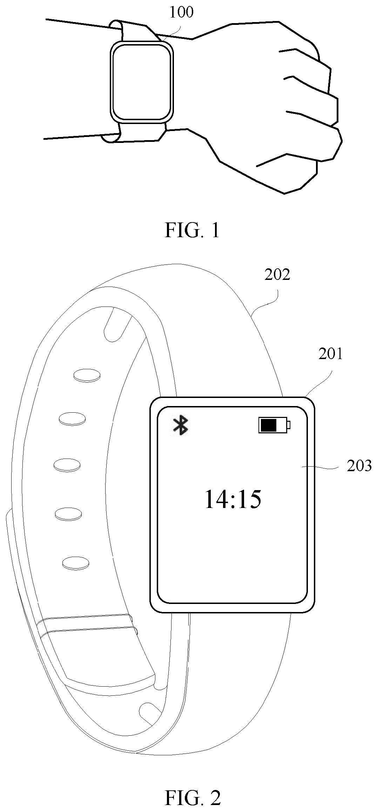

FIG. 1 is a diagram in which a user wears a wearable device 100.

As shown in FIG. 1, the user may wear the wearable device 100 on a wrist of the user.

FIG. 2 is a diagram of a composition structure of a wearable device 100.

As shown in FIG. 2, the wearable device 100 may include a watch body 201 and a wearable component 202.

The watch body 201 is equipped with a motion sensor, for example, a gyro sensor and an acceleration sensor. The motion sensor is configured to: collect motion data, and determine, based on a motion state obtained through analysis based on the motion data, whether the user is in a sleep state.

The watch body 201 may include a display 203. The display 203 may be configured to display time, a battery level of the watch body 201, a Bluetooth identifier, a received message, motion data of the user, and other content. The display 203 may be configured to receive a tap operation of the user to turn on the display, enable or disable a sport mode, or the like. The display 203 may further record a moving step count and consumed energy of the user, and has basic functions such as an incoming call reminder and a message notification. In a possible implementation, the watch body 201 may establish a wireless communication connection to the wearable device 100 through Bluetooth. The watch body 201 may send the motion data of the user to the wearable device 100 to which the connection is established. In addition, when the wearable device 100 receives an incoming call or a message notification, the watch body 201 may receive an instruction from a mobile phone, to remind the user about the incoming call or the message notification.

The wearable component 202 is used for mounting the watch body 201. For example, the wearable component 202 may be a wristband strap, a watch strap, or another apparatus. The wearable component 202 is an apparatus that can attach the watch body 201 to the wrist of the user. The wearable device 100 is attached to the wrist of the user, so that an inertial sensor collects motion data of the wrist of the user, to monitor motion of the wrist of the user and determine a user posture. When the wearable device 100 starts to measure blood pressure, the wearable device 100 may control the wearable component 202 to shrink and then expand, to measure user blood pressure. In some embodiments, a process in which the wearable device 100 measures blood pressure may include: The wearable device 100 first inflates the wearable component 202 to temporarily obstruct a brachial arterial vessel; then records, during slow deflation, a barometric pressure value of the wearable component 202 and a pulse signal generated by a pulse; and finally determines the user blood pressure based on the barometric pressure value of the wearable component 202 and an amplitude or an envelope of the pulse signal. Blood flow causes lateral pressure to a blood vessel wall. A change in a magnitude of the lateral pressure causes slight vibration of the blood vessel wall. The pulse signal is a signal generated through the slight vibration of the blood vessel wall. Determining the user blood pressure based on the barometric pressure value of the wearable component 202 and the amplitude or the envelope of the pulse signal is also referred to as an oscillometric method.

In another embodiment, a process in which the wearable device 100 measures blood pressure may include: The wearable device 100 may gradually inflate the wearable component 202, so that the brachial arterial vessel changes from being gradually blocked to being completely blocked; record a barometric pressure value of the wearable component 202 and a pulse signal generated by a pulse; then determine user blood pressure based on the barometric pressure value of the wearable component 202 and an amplitude or an envelope of the pulse signal; and finally perform deflation. Blood flow causes lateral pressure to a blood vessel wall. A change in a magnitude of the lateral pressure causes slight vibration of the blood vessel wall. The pulse signal is a signal generated through the slight vibration of the blood vessel wall. Determining the user blood pressure based on the barometric pressure value of the wearable component 202 and the amplitude or the envelope of the pulse signal is also referred to as an oscillometric method.

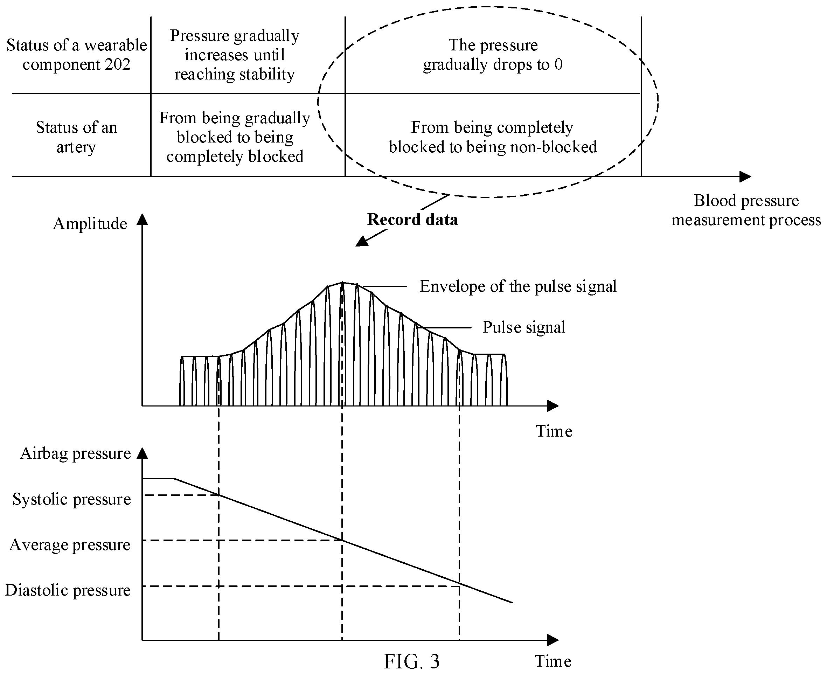

FIG. 3 is an example diagram of a principle of an oscillometric method according to an embodiment of this application.

As shown in FIG. 3, in a process in which a wearable device 100 inflates a wearable component 202 to temporarily obstruct a brachial arterial vessel, a status of the wearable component 202 is that pressure gradually increases until reaching stability, and a status of an artery is from being gradually blocked to being completely blocked. Then, during slow deflation, the status of the wearable component 202 is that pressure gradually drops to 0, and the status of the artery is from being completely blocked to being non-blocked. When the status of the wearable component 202 is that the pressure gradually drops to 0, a barometric pressure value and a pulse signal of the wearable component 202 are recorded. When the barometric pressure value of the wearable component 202 is greater than or equal to systolic pressure, the artery is obstructed, and the pulse signal is a fine oscillation wave. When the barometric pressure value of the wearable component 202 gradually decreases and is less than the systolic pressure and greater than average pressure, the artery gradually becomes non-blocked, and an amplitude of the pulse signal continuously increases. When the barometric pressure value of the wearable component 202 is equal to the average pressure, the amplitude of the pulse signal reaches a maximum value. When the barometric pressure value of the wearable component 202 continues to gradually decrease and is greater than diastolic pressure and less than the average pressure, the amplitude of the pulse signal gradually decreases. When the barometric pressure value of the wearable component 202 is less than the diastolic pressure, the pulse signal is a fine oscillation wave. Therefore, the wearable device 100 may determine the systolic pressure and the diastolic pressure of the user based on a change in the amplitude of the pulse signal and the barometric pressure value of the wearable component 202. In a possible implementation, the barometric pressure value and the pulse signal of the wearable component 202 may be determined by a barometric pressure sensor built in the wearable device 100.

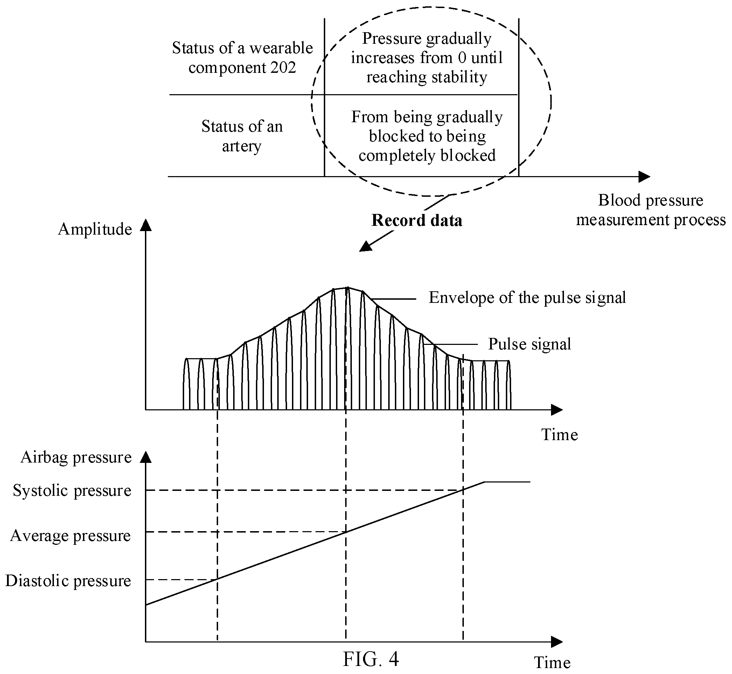

FIG. 4 is another example diagram of a principle of an oscillometric method according to an embodiment of this application.

As shown in FIG. 4, in a process in which a wearable device 100 inflates a wearable component 202 to temporarily obstruct a brachial arterial vessel, a status of the wearable component 202 is that pressure gradually increases until reaching stability, and a status of an artery is from being gradually blocked to being completely blocked. When the status of the wearable component 202 is that the pressure gradually increases until reaching stability, a barometric pressure value and a pulse signal of the wearable component 202 are recorded. When the barometric pressure value of the wearable component 202 gradually increases and diastolic pressure is less than average pressure, the pulse signal is a fine oscillation wave. When the barometric pressure value of the wearable component 202 continues to gradually increase and is greater than the diastolic pressure and less than the average pressure, an amplitude of the pulse signal gradually increases. When the barometric pressure value of the wearable component 202 is equal to the average pressure, the amplitude of the pulse signal reaches a maximum value. When the barometric pressure value of the wearable component 202 gradually increases and is greater than the average pressure and less than systolic pressure, the artery is gradually blocked, and the amplitude of the pulse signal continuously decreases. When the barometric pressure value of the wearable component 202 is greater than or equal to the systolic pressure, the artery is blocked, and the pulse signal is a fine oscillation wave. Therefore, the wearable device 100 may determine the systolic pressure and the diastolic pressure of the user based on a change in the amplitude of the pulse signal and the barometric pressure value of the wearable component 202. In a possible implementation, the barometric pressure value and the pulse signal of the wearable component 202 may be determined by a barometric pressure sensor built in the wearable device 100.

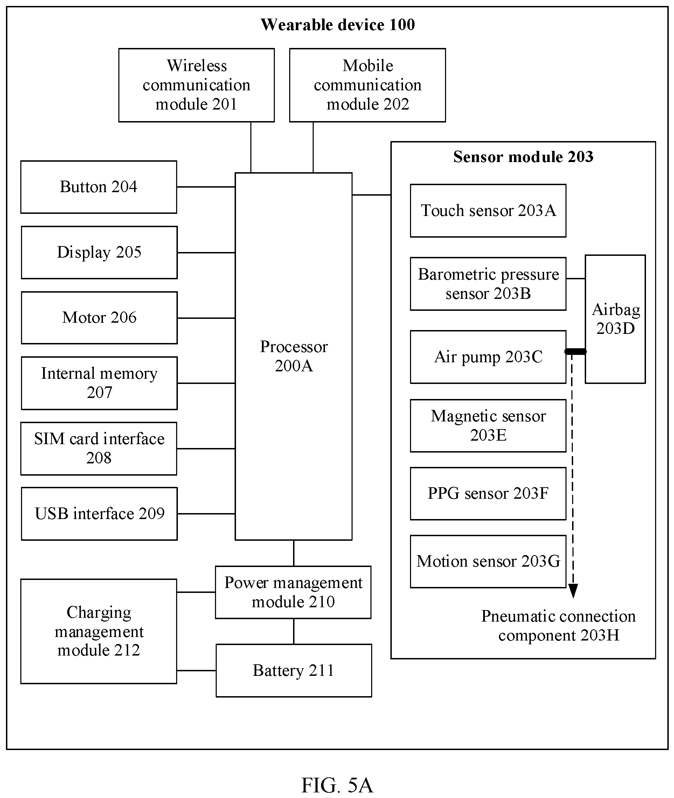

FIG. 5A is a diagram of a hardware structure of a wearable device 100.

As shown in FIG. 5A, the wearable device may be a band, a watch, or another wearable device; or the wearable device 100 may be a non-wearable device like a wall-mounted blood pressure monitor. A specific type of the wearable device is not particularly limited in this embodiment of this application. In this embodiment of this application, only an example in which the wearable device 100 is a watch is used for description.

The wearable device 100 may include a processor 200A, a wireless communication module 201, a mobile communication module 202, a sensor module 203, a button 204, a display 205, a motor 206, an internal memory 207, a SIM card interface 208, a USB interface 209, a power management module 210, a battery 211, and a charging management module 212. The sensor module 203 may include a touch sensor 203A, a barometric pressure sensor 203B, an air pump 203C, an airbag 203D, a magnetic sensor 203E, a photoplethysmography (photoplethysmography, PPG) sensor 203F, a motion sensor 203G, and a pneumatic connection component 203H. A function of the airbag 203D is similar to a function of a wearable component 202.

It can be understood that the structure shown in this embodiment of the present invention does not constitute a specific limitation on the wearable device. In some other embodiments of this application, the wearable device may include more or fewer components than those shown in the figure, or some components may be combined, or some components may be split, or the components may be arranged differently. The components shown in the figure may be implemented by hardware, software, or a combination of software and hardware.

The processor 200A may include one or more processing units. For example, the processor 200A may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, and/or a neural-network processing unit (neural-network processing unit, NPU). Different processing units may be independent components, or may be integrated into one or more processors.

In some embodiments, the processor 200A may include one or more interfaces. The interface may include an inter-integrated circuit (inter-integrated circuit, I2C) interface, an inter-integrated circuit sound (inter-integrated circuit sound, 12S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

In some embodiments, the processor 200A may alternatively be a micro processing unit (microcontroller unit, MCU).

The I2C interface is a bidirectional synchronous serial bus, and includes a serial data line (serial data line, SDA) and a serial clock line (serial clock line, SCL). In some embodiments, the processor 200A may include a plurality of groups of I2C buses. The processor 200A may be coupled to the touch sensor 203A, the power management module 210, and the like separately through different I2C bus interfaces. For example, the processor 200A may be coupled to the touch sensor 203A through the I2C interface, so that the processor 200A communicates with the touch sensor 203A through the I2C bus interface, to implement a touch function of the wearable device.

The I2S interface may be configured to perform audio communication. The PCM interface may also be used to perform audio communication, and sample, quantize, and code an analog signal. The UART interface is a universal serial data bus, and is configured to perform asynchronous communication. The bus may be a two-way communication bus. The bus converts to-be-transmitted data between serial communication and parallel communication. In some embodiments, the UART interface is usually configured to connect the processor 200A to the wireless communication module 201. For example, the processor 200A communicates with a Bluetooth module in the wireless communication module 201 through the UART interface, to implement a Bluetooth function.

The MIPI interface may be configured to connect the processor 200A to a peripheral component like the display 205. The MIPI interface includes a camera serial interface (camera serial interface, CSI), a display serial interface (display serial interface, DSI), and the like. The processor 200A communicates with the display 205 through the DSI interface, to implement a display function of the wearable device.

The GPIO interface may be configured by software. The GPIO interface may be configured as a control signal or a data signal. The USB interface 209 is an interface that conforms to a USB standard specification, and may be specifically a mini USB interface, a micro USB interface, a USB Type-C interface, or the like. The USB interface 209 may be configured to connect to a charger to charge the wearable device, or may be configured to transmit data between the wearable device and a peripheral device.

It can be understood that an interface connection relationship between the modules in this embodiment of the present invention is merely an example for description, and does not constitute a limitation on a structure of the wearable device. In some other embodiments of this application, the wearable device may alternatively use an interface connection mode different from that in the foregoing embodiment, or use a combination of a plurality of interface connection modes.

The charging management module 212 is configured to receive a charging input from a charger. The charger may be a wireless charger or a wired charger. In some embodiments of wired charging, the charging management module 212 may receive a charging input from a wired charger through the USB interface 209. In some embodiments of wireless charging, the charging management module 212 may receive a wireless charging input through a wireless charging coil of the wearable device. When charging the battery 211, the charging management module 212 may further supply power to the wearable device through the power management module 210.

The power management module 210 is configured to connect to the battery 211, the charging management module 212, and the processor 200A. The power management module 210 receives an input from the battery 211 and/or the charging management module 212, and supplies power to the processor 200A, the internal memory 207, the display 205, the wireless communication module 201, and the like. The power management module 210 may be further configured to monitor parameters such as a battery capacity, a quantity of battery cycles, and a battery health status (electric leakage or impedance). In some other embodiments, the power management module 210 may alternatively be disposed in the processor 200A. In some other embodiments, the power management module 210 and the charging management module 212 may alternatively be disposed in a same component.

A wireless communication function of the wearable device may be implemented by the mobile communication module 202, the wireless communication module 201, the modem processor, the baseband processor, and the like.

The mobile communication module 202 may provide a solution applied to the wearable device for wireless communication such as 2G/3G/4G/5G. The mobile communication module 202 may include at least one filter, a switch, a power amplifier, a low noise amplifier (low noise amplifier, LNA), and the like. The mobile communication module 202 may receive an electromagnetic wave through the antenna, perform processing such as filtering or amplification on the received electromagnetic wave, and transmit a processed electromagnetic wave to the modem processor for demodulation. In some embodiments, at least some functional modules of the mobile communication module 202 may be disposed in the processor 200A. In some embodiments, at least some functional modules of the mobile communication module 202 may be disposed in a same component as at least some modules of the processor 200A.

The wireless communication module 201 may provide a solution applied to the wearable device for wireless communication such as a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, or an infrared (infrared, IR) technology. The wireless communication module 201 may be one or more components integrating at least one communication processing module. The wireless communication module 201 receives an electromagnetic wave through the antenna, performs frequency modulation and filtering on an electromagnetic wave signal, and sends a processed signal to the processor 200A. The wireless communication module 201 may further receive a to-be-sent signal from the processor 200A, perform frequency modulation and amplification on the signal, and convert a processed signal into an electromagnetic wave for radiation through the antenna.

The button 204 includes a power button, a volume button, and the like. The button 204 may be a mechanical button or a touch button. The wearable device may receive an input on the button, and generate a button signal input related to a user setting and function control of the wearable device. The display 205 is configured to display an image, a video, or the like. The display 205 includes a display panel. The display panel may be a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flex light-emitting diode, FLED), a quantum dot light-emitting diode (quantum dot light-emitting diode, QLED), or the like. In some embodiments, the wearable device may include one or N displays 205, where N is a positive integer greater than 1.

The motor 206 may generate a vibration prompt. The motor 206 may be configured to produce an incoming call vibration prompt or a touch vibration feedback. For example, touch operations performed on different applications (for example, photographing and audio playback) may correspond to different vibration feedback effects. The motor 206 may also correspond to different vibration feedback effects for touch operations performed on different areas of the display 205. The internal memory 207 may include one or more random access memories (random access memory, RAM) and one or more non-volatile memories (non-volatile memory, NVM).

The random access memory may include a static random access memory (static random access memory, SRAM), a dynamic random access memory (dynamic random access memory, DRAM), a synchronous dynamic random access memory (synchronous dynamic random access memory, SDRAM), a double data rate synchronous dynamic random access memory (double data rate synchronous dynamic random access memory, DDR SDRAM, for example, a 5th generation DDR SDRAM is usually referred to as a DDR5 SDRAM), and the like.

The non-volatile memory may include a magnetic disk storage device and a flash memory (flash memory). According to an operating principle, the flash memory may be classified into NOR FLASH, NAND FLASH, 3D NAND FLASH, and the like; according to potential orders of cells, the flash memory may be classified into a single-level cell (single-level cell, SLC), a multi-level cell (multi-level cell, MLC), a triple-level cell (triple-level cell, TLC), a quad-level cell (quad-level cell, QLC), and the like; and according to storage specifications, the flash memory may be classified into a universal flash storage (English: universal flash storage, UFS), an embedded multi media card (embedded multi media Card, eMMC), and the like. The processor 200A may directly perform a read or write operation on the random access memory. The random access memory may be configured to store executable programs (for example, machine instructions) of an operating system or another running program, and may be further configured to store data of a user and an application, and the like. The non-volatile memory may also store an executable program, data of the user and an application, and the like, which may be pre-loaded to the random access memory for the processor 200A to directly perform a read or write operation.

The SIM card interface 208 is used for connecting a SIM card. The SIM card may be inserted into the SIM card interface 208 or removed from the SIM card interface 208, to implement contact with or separation from the wearable device. The wearable device may support one or N SIM card interfaces, where N is a positive integer greater than 1. The SIM card interface 208 may support a nano-SIM card, a micro-SIM card, a SIM card, and the like. A plurality of cards may be inserted in a same SIM card interface 208 at the same time. The plurality of cards may be of a same type or different types. The SIM card interface 208 is also compatible with different types of SIM cards. The SIM card interface 208 is also compatible with an external memory card. The wearable device interacts with a network through the SIM card, to implement functions such as calling and data communication. In some embodiments, an eSIM, namely, an embedded SIM card, is used for the wearable device. The eSIM card may be embedded into the wearable device, and cannot be separated from the wearable device.

In some embodiments, the wearable device 100 may alternatively not include the SIM card interface 208.

The touch sensor 203A is also referred to as a "touch device". The touch sensor 203A may be disposed in the display 205. The touch sensor 203A and the display 205 constitute a touchscreen, which is also referred to as a "touch screen". The touch sensor 203A is configured to detect a touch operation performed on or near the touch sensor. The touch sensor may transfer the detected touch operation to the application processor to determine a type of the touch event. The display 205 may provide a visual output related to the touch operation. In some other embodiments, the touch sensor 203A may alternatively be disposed on a surface of the wearable device at a location different from a location of the display 205.

The barometric pressure sensor 203B is configured to measure barometric pressure. In some embodiments of this application, the wearable device measures barometric pressure in the airbag 203D through the barometric pressure sensor 203B. In some embodiments of this application, some components of the barometric pressure sensor 203B are located in the airbag 203D, and are configured to sense barometric pressure in the airbag 203D.

The air pump 203C is configured to perform inflation and deflation. In some embodiments of this application, the wearable device inflates the airbag 203D through the air pump 203C, where the air pump 203C is connected to the airbag 203D through the pneumatic connection component 203H. The airbag 203D is configured to press against a blood vessel of the user.

The magnetic sensor 203E includes a Hall effect sensor. In some embodiments of this application, the wearable device may determine, through the magnetic sensor 203F, whether the airbag 203D on the wearable device is removed. For example, the airbag 203D or a watch strap connected to the airbag 203D may be equipped with a magnet. The wearable device may determine, through the magnetic sensor, a magnetic flux generated by the airbag 203D or the magnet on the airbag 203D, to determine whether the airbag 203D on the wearable device is removed.

The PPG sensor 203F is configured to obtain health data of the user based on a PPG signal collected by the PPG sensor 203F. The health data of the user includes but is not limited to a heart rate, blood oxygen, a respiratory rate, blood oxygen saturation (SaO2), and the like. In some embodiments, the PPG sensor 203F may also be referred to as a PPG module.

In addition to the PPG sensor 203F, the health data of the user may be obtained based on another component. In this application, the PPG sensor 203F is only used as an example for description, and should not constitute a limitation.

The motion sensor 203G includes but is not limited to an acceleration sensor and an angular velocity sensor. The motion sensor 203G may be configured to collect motion data, determine a motion status of the user based on the motion data, and then determine, based on the motion status of the user, whether the user is in a sleep state.

It should be noted that the pneumatic connection component 203H may be an independent component, or the pneumatic connection component 203H may be a pneumatic line formed by combining other hardware modules, or the pneumatic connection component 203H may be a part of another component, for example, a part of the air pump 203C, or a part of the airbag 203D.

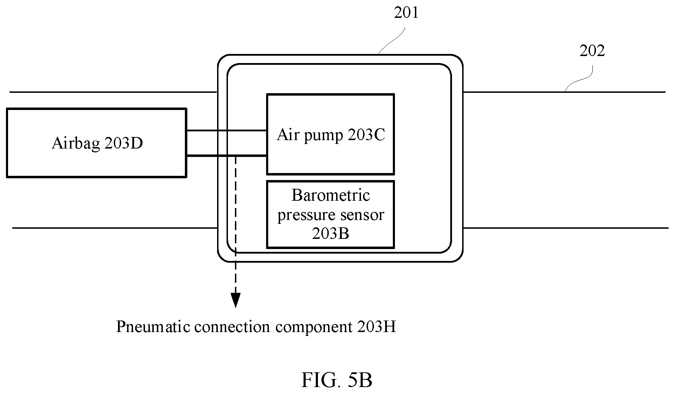

It should be noted that the sensor module 203 may further include an infrared sensor, and the like. As shown in FIG. 5B, when the wearable device 100 is a watch, the airbag 203D is attached to a side, close to a body, of the wearable component 202. The air pump 203C is connected to the airbag 203D through the pneumatic connection component 203H. The airbag 203D may be attached only to one side of the wearable component 202, and the side of the wearable component 202 may be located above an artery location on a wrist of the user, for example, above a radial artery location.

The air pump 203C may be located in a watch body of the smartwatch. The airbag 203D may be connected to a buckle of a watch strap, and the airbag 203D is connected to a watch face through an air vent cover. Correspondingly, the airbag 203D may be separated from the watch strap, or may be separated from the watch face.

The following describes how a wearable device 100 monitors nocturnal blood pressure of a user.

### I. Enable a nocturnal blood pressure measurement mode

A wearable device 100 may start to measure nocturnal blood pressure based on but not limited to any one or more of the following manners.

Manner 1: The wearable device 100 receives a user operation and enables a nocturnal blood pressure measurement mode.

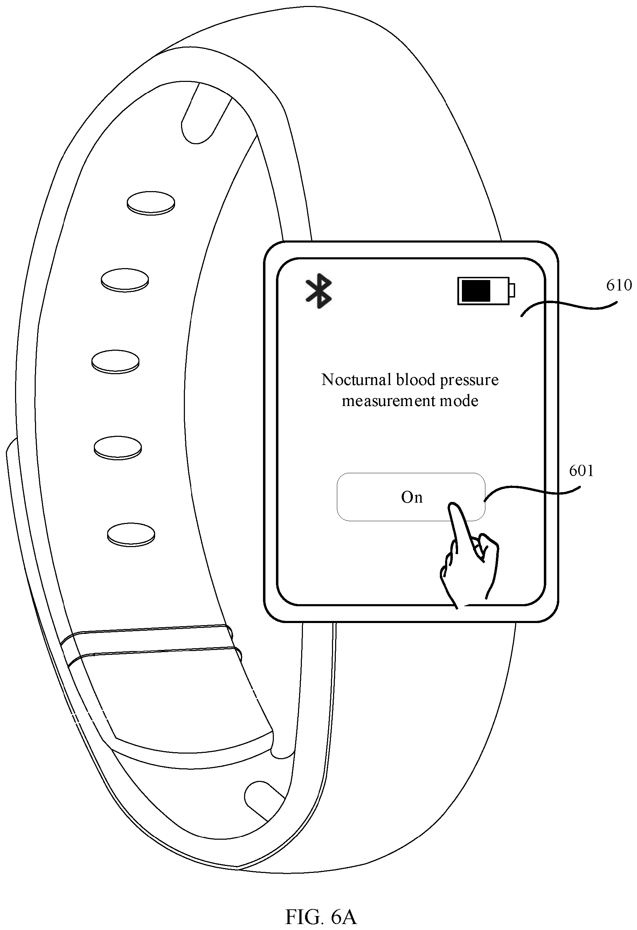

FIG. 6A to FIG. 6E are diagrams in which a wearable device 100 receives a user operation and enables a nocturnal blood pressure measurement mode.

For example, as shown in FIG. 6A, the wearable device 100 receives a user, and in response to the user operation, displays a user interface 610 shown in FIG. 6A. The user interface 610 may include an option 601, and the option 601 is used to enable a nocturnal blood pressure measurement mode of the wearable device 100.

As shown in FIG. 6A, the wearable device 100 may receive an input operation (for example, a tap) performed by a user on the option 601, and in response to the input operation performed by the user, the wearable device 100 may enable the nocturnal blood pressure measurement mode. That the wearable device 100 enables the blood pressure measurement mode may mean that after the wearable device 100 detects that the user falls asleep or a preset time arrives, the wearable device 100 starts to monitor a sleep event, and measures user blood pressure after detecting the sleep event.

In some embodiments, after the wearable device 100 enables the nocturnal blood pressure measurement mode, the wearable device 100 may prompt the user to select a to-be-monitored sleep event type.

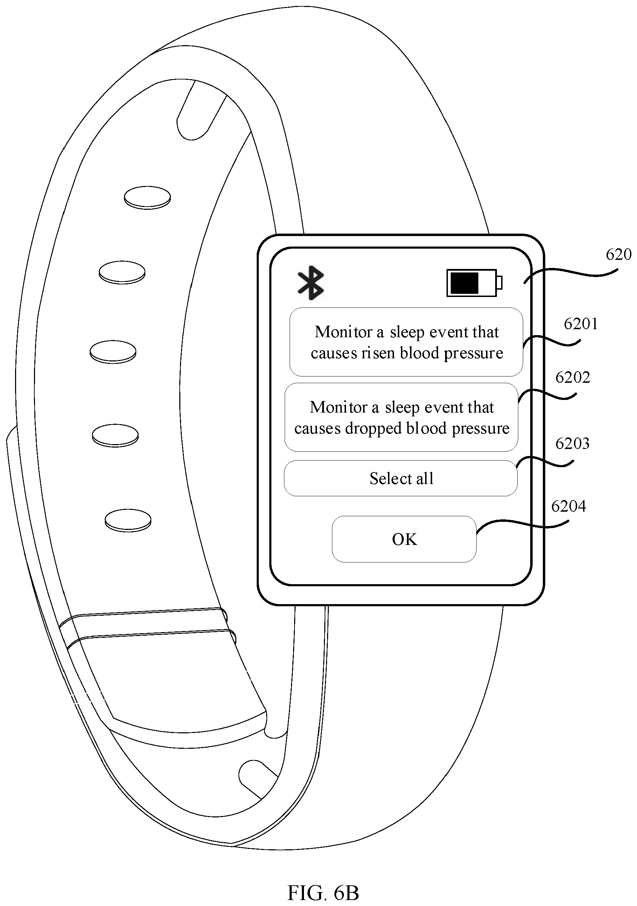

For example, in response to the input operation performed by the user on the option 601 in the user interface 610, the wearable device 100 may display a user interface 620 shown in FIG. 6B. The user interface 620 includes a plurality of options, and the plurality of options may include an option 6201, an option 6202, and an option 6203. The option 6201 is used by the user to select the wearable device 100 to monitor a sleep event that causes risen blood pressure, the option 6202 is used by the user to select the wearable device 100 to monitor a sleep event that causes dropped blood pressure, and the option 6203 is used by the user to select the wearable device 100 to monitor both the sleep event that causes risen blood pressure and the sleep event that causes dropped blood pressure.

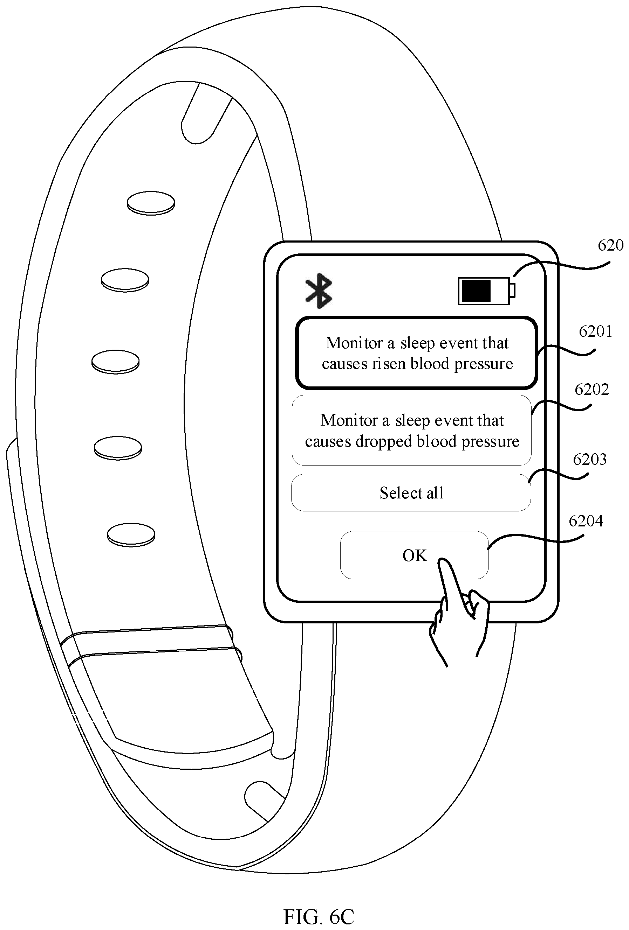

As shown in FIG. 6C, the wearable device 100 may receive an input operation (for example, a tap) performed by the user on the option 6201, and in response to the input operation performed by the user, the wearable device 100 may monitor, at night, whether a sleep event that causes risen blood pressure occurs, and start to measure user blood pressure after detecting that the sleep event that causes risen blood pressure occurs.

In response to selecting the option 6201, the wearable device 100 may change a display form of the option 6201, for example, deepen display. Then, the wearable device 100 may receive an input operation (for example, a tap) performed by the user on the option 6204 in the user interface 620, and in response to the input operation performed by the user, the wearable device 100 may start to monitor, at night, the sleep event that causes risen blood pressure.

In another embodiment, the wearable device 100 may alternatively not display user interfaces shown in FIG. 6B and FIG. 6C. After the wearable device 100 receives a user operation and enables the nocturnal blood pressure measurement mode, the wearable device 100 may start, by default, to monitor the sleep event that causes risen blood pressure, or the wearable device 100 may start, by default, to monitor the sleep event that causes dropped blood pressure, or the wearable device 100 may start, by default, to monitor the sleep event that causes risen blood pressure and the sleep event that causes dropped blood pressure.

In some embodiments, after the wearable device 100 receives a user operation and chooses to monitor the sleep event that causes risen blood pressure, the wearable device 100 may further prompt the user to choose to monitor a specific event of the sleep event that causes risen blood pressure.

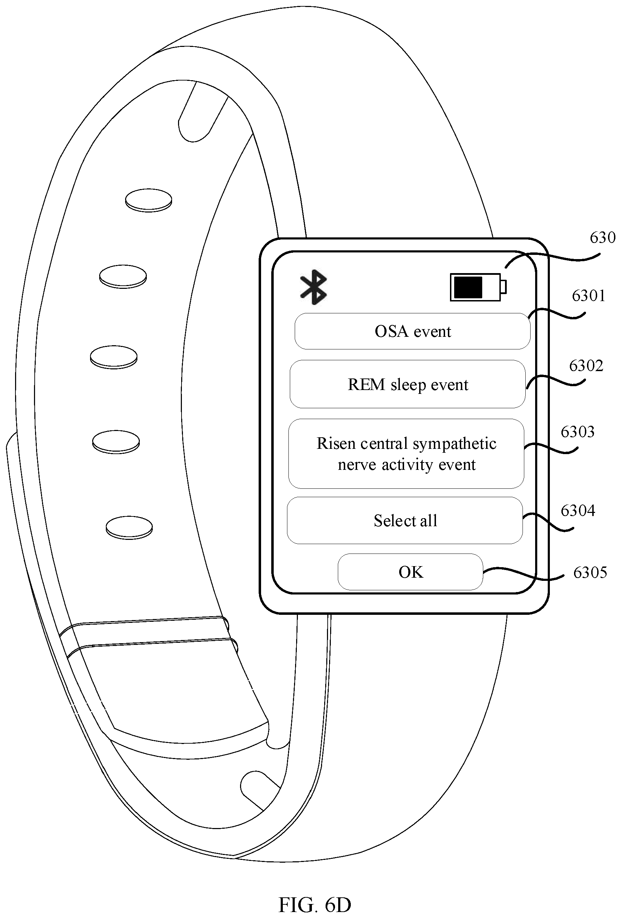

For example, in response to the input operation performed by the user on the option 6204 in the user interface 620, the wearable device 100 may display a user interface 630 shown in FIG. 6D. The user interface 630 includes a plurality of options, and the plurality of options may include an option 6301, an option 6302, an option 6303, and an option 6034. The option 6301 is used by the user to select the wearable device 100 to monitor an OSA event that causes risen blood pressure. The option 6302 is used by the user to select the wearable device 100 to monitor a REM sleep event that causes risen blood pressure. The option 6303 is used by the user to select the wearable device 100 to monitor an elevated central sympathetic nerve activity event that causes risen blood pressure. The option 6304 is used by the user to select the wearable device 100 to monitor an OSA event, a REM sleep event, and an elevated central sympathetic nerve activity event that cause risen blood pressure.

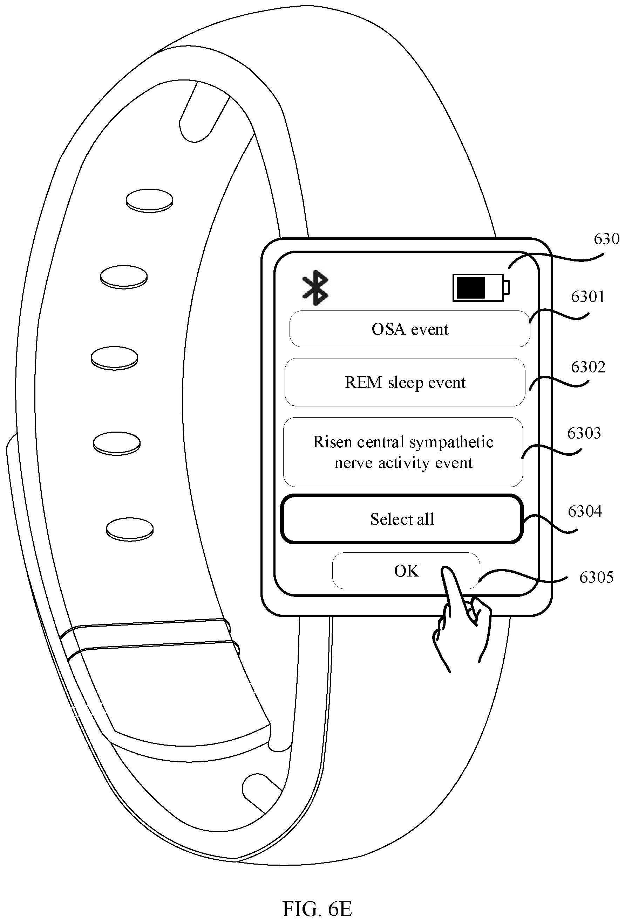

As shown in FIG. 6E, the wearable device 100 may receive the input operation (for example, a tap) performed by the user on the option 6304, and in response to the input operation performed by the user, the wearable device 100 may monitor, at night, whether an OSA event, a REM sleep event, or an elevated central sympathetic nerve activity event that causes risen blood pressure occurs, and start to measure user blood pressure after detecting that the OSA event, the REM sleep event, or the elevated central sympathetic nerve activity event occurs.

In response to selecting the option 6304, the wearable device 100 may change a display form of the option 6304, for example, deepen display. Then, the wearable device 100 may receive an input operation (for example, a tap) performed by the user on an option 6305 in the user interface 630, and in response to the input operation performed by the user, the wearable device 100 may start to monitor, at night, an OSA event, a REM sleep event, or an elevated central sympathetic nerve activity event that causes risen blood pressure.

In some embodiments, after the wearable device 100 receives the user operation and chooses to monitor the sleep event that causes risen blood pressure, the wearable device 100 may alternatively not display user interfaces shown in FIG. 6D and FIG. 6E. After the wearable device 100 receives the user operation and monitors the sleep event that causes risen blood pressure, the wearable device 100 may start to monitor, by default, any one or more of the OSA event, the REM sleep event, and the elevated central sympathetic nerve activity event that cause risen blood pressure. This is not limited in this application either.

Manner 2: The wearable device 100 receives a user operation by using an electronic device 200 that establishes a communication connection to the wearable device 100, and enables a nocturnal blood pressure measurement mode.

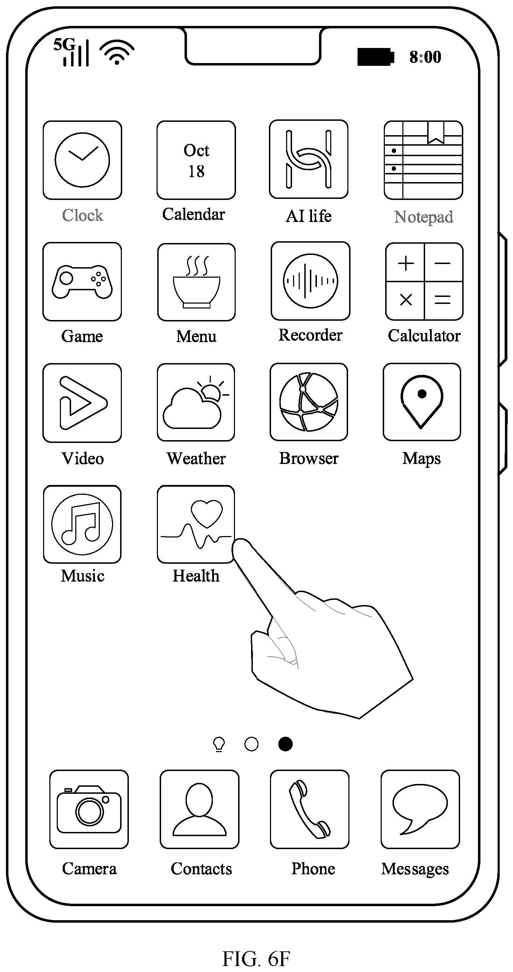

FIG. 6F to FIG. 6L are diagrams in which an electronic device 200 receives a user operation and enables a nocturnal blood pressure measurement mode.

For example, the wearable device 100 may establish a Bluetooth connection to the electronic device 200.

For example, as shown in FIG. 6F, the electronic device 200 displays a home screen, and the home screen shows application icons of a plurality of applications.

In some embodiments, the user may enable the blood pressure measurement mode in a Health application of the electronic device 200.

In addition to the Health application, a user may enable the blood pressure measurement mode in another application. The Health application is only used as an example for description in this application, but shall not constitute a limitation.

For example, the user may further enable the blood pressure measurement mode in an AI life application.

As shown in FIG. 6F, the home screen may include a Health application icon. In response to an operation performed on the Health application icon, the electronic device may open the Health application.

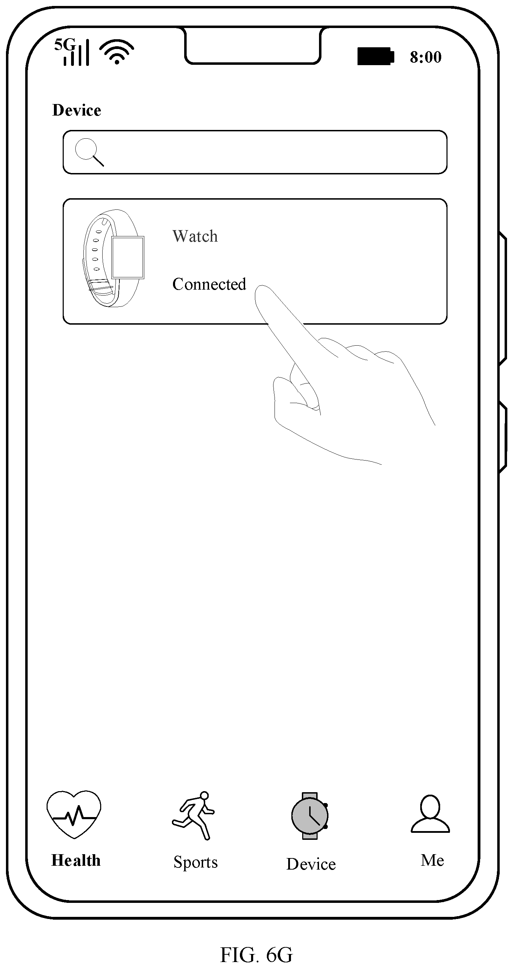

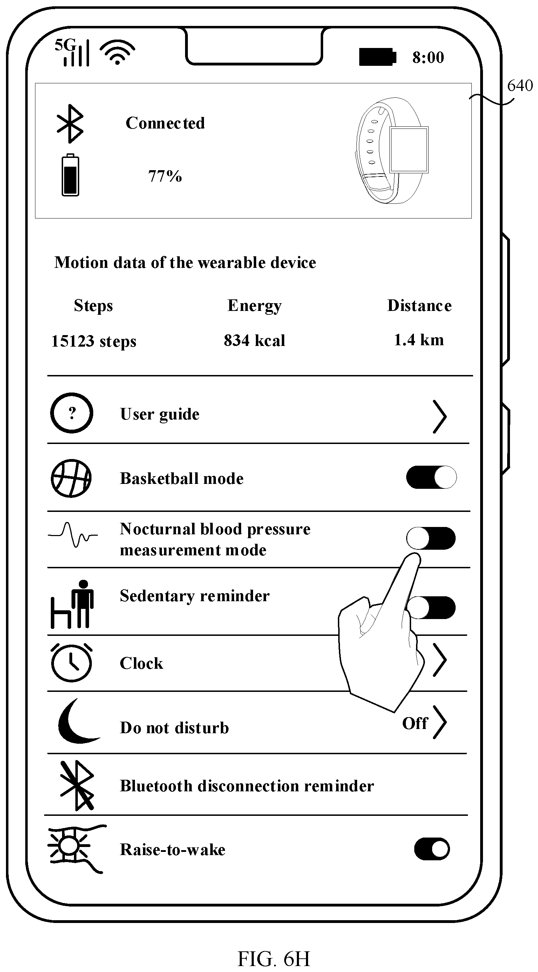

As shown in FIG. 6G, when the Health application is opened, the electronic device may display a user interface shown in FIG. 6G. The user interface shown in FIG. 6G may include a "Device" option. The "Device" option may be a "Device" option corresponding to the wearable device 100. A device icon and a device name of the wearable device 100 and a connection state between the wearable device 100 and the electronic device 200 may be displayed on the "Device" option. Content displayed on the "Device" option is not limited in this embodiment of this application. In response to an operation performed on the "Device" option, the electronic device may display a user interface 640 shown in FIG. 6H. The user interface 640 may be a user interface used to manage the wearable device 100 in the Health application.

The user interface 640 may include a device state, motion data, and a professional sports mode.

The device state may be used to indicate the connection state between the wearable device 100 and the electronic device and a battery level of the wearable device 100. For example, when it is detected that the electronic device establishes a communication connection relationship with the wearable device 100 in a Bluetooth connection manner, the device state may indicate that a connection manner is a Bluetooth connection and the connection state is "Connected". Further, the electronic device may obtain battery level information of the wearable device 100. The device state may indicate a current battery level of the wearable device 100, for example, 77%. Content indicated by the device state may further include more content. This is not limited in this embodiment of this application.

The motion data may include a moving step count, consumed energy, and a moving distance of the user that are recorded by the wearable device 100. Data in the motion data is one-day data that is of the user and that is recorded by the wearable device 100 in an operating state. The data may include a total moving step count, total consumed energy, and a total moving distance of the user in daily activities such as walking, playing basketball, and running.

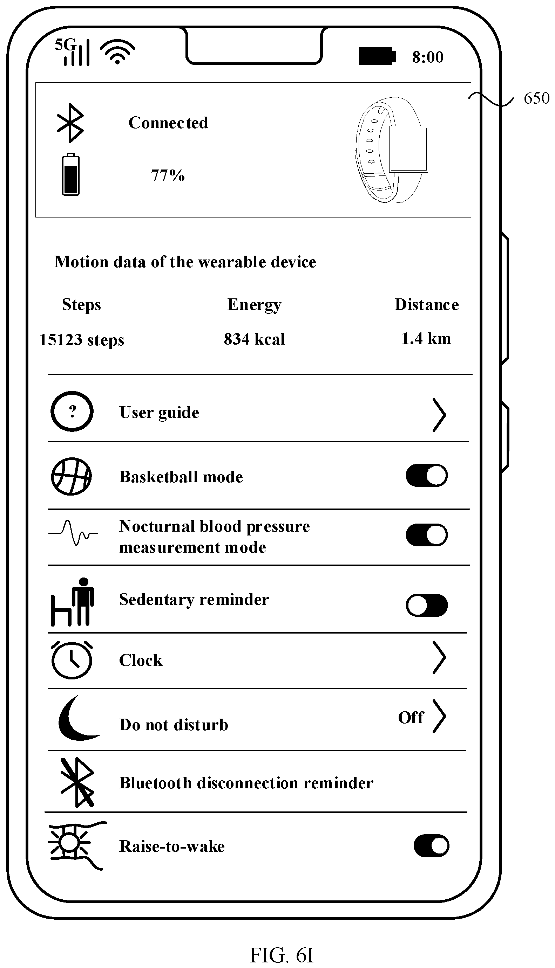

The professional sports mode may be used to enable or disable the nocturnal blood pressure measurement mode and a running mode. The professional sports mode may include an enabling button for the blood pressure measurement mode and an enabling button used to enable a running mode. In response to a user operation, for example, a touch operation, performed on the enabling button for the nocturnal blood pressure measurement mode, the electronic device may send, to the wearable device 100, an instruction for enabling the nocturnal blood pressure measurement mode, and display a user interface 650 shown in FIG. 6I. The electronic device may display a disabling button used to disable the nocturnal blood pressure measurement mode. In response to a user operation, for example, a touch operation, performed on the disabling button for the nocturnal blood pressure measurement mode, the electronic device 200 may send, to the wearable device 100, an instruction for disabling the nocturnal blood pressure measurement mode, and display the user interface 640 shown in FIG. 6H.

In some embodiments, after the electronic device 200 enables the nocturnal blood pressure measurement mode, the electronic device 200 may prompt the user to select a to-be-monitored sleep event type.

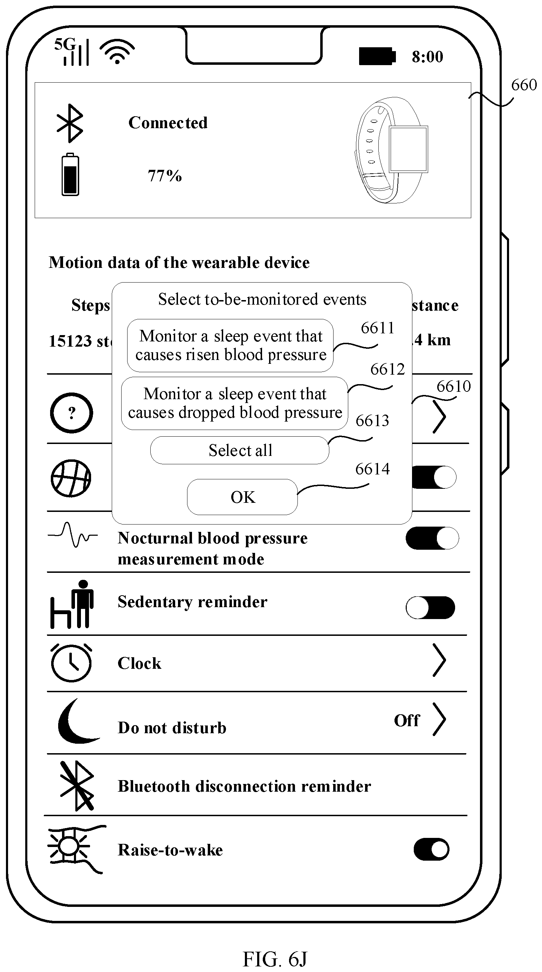

For example, in response to the user operation performed on the enabling button for the nocturnal blood pressure measurement mode, the electronic device 200 may display a user interface 660 shown in FIG. 6J. The user interface 660 includes a plurality of options, and the plurality of options may include an option 6611, an option 6612, and an option 6613. The option 6611 is used by the user to select the wearable device 100 to monitor a sleep event that causes risen blood pressure, the option 6612 is used by the user to select the wearable device 100 to monitor a sleep event that causes dropped blood pressure, and the option 6613 is used by the user to select the wearable device 100 to monitor both the sleep event that causes risen blood pressure and the sleep event that causes dropped blood pressure.

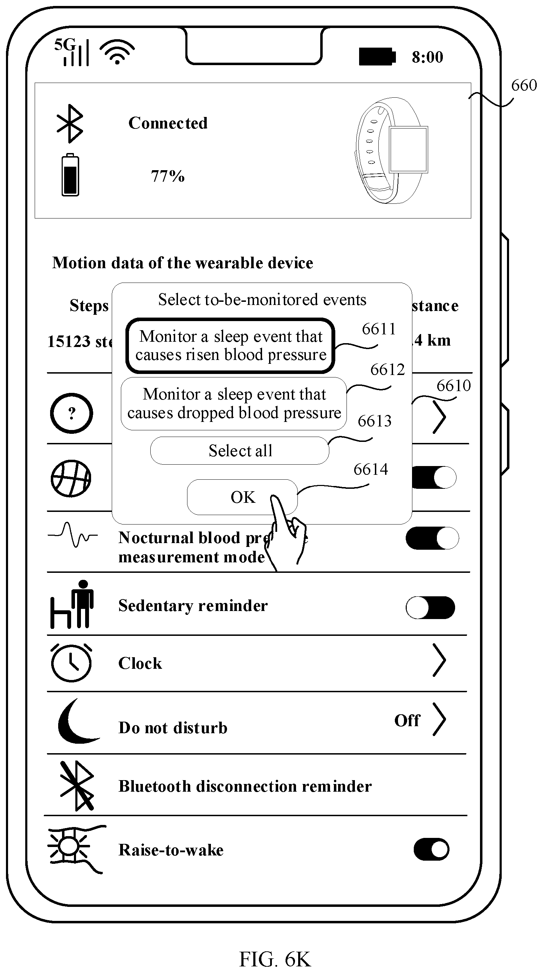

As shown in FIG. 6K, the electronic device 200 may receive an input operation (for example, a tap) performed by the user on the option 6611, and in response to the input operation performed by the user, the electronic device 200 may send, to the wearable device 100, an instruction for monitoring the sleep event that causes risen blood pressure. The wearable device 100 may monitor, at night, whether the sleep event that causes risen blood pressure occurs, and start to measure user blood pressure after detecting that the sleep event that causes risen blood pressure occurs.

In response to selecting the option 6611, the electronic device 200 may change a display form of the option 6611, for example, deepen display. Then, the electronic device 200 may receive an input operation (for example, a tap) performed by the user on the option 6614 in the user interface 660, and in response to the input operation performed by the user, the wearable device 100 may start to monitor, at night, the sleep event that causes risen blood pressure.

In another embodiment, the electronic device 200 may alternatively not display user interfaces shown in FIG. 6J and FIG. 6K. After the electronic device 200 receives a user operation and enables the nocturnal blood pressure measurement mode of the wearable device 100, the wearable device 100 may start, by default, to monitor the sleep event that causes risen blood pressure, or the wearable device 100 may start, by default, to monitor the sleep event that causes dropped blood pressure, or the wearable device 100 may start, by default, to monitor the sleep event that causes risen blood pressure and the sleep event that causes dropped blood pressure.

In some embodiments, after the electronic device 200 receives a user operation and chooses to monitor the sleep event that causes risen blood pressure, the electronic device 200 may further prompt the user to choose to monitor a specific event of the sleep event that causes risen blood pressure.

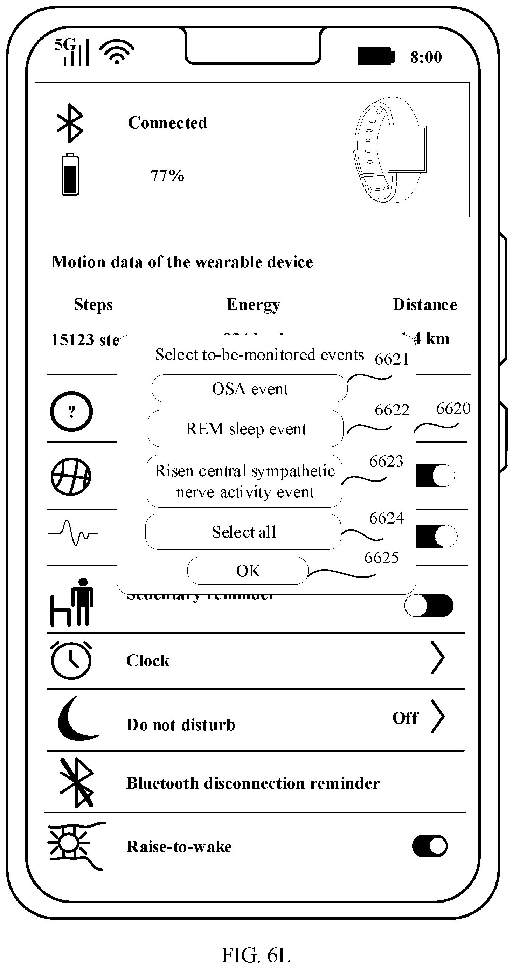

For example, in response to the input operation performed by the user on the option 6614 in the user interface 660, the electronic device 200 may display a user interface 6620 shown in FIG. 6L. The user interface 6620 includes a plurality of options, and the plurality of options may include an option 6621, an option 6622, an option 6623, and an option 6624. The option 6621 is used by the user to select the wearable device 100 to monitor an OSA event that causes risen blood pressure. The option 6622 is used by the user to select the wearable device 100 to monitor a REM sleep event that causes risen blood pressure. The option 6623 is used by the user to select the wearable device 100 to monitor an elevated central sympathetic nerve activity event that causes risen blood pressure. The option 6624 is used by the user to select the wearable device 100 to monitor an OSA event, a REM sleep event, and an elevated central sympathetic nerve activity event that cause risen blood pressure.

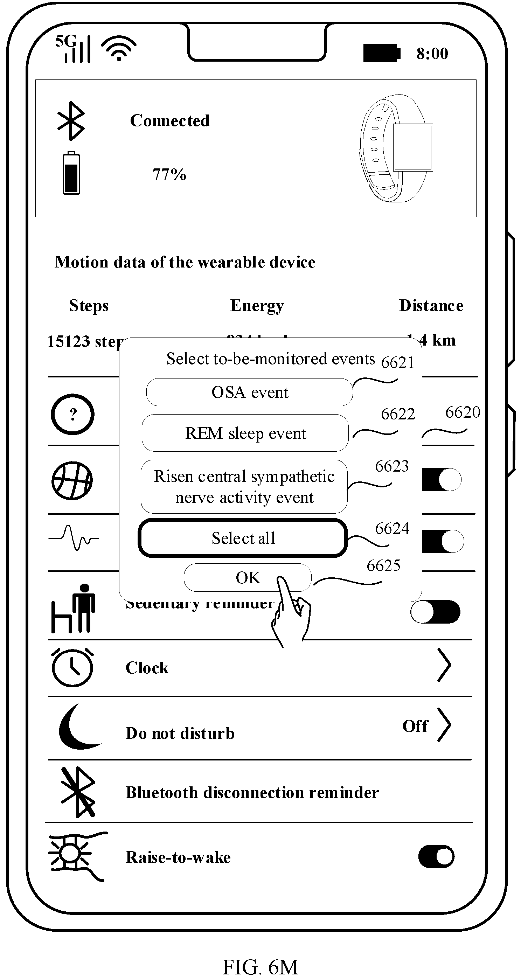

As shown in FIG. 6M, the electronic device 200 may receive an input operation (for example, a tap) performed by the user on the option 6624, and in response to the input operation performed by the user, the electronic device 200 may send, to the wearable device 100, an instruction for monitoring all sleep events that cause risen blood pressure. The wearable device 100 may monitor, at night, whether an OSA event, a REM sleep event, or an elevated central sympathetic nerve activity event that causes risen blood pressure occurs, and start to measure user blood pressure after detecting that the OSA event, the REM sleep event, or the elevated central sympathetic nerve activity event occurs.

In response to selecting the option 6624, the electronic device 200 may change a display form of the option 6624, for example, deepen display. Then, the electronic device 200 may receive an input operation (for example, a tap) performed by the user on an option 6625 in the user interface 660, and in response to the input operation performed by the user, the wearable device 100 may start to monitor, at night, an OSA event, a REM sleep event, or an elevated central sympathetic nerve activity event causes risen blood pressure.

In some embodiments, after the electronic device 200 receives the user operation and chooses to monitor the sleep event that causes risen blood pressure, the electronic device 200 may alternatively not display user interfaces shown in FIG. 6L and FIG. 6M. After the electronic device 200 receives the user operation and monitors the sleep event that causes risen blood pressure, the wearable device 100 may start to monitor, by default, any one or more of the OSA event, the REM sleep event, and the elevated central sympathetic nerve activity event that cause risen blood pressure. This is not limited in this application either.

Manner 3: The wearable device 100 automatically enables a nocturnal blood pressure measurement mode based on historical blood pressure data within first duration.

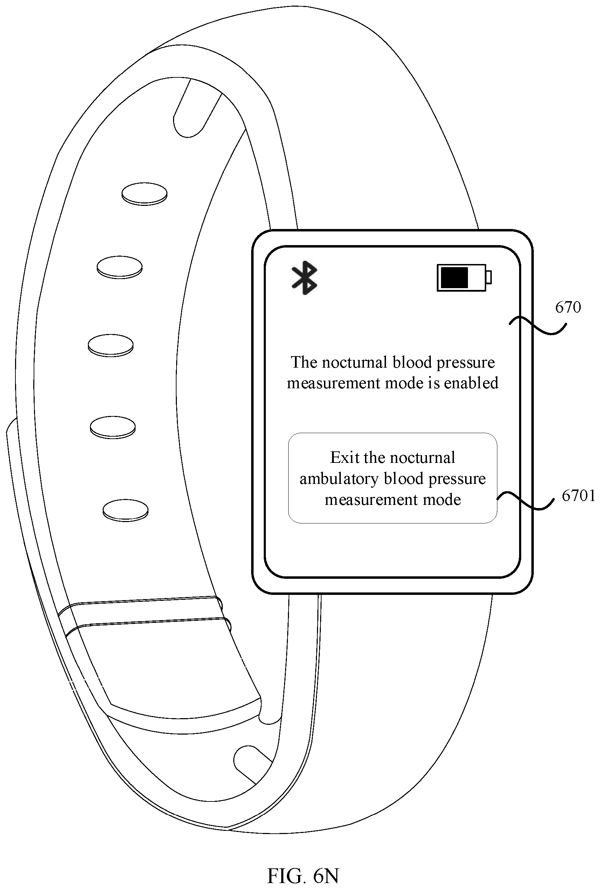

FIG. 6N to FIG. 6O are diagrams in which a wearable device 100 automatically enables a nocturnal blood pressure measurement mode.

Generally, normal blood pressure of a user is between 90 mmHg and 120 mmHg. If lowest user blood pressure is less than 90 mmHg and exceeds a specific threshold, it indicates that the user blood pressure is low, and the user may suffer from hypotension. If the lowest user blood pressure is greater than 120 mmHg and exceeds a specific threshold, it indicates that the user blood pressure is high, and the user may suffer from hypertension.

Optionally, normal blood pressure ranges of users with different physiological features may be different. That the normal blood pressure is between 90 mmHg and 120 mmHg is only used as an example for description in this application.

The wearable device 100 may collect statistics on historical blood pressure data of the user within the first duration, to obtain a blood pressure analysis result. For example, the user blood pressure is high, low, or normal.

For example, when the wearable device 100 learns, based on the historical blood pressure data through analysis, that the user blood pressure is high, the wearable device 100 may automatically monitor, at night, one or both of a sleep event that causes risen blood pressure and a sleep event that causes dropped blood pressure, and start to measure the user blood pressure after detecting that one or both of the sleep event that causes risen blood pressure and the sleep event that causes dropped blood pressure occur.

For example, when the wearable device 100 obtains, based on the historical blood pressure data through analysis, that the user blood pressure is low, the wearable device 100 may automatically monitor, at night, the sleep event that causes dropped blood pressure, and start to measure the user blood pressure after detecting that the sleep event that causes dropped blood pressure occurs.

In addition to the foregoing manner of enabling the nocturnal blood pressure measurement mode of the wearable device 100, the blood pressure measurement mode of the wearable device 100 may be enabled in another manner. This is not limited in this application.

In response to enabling the nocturnal blood pressure measurement mode, the wearable device 100 may enable the nocturnal blood pressure measurement mode after a countdown of 3 seconds after vibration. When the nocturnal blood pressure measurement mode is enabled, the user may be prompted that the nocturnal blood pressure measurement mode is enabled.

In some embodiments, after the wearable device 100 enables the nocturnal blood pressure measurement mode, the wearable device 100 may display a user interface 670 shown in FIG. 6N. The user interface 670 includes prompt information "The nocturnal blood pressure measurement mode is enabled". The prompt information is used to prompt the user that the wearable device 100 has enabled the nocturnal blood pressure measurement mode. The user interface 670 further includes an option 6701 of "Exit the nocturnal blood pressure measurement mode". The user may enable the wearable device 100 to disable the nocturnal blood pressure measurement mode by using the option 6701 of "Exit the nocturnal blood pressure measurement mode".

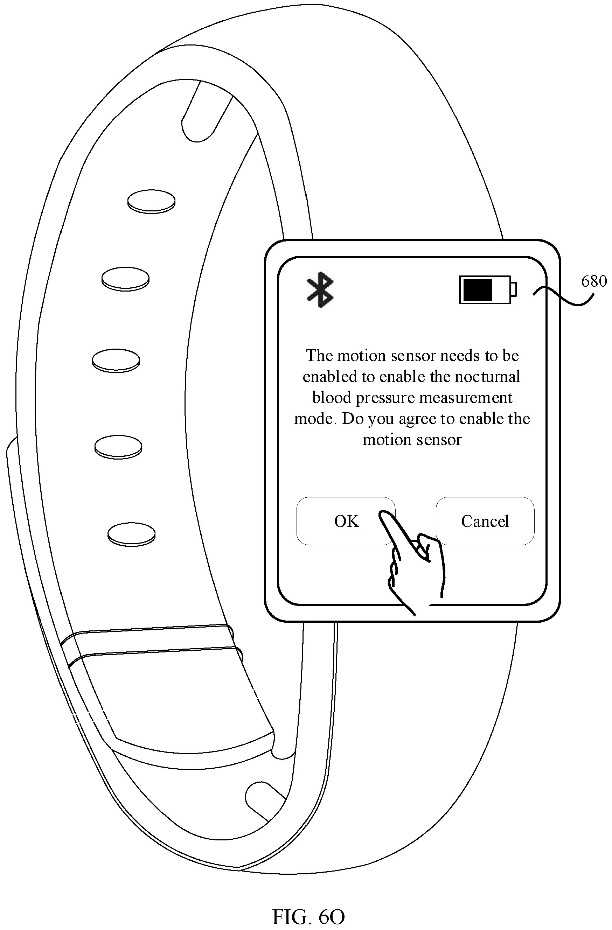

In some embodiments, after the wearable device 100 enables the nocturnal blood pressure measurement mode, the wearable device 100 may detect whether a motion sensor in the wearable device 100 is enabled, to ensure that the wearable device 100 can detect whether the user enters a sleep state. When the wearable device 100 detects that the motion sensor in the wearable device 100 is not enabled, the wearable device 100 may display a user interface 680 shown in FIG. 6O. The user interface 680 includes prompt information "The motion sensor needs to be enabled to enable the blood pressure measurement mode. Do you agree to enable the motion sensor", and the prompt information is used to prompt the user to enable the motion sensor in the wearable device 100. The user interface 680 further includes an "OK" option and a "Cancel" option. The user may enable the motion sensor in the wearable device 100 by using the "OK" option, or the user may not enable the motion sensor in the wearable device 100 by using the "Cancel" option.

For example, as shown in FIG. 6O, the wearable device 100 may receive an input operation (for example, a tap) performed by the user on the "OK" option in the user interface 680, and in response to the input operation performed by the user, the wearable device 100 may enable the motion sensor in the wearable device 100.

In another embodiment, after the wearable device 100 enables the nocturnal blood pressure measurement mode, when the wearable device 100 detects that the motion sensor in the wearable device 100 is enabled, the wearable device 100 may not display the prompt information shown in FIG. 6O.

In some embodiments, the wearable device 100 may collect statistics on a blood pressure measurement value of a same user in a period of time, analyze the blood pressure measurement value of the user in the period of time, and provide a targeted opinion on the blood pressure measurement value of the same user.

After the wearable device 100 enables the blood pressure measurement mode, and before the wearable device 100 starts to measure the user blood pressure, the wearable device 100 needs to confirm a user identity. In this way, blood pressure measurement values of different users may be separately stored, to avoid a problem that the wearable device 100 subsequently provides an inaccurate targeted opinion on the blood pressure measurement value of the same user because the blood pressure measurement values of the different users are mixed together.

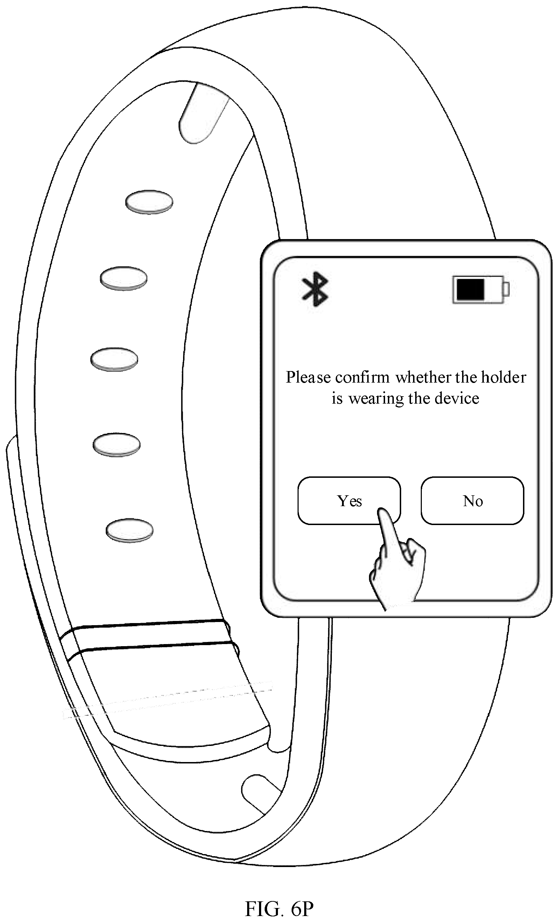

FIG. 6P to FIG. 6V are diagrams in which a wearable device 100 confirms a user identity.

Optionally, when the user wears the wearable device 100 a first time, the wearable device 100 may confirm the user identity. That the user wears the wearable device 100 the first time may mean that after being removed from a wrist by the user, the wearable device 100 is worn on the wrist of the user again, or is worn on the wrist of the user after a specific period of time.

For example, after the wearable device 100 enables the blood pressure measurement mode, the wearable device 100 may display a user interface shown in FIG. 6P. The user interface shown in FIG. 6P includes a prompt message "Please confirm whether the current person is wearing the device", and the prompt information is used to prompt the user to confirm an identity of a user who wears the wearable device 100. The current person may be an owner of the wearable device 100, and the owner of the wearable device 100 is a user who wears the wearable device 100 a long period of time. The user interface shown in FIG. 6P further includes a "Yes" option and a "No" option. The "Yes" option is used to confirm that the wearable device 100 is worn by the current person currently. The "No" option is used to confirm that the wearable device 100 is not worn by the current person currently.

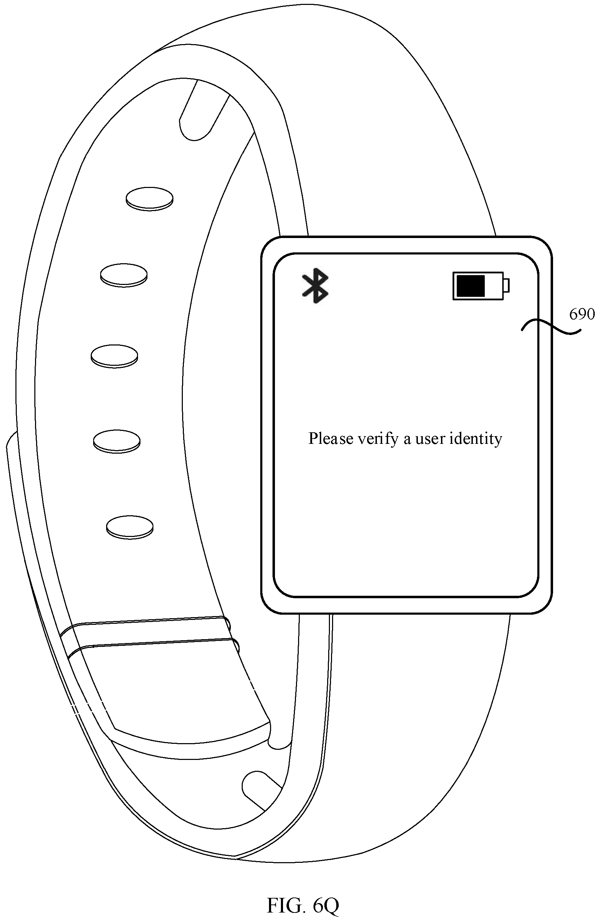

In a possible implementation, the wearable device 100 may receive an input operation (for example, a tap) performed by the user on the "Yes" option in the user interface shown in FIG. 6P, and in response to the input operation performed by the user, the wearable device 100 may display a user interface 690 shown in FIG. 6Q. The user interface 690 includes prompt information "Please verify a user identity", and the prompt information is used to prompt the user to verify whether a current user is the current person, to avoid a misoperation.

An identity verification manner includes but is not limited to facial recognition, fingerprint recognition, voiceprint recognition, and the like. Identity verification may alternatively be performed in another manner. This is not limited in this application.

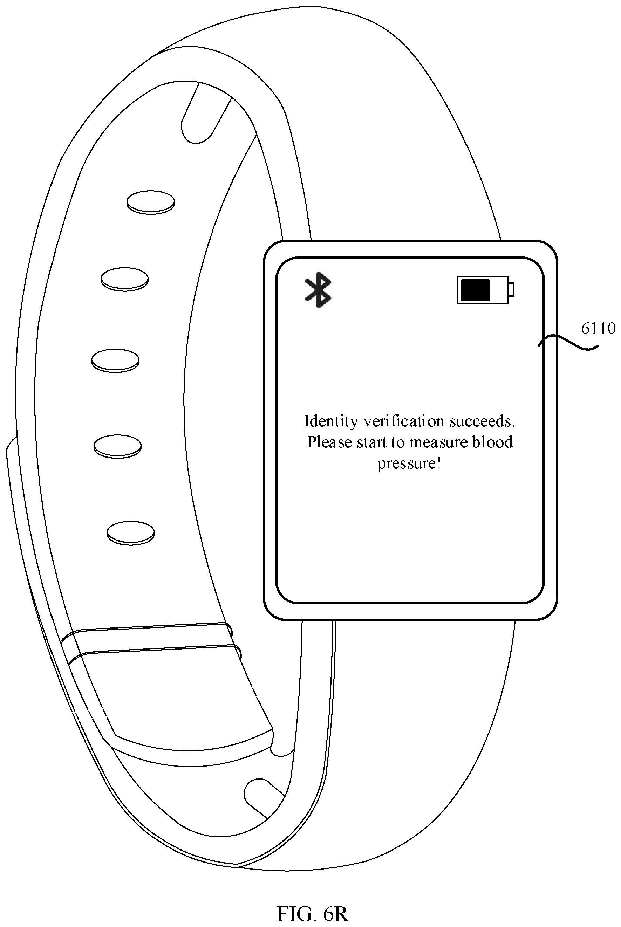

When identity verification succeeds, that is, it is determined that the owner of the wearable device 100 is wearing the device, the wearable device 100 may display a user interface 6110 shown in FIG. 6R. The user interface 6110 includes prompt information "Identity verification succeeds. Please start to measure blood pressure!", and the prompt information indicates, to the user, that the owner of the wearable device 100 is wearing the device.

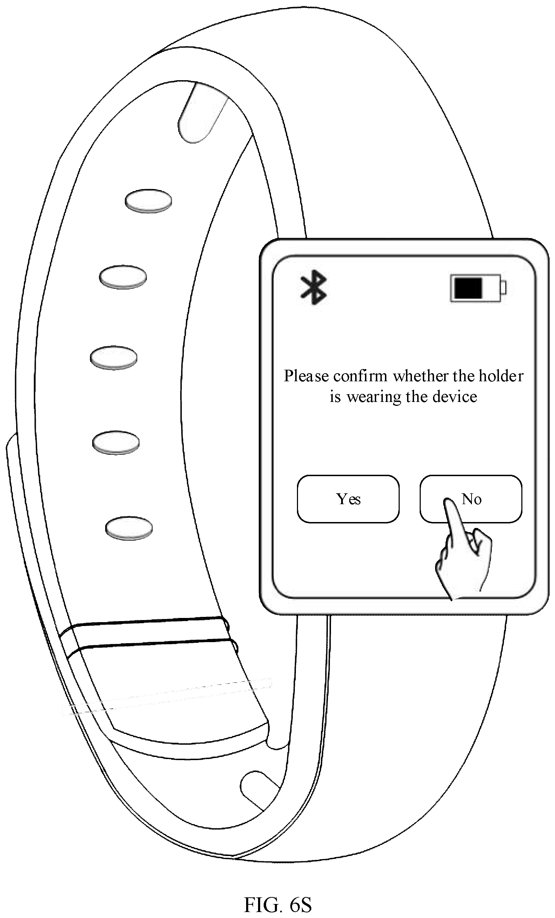

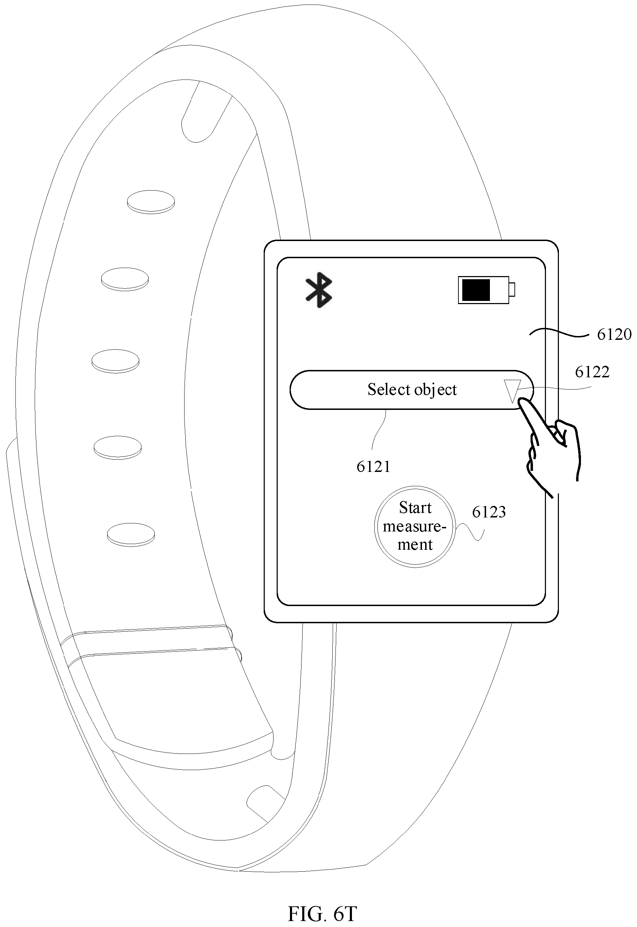

In another possible implementation, as shown in FIG. 6S, the wearable device 100 may receive an input operation (for example, a tap) performed by the user on a "No" option in a user interface shown in FIG. 6S, and in response to the input operation performed by the user, the wearable device 100 may display a user interface 6120 shown in FIG. 6T. The user interface 6120 includes a selection bar 6121, and the selection bar 6121 is used by the user to select a user name.

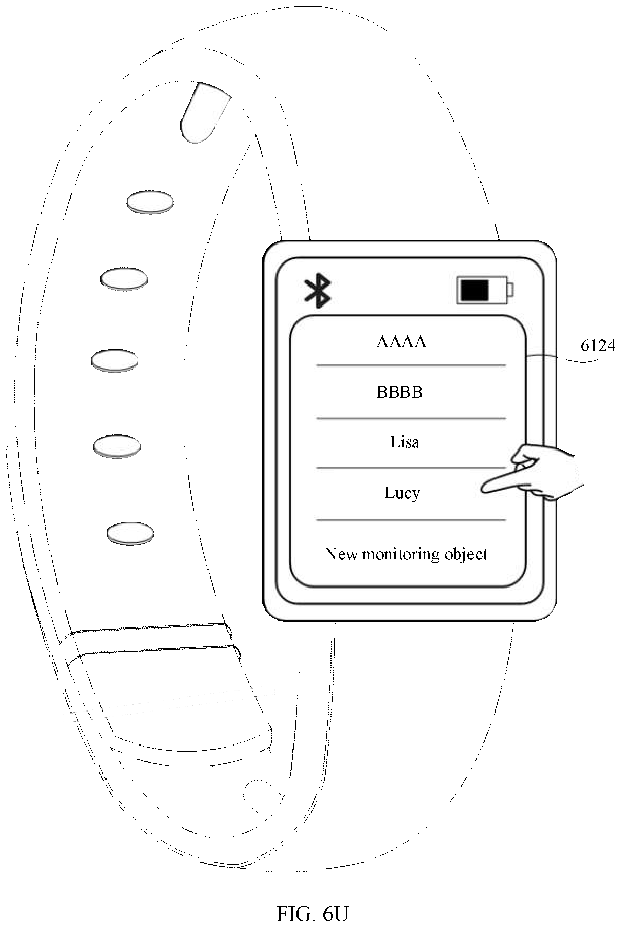

As shown in FIG. 6T, the wearable device 100 may receive an input operation (for example, a tap) performed by the user on an option 6122 in the selection bar 6121, and in response to the input operation performed by the user, the wearable device 100 may display a selection bar 6124 shown in FIG. 6U. Options of a plurality of monitoring objects are shown in the selection bar 6124. For example, the plurality of monitoring objects include but are not limited to a monitoring object "AAAA", a monitoring object "BBBB", a monitoring object "Lisa", and a monitoring object "Lucy". The user may select any monitoring object, and then start to measure the blood pressure; and bind current monitoring data and a currently selected monitoring object for storage, to avoid mixing blood pressure measurement results of different users.

Optionally, if the selection bar 6124 does not include an option of a user who needs to be detected currently, the wearable device 100 may receive an input operation performed by the user on an option of "New monitoring object" in the selection bar 6124, and add the option of the user who needs to be detected currently.

In this way, monitoring data of different monitoring objects may be stored separately, to facilitate subsequent view of the monitoring data of the different monitoring objects in specific duration.

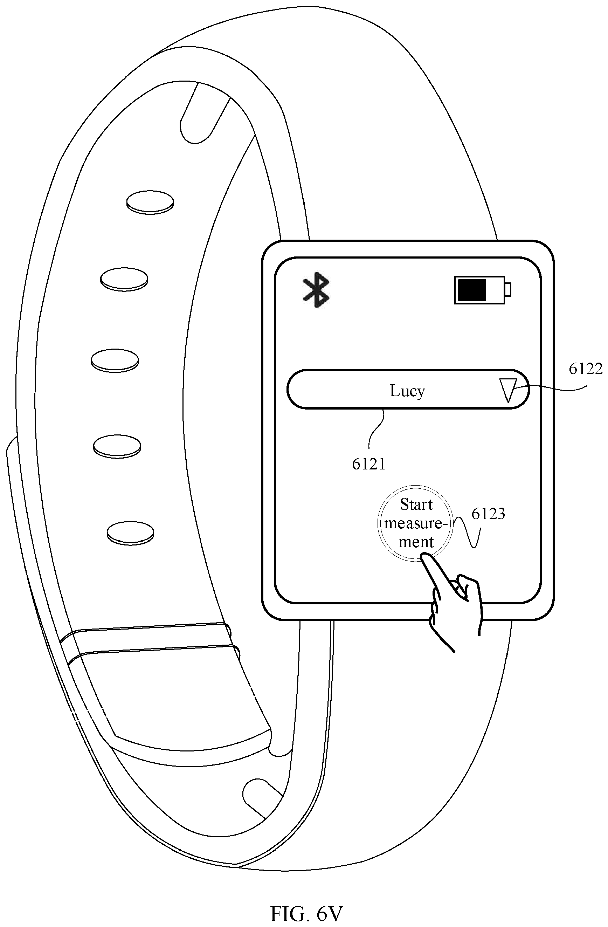

For example, as shown in FIG. 6U, the wearable device 100 may receive an input operation (for example, a tap) performed by the user on the option of the monitoring object "Lucy" in the selection bar 6124, and in response to the input operation performed by the user, the wearable device 100 may determine that a current monitoring object is "Lucy", and the wearable device 100 may display, in the selection bar 6121, a "Lucy" identifier shown in FIG. 6V.

Then, the wearable device 100 may receive an input operation (for example, a tap) performed by the user on a "Start measurement" option 6123, and in response to the input operation performed by the user, may start to measure the blood pressure.

Then, the electronic device 200 may bind a blood pressure measurement value of the monitoring object "Lucy" and the monitoring object "Lucy" for storage. Specifically, the electronic device 200 may find a storage area of the monitoring object "Lucy", and store the blood pressure measurement value of the monitoring object "Lucy" in the storage area of the monitoring object "Lucy". It should be noted that storage areas of different monitoring objects are different and are isolated from each other, to avoid mixing blood pressure measurement results of different users.

In another embodiment, as shown in FIG. 6S, after the wearable device 100 receives an input operation (for example, a tap) performed by the user on the "No" option in the user interface shown in FIG. 6S, the wearable device 100 may start to measure the user blood pressure, but does not store a blood pressure measurement value of the current user and a blood pressure measurement value of a previous user together, or the wearable device 100 may not store a blood pressure measurement value of the current user.

Obtain a correspondence between airbag pressure and blood pressure or a correspondence between a PPG signal and blood pressure.

### 1. Correspondence between airbag pressure and blood pressure

In some embodiments, the correspondence between airbag pressure and blood pressure may be a preset target model 1, and the preset target model 1 represents the correspondence between airbag pressure and blood pressure. For example, before a wearable device 100 is delivered from a factory, the target model 1 is preconfigured in the wearable device 100, or the wearable device 100 may dynamically obtain the target model 1 from a server. An input into the target model 1 may be airbag pressure applied by the wearable device 100, and an output of the target model 1 may be a group of blood pressure values of a user. In other words, when the airbag pressure applied by the wearable device 100 is input into the target model 1, a group of blood pressure values of the user may be obtained by using the target model 1.

It should be understood that the group of blood pressure values of the user may include systolic pressure of the user and diastolic pressure of the user. For ease of description, in this application, a group of blood pressure values are used to replace the systolic pressure of the user and the diastolic pressure of the user.

In some embodiments, to improve accuracy of the group of blood pressure values of the user that are obtained based on the airbag pressure, the target model 1 may be updated based on a plurality of groups of airbag pressure and blood pressure values corresponding to the airbag pressure, to obtain a target model 2. The target model 2 is obtained by updating the target model 1 based on the plurality of groups of airbag pressure of the user and the blood pressure values. Therefore, the blood pressure value that is of the user and that is obtained based on the airbag pressure by using the target model 2 is more accurate than a blood pressure value obtained by using the target model 1.

In another embodiment, when the wearable device 100 does not obtain the target model 1, the wearable device 100 may obtain, in real time, N groups of airbag pressure and N groups of blood pressure values corresponding to the N groups of airbag pressure, and obtain the correspondence between airbag pressure and blood pressure based on the N groups of airbag pressure and the blood pressure values corresponding to the airbag pressure. The wearable device 100 may obtain a group of target blood pressure values based on a target airbag pressure and the correspondence between airbag pressure and blood pressure. A group of target blood pressure values includes target systolic pressure and target diastolic pressure.

Optionally, to update accuracy of the target model 1 or to obtain the correspondence between airbag pressure and blood pressure, before starting to measure nocturnal blood pressure, the wearable device 100 may measure a blood pressure value of the user in real time, to obtain a plurality of groups of airbag pressure and a blood pressure value corresponding to the airbag pressure.

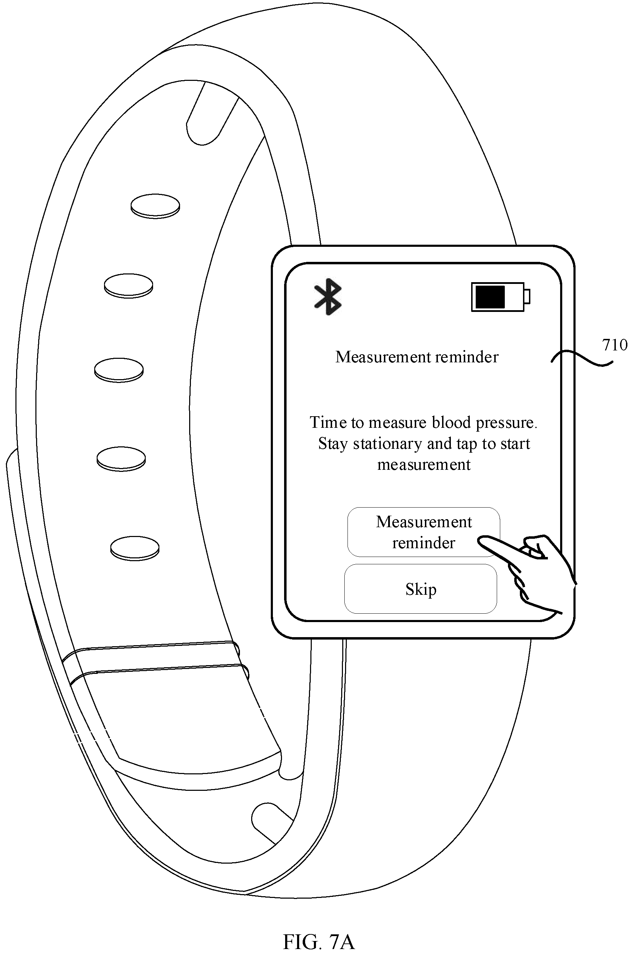

FIG. 7A to FIG. 7D are diagrams in which a wearable device 100 measures user blood pressure. For example, as shown in FIG. 7A, when the wearable device 100 starts to measure the blood pressure, to ensure accuracy of a blood pressure measurement result, the wearable device 100 may display a user interface 710 shown in FIG. 7A. The user interface 710 shows prompt information "Time to measure blood pressure. Stay stationary and tap to start measurement". The prompt information is used to prompt the user to remain in a stationary state during blood pressure measurement, to avoid an inaccurate blood pressure measurement result caused by motion. The user interface 710 further includes a "Measurement reminder" option and a "Skip" option. The user may view precautions for blood pressure measurement by using the "Measurement reminder" option. Alternatively, the user may directly start to measure the blood pressure without viewing the precautions for blood pressure measurement by using the "Skip" option.

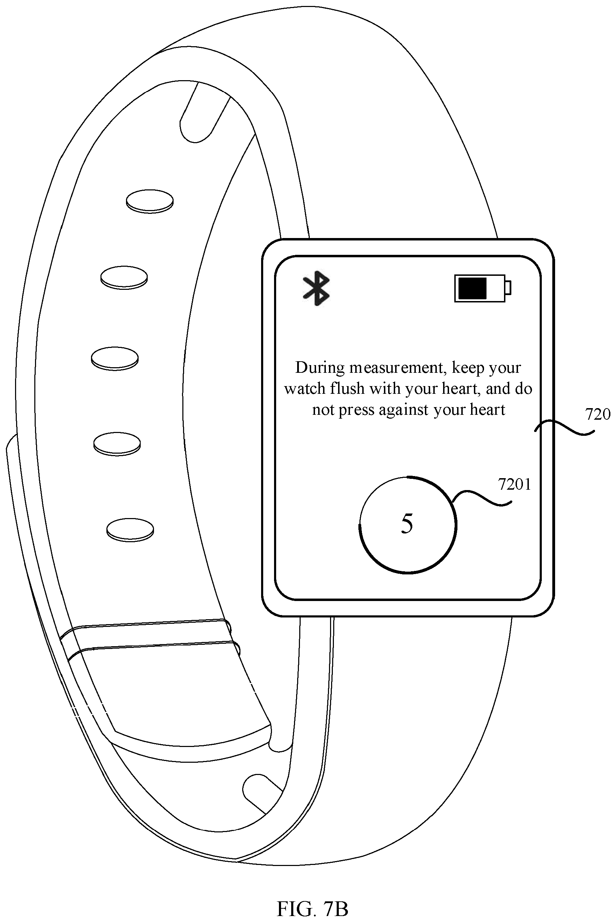

For example, as shown in FIG. 7A, the wearable device 100 may receive an input operation (for example, a tap) performed by the user on the "Measurement reminder" option in the user interface 710, and in response to the input operation performed by the user, the wearable device 100 may display a user interface 720 shown in FIG. 7B. The user interface 720 includes prompt information: "During measurement, keep your watch flush with your heart, and do not press against your heart", and the prompt information is used to prompt the user with a correct measurement posture. The user interface 720 includes a "Timer" option 7201. The "Timer" option 7201 is used to prompt the user to raise the watch to a location flush with the heart within preset time.

After countdown time displayed on the wearable device 100 reaches 0, the wearable device 100 may start to measure the user blood pressure.

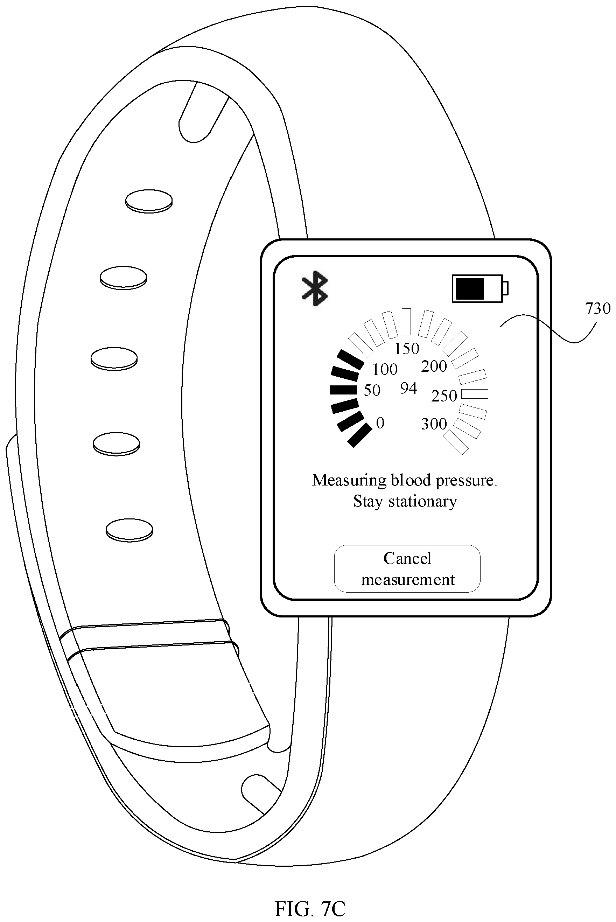

Optionally, in a process in which the wearable device 100 measures the user blood pressure, the wearable device 100 may display a user interface 730 shown in FIG. 7C. The user interface 730 includes prompt information "Measuring blood pressure. Stay stationary", to prompt the user to remain stationary during blood pressure measurement, to avoid an inaccurate blood pressure measurement result caused by motion. The user interface 730 further includes a "Cancel measurement" option. The user may stop the blood pressure measurement by using the "Cancel measurement" option.

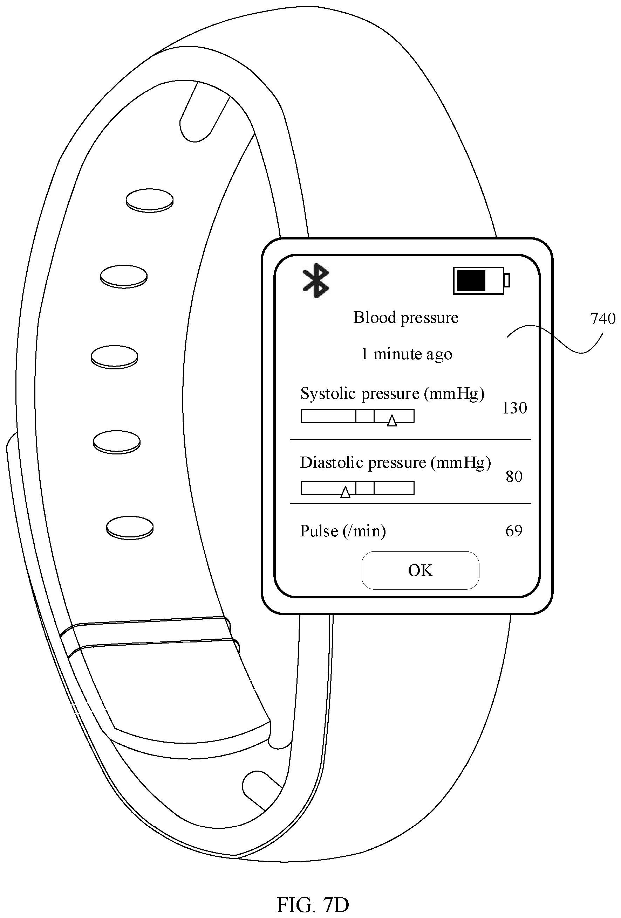

In some embodiments, after the wearable device 100 obtains a blood pressure monitoring value, the wearable device 100 may display a user interface 740 shown in FIG. 7D. The user interface 740 includes the blood pressure measurement value. The blood pressure measurement value may include systolic pressure and diastolic pressure. For example, the systolic pressure may be 130 mmHg, and the diastolic pressure may be 80 mmHg. In some embodiments, the user interface 740 may further include a pulse. For example, the pulse may be 69 times per minute.

In some embodiments, the wearable device 100 may alternatively not display the prompt information shown in FIG. 7A to FIG. 7D, but directly start to measure the user blood pressure, to avoid frequently disturbing the user. For example, at night, the wearable device 100 may not display the prompt information shown in FIG. 7A to FIG. 7D, and the wearable device 100 may automatically measure the user blood pressure without displaying the prompt information, to avoid disturbing rest of the user.

### 2. Correspondence between a PPG signal and blood pressure

In some embodiments, at night, a wearable device 100 measures user blood pressure through airbag inflation and deflation, which affects rest of a user. To avoid impact on the user, the wearable device 100 may obtain the user blood pressure by measuring a PPG signal, to reduce impact on sleep of the user.

In addition to the PPG signal, a correspondence between another signal and blood pressure may be obtained, to reduce impact on sleep of the user. In this application, the PPG signal is only used as an example for description.

In some embodiments, the correspondence between a PPG signal and blood pressure may be a preset target model 3, and the preset target model 3 represents the correspondence between a PPG signal and blood pressure. For example, before a wearable device 100 is delivered from a factory, the target model 3 is preconfigured in the wearable device 100, or the wearable device 100 may dynamically obtain the target model 3 from a server. An input into the target model 3 may be a PPG signal obtained by the wearable device 100 through measurement by using a PPG module, and an output of the target model 3 may be a group of blood pressure values of the user. In other words, when the PPG signal obtained by the wearable device 100 through measurement is input into the target model 3, a group of blood pressure values of the user may be obtained by using the target model 3.

In some embodiments, to improve accuracy of the group of blood pressure values of the user that are obtained based on the PPG signal, the target model 3 may be updated based on a plurality of groups of PPG signals and blood pressure values corresponding to the PPG signals, to obtain a target model 4. The target model 4 is obtained by updating the target model 3 based on the plurality of groups of PPG signals of the user and the blood pressure values. Therefore, the blood pressure value that is of the user and that is obtained based on the PPG signal by using the target model 4 is more accurate than a blood pressure value obtained by using the target model 3.

In another embodiment, when the wearable device 100 does not obtain the target model 3, the wearable device 100 may obtain, in real time, N groups of PPG signals and N groups of blood pressure values corresponding to the PPG signals, and obtain the correspondence between a PPG signal and blood pressure based on the N groups of PPG signals and the blood pressure values corresponding to the PPG signals. The wearable device 100 may obtain a group of target blood pressure values based on a target PPG signal and the correspondence between a PPG signal and blood pressure. A group of target blood pressure values includes target systolic pressure and target diastolic pressure.

Optionally, to update accuracy of the target model 3 or to obtain the correspondence between a PPG signal and blood pressure, before starting to measure nocturnal blood pressure, the wearable device 100 may measure a user blood pressure value corresponding to the PPG signal in real time, to obtain a plurality of groups of PPG signals and blood pressure values corresponding to the PPG signals.

Optionally, the wearable device 100 may alternatively prompt, in a manner shown in FIG. 7A to FIG. 7D, the user to measure the PPG signal and the blood pressure, to obtain a plurality of groups of PPG signals and blood pressure values corresponding to the PPG signals. Details are not described herein in this application.

The PPG signal may be obtained by the wearable device 100 by using a preconfigured PPG module. The PPG module in this application may include at least one light source and at least one photoelectric detector. The at least one light source may emit a light source, the light source may be partially absorbed by a human body, and a part of the light source is reflected by the human body. The at least one photoelectric detector may receive the reflected light, and obtain a PPG signal based on the reflected light.

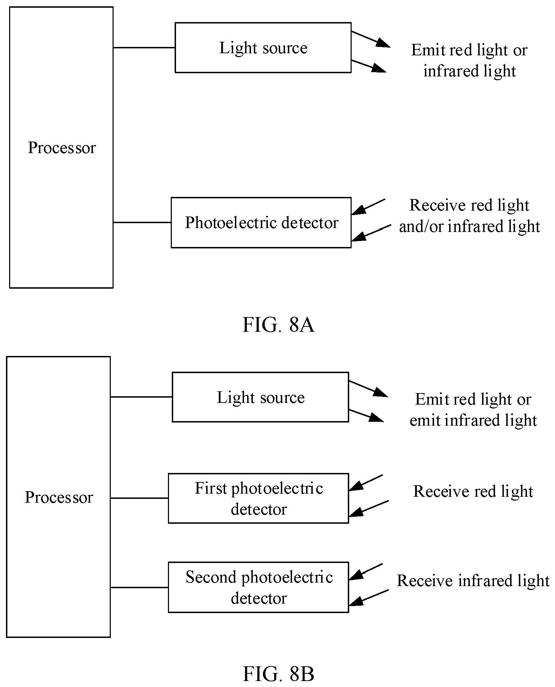

FIG. 8A to FIG. 8D are diagrams of composition structures of several PPG.

For example, as shown in FIG. 8A, the PPG module includes a processor, a light source, and a photoelectric detector.

There may be one or more light sources, and there may be one or more photoelectric detectors. This is not limited in this application.

Different light may be emitted by a same light source, and different light sources are emitted through time division multiplexing.

The light source may emit red light, or may emit infrared light, or may emit another light source, for example, green light or blue light. This is not limited in this application either. In some embodiments, the light source may be a light emitting apparatus such as a light emitting diode (light emitting diode, LED).

Different light may be received by a same photoelectric detector, and different light sources are received through time division multiplexing. In some embodiments, the photoelectric detector may be a receiving apparatus such as a photodiode (photodiode, PD).

For example, when the light source emits red light, the photoelectric detector may receive reflected red light.

When the light source emits infrared light, the photoelectric detector may receive reflected infrared light.

The processor may obtain the PPG signal based on the reflected red light and/or the reflected infrared light collected by the photoelectric detector, and obtain health data monitoring data based on the PPG signal.

For example, as shown in FIG. 8B, the PPG module includes a processor, a light source, a first photoelectric detector, and a second photoelectric detector.

There may be one or more light sources, there may be one or more first photoelectric detectors, and there may be one or more second photoelectric detectors. This is not limited in this application. Different light may be emitted by a same light source, and different light sources are emitted through time division multiplexing.

The light source may emit red light, or may emit infrared light, or may emit another light source. In some embodiments, the light source may be a light emitting apparatus such as a light emitting diode.

Different light needs to be received by different photoelectric detectors. In some embodiments, the photoelectric detector may be a receiving apparatus such as a photodiode (photodiode, PD).

For example, when the light source emits red light, the first photoelectric detector may receive reflected red light.

When the light source emits infrared light, the second photoelectric detector may receive reflected infrared light.

The processor may obtain a PPG signal based on the reflected red light collected by the first photoelectric detector and/or the reflected infrared light collected by the second photoelectric detector.

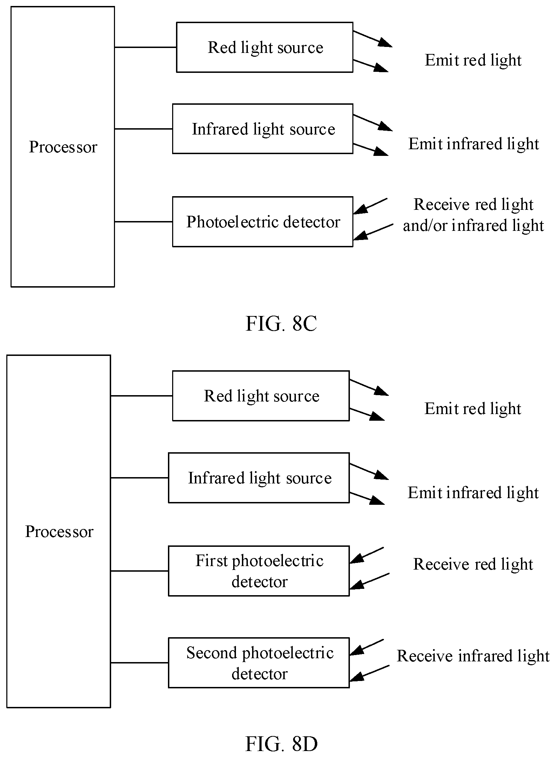

For example, as shown in FIG. 8C, the PPG module includes a processor, a red light source, an infrared light source, and a photoelectric detector.

Different light needs to be emitted by different light sources.

The red light source may emit red light, and there may be one or more red light sources. In some embodiments, the infrared light source may be a light emitting apparatus such as a light emitting diode.

The infrared light source may emit infrared light, and there may be one or more infrared light sources. In some embodiments, the infrared light source may be a light emitting apparatus such as a light emitting diode.

Different light may be received by a same photoelectric detector. In some embodiments, the photoelectric detector may be a receiving apparatus such as a photodiode (photodiode, PD).

For example, when the red light source emits red light, the photoelectric detector may receive reflected red light.

When the infrared light source emits infrared light, the photoelectric detector may receive reflected infrared light.

The processor may obtain a PPG signal based on the reflected red light and/or the reflected infrared light collected by the photoelectric detector.

For example, as shown in FIG. 8D, the PPG module includes a processor, a red light source, an infrared light source, a first photoelectric detector, and a second photoelectric detector.

Different light needs to be emitted by different light sources.

The red light source may emit red light, and there may be one or more red light sources. In some embodiments, the infrared light source may be a light emitting apparatus such as a light emitting diode.

The infrared light source may emit infrared light, and there may be one or more infrared light sources. In some embodiments, the infrared light source may be a light emitting apparatus such as a light emitting diode.

Different light may be received by a same photoelectric detector, and different light sources are received through time division multiplexing. In some embodiments, the photoelectric detector may be a receiving apparatus such as a photodiode (photodiode, PD).

For example, when the red light source emits red light, the first photoelectric detector may receive reflected red light.

When the infrared light source emits infrared light, the second photoelectric detector may receive reflected infrared light.

The processor may obtain a PPG signal based on the reflected red light collected by the first photoelectric detector and/or the reflected infrared light collected by the second photoelectric detector.

FIG. 8A to FIG. 8D are diagrams of structures of several PPG modules. FIG. 8A to FIG. 8D are also merely used to explain this application, but should not constitute a limitation.

III. A wearable device 100 monitors a sleep event, and measures blood pressure based on a detected abnormal sleep event.

### 1. The wearable device 100 detects that a user is in a sleep state.

In some embodiments, the wearable device 100 monitors the sleep event only after the wearable device 100 detects that the user is in the sleep state. When it is detected that the user is not in the sleep state, the wearable device 100 may not monitor the sleep event, to reduce power consumption of the wearable device 100.

In another embodiment, the wearable device 100 may alternatively monitor the sleep event in real time/periodically/aperiodically. This is not limited in this application either.

Optionally, the wearable device 100 may determine, based on motion data collected by a preset motion sensor, whether the user is in the sleep state.

The motion sensor may include but is not limited to an acceleration sensor, a gyro sensor, and the like. The motion sensor may collect the motion data or a motion trajectory. The wearable device 100 may determine, based on the motion data or the motion trajectory, whether the user is in the sleep state.

Usually, the user is in a sleep state, and the user has almost no activity, and only has a small amount of motion such as turning over. The motion sensor collects a small amount of motion data, or a motion trajectory that is of the wearable device 100 and that is collected by the motion sensor is consistent with a motion trajectory of a wrist during sleep. The wearable device 100 may determine, based on this, whether the user is in the sleep state.

Optionally, the wearable device 100 may alternatively determine, based on information such as a heart rate collected by a PPG sensor, whether the user is in the sleep state.

Usually, the user is in the sleep state, the user has almost no activity, a heart rate of the user is stable, and a heart rate of the user in the sleep state is lower than a heart rate of the user in an awake state. The wearable device 100 may determine, based on this, whether the user is in the sleep state. In addition to information such as a heart rate collected by a PPG module, whether the user is in the sleep state may be determined based on information such as a heart rate collected by another component. This is not limited in this application.

Optionally, the wearable device 100 may alternatively determine, based on a time, whether the user is in the sleep state.

Usually, the user is in the sleep state between 12:00 at night and 6:00 in the morning. The wearable device 100 may determine, based on this time period, whether the user is in the sleep state.

In some embodiments, the PPG module may alternatively be replaced with another component. In this application, only the PPG module is used as an example for description. This is not limited in this application.

In some embodiments, the motion sensor may alternatively be replaced with another component.

In this application, only the motion sensor is used as an example for description. This is not limited in this application.

In some embodiments, in addition to the PPG module and the motion sensor, the wearable device may further determine, by collecting an ambient sound and a speaking sound of a person by a microphone, whether the user is asleep. Usually, a surrounding environment is quiet, and the user hardly speaks after falling asleep. When intensity of the ambient sound is less than preset intensity and/or the speaking sound of the person is less than the preset intensity, it may be determined that the user is in a sleep state.

In some embodiments, the wearable device may further determine, based on ambient luminance collected by an optical sensor, whether the user is asleep. Usually, ambient light when the user falls asleep is dark. When the ambient luminance collected by the optical sensor is less than preset luminance, it may be determined that the user is in the sleep state.

In some embodiments, the wearable device may further determine, based on a signal such as an EMG electromyography signal, an EEG electroencephalography signal, or a GSR galvanic skin response signal collected by an electrode, whether the user is asleep. When the EMG electromyography signal, the EEG electroencephalography signal, and the GSR galvanic skin response signal meet a preset condition, it may be determined that the user is in the sleep state. The wearable device 100 may alternatively determine, based on one or more other conditions, whether the user is in the sleep state. This is not limited in this application either.

The foregoing one or more manners of determining whether the user is asleep may be used independently to determine whether the user is asleep, or two or more manners may be used together to determine whether the user is asleep. This is not limited in this application.

### 2. The wearable device 100 detects the sleep event, and measures user blood pressure.

After determining that the user is in the sleep state, and after the wearable device 100 detects the sleep event, the wearable device 100 may measure the user blood pressure.

Optionally, the wearable device 100 may stop measurement after measuring blood pressure a single time. Alternatively, the wearable device 100 may stop measurement after measuring blood pressure a plurality of times based on a blood pressure result.

Optionally, after determining that the user is in the sleep state, and after the wearable device 100 detects that the quantity of occurrence times of the sleep event is greater than a preset quantity of times, the wearable device 100 starts to measure the user blood pressure.

In some embodiments, after determining that the user is in the sleep state, the wearable device 100 may measure the user blood pressure after detecting an abnormal event that causes risen blood pressure. The abnormal event that causes risen blood pressure may include but is not limited to an OSA event, a REM sleep event, and an elevated central sympathetic nerve activity event. Optionally, for the OSA event, when the wearable device 100 determines, based on one or more of a PPG signal collected by the PPG module, personal data of the user, and the like and based on the PPG signal and the personal data of the user, that the PPG signal meets a preset condition, the wearable device 100 may determine that the OSA event occurs. The PPG signal may include but is not limited to a heart rate, blood oxygen, a respiratory rate, and the like. The personal data of the user includes but is not limited to a height, a weight, an age, and a gender.

In some embodiments, the wearable device 100 determines, based on a past period of time, that the user frequently encounters the OSA event at night. After the wearable device 100 detects that the quantity of occurrence times of the sleep event is greater than the preset quantity of times, the wearable device 100 may determine that the user frequently encounters the OSA event. In this case, the wearable device 100 may not measure the user blood pressure, to avoid affecting rest of the user.

Optionally, for the REM sleep event, when the wearable device 100 determines, based on one or more of a PPG signal collected by the PPG module, personal data of the user, and the like and based on the PPG signal and the personal data of the user, that the PPG signal meets a preset condition, the wearable device 100 may determine that the REM sleep event occurs. The PPG signal may include but is not limited to a heart rate, a respiratory rate, and the like. The personal data of the user includes but is not limited to a height, a weight, an age, and a gender. Optionally, for the elevated central sympathetic nerve activity event, when the wearable device 100 determines, based on one or more of a PPG signal collected by the PPG module, personal data of the user, and the like and based on the PPG signal and the personal data of the user, that the PPG signal meets a preset condition, the wearable device 100 may determine that the elevated central sympathetic nerve activity event occurs. The PPG signal may include but is not limited to a heart rate, blood oxygen, a respiratory rate, and the like. The personal data of the user includes but is not limited to a height, a weight, an age, and a gender.

Specifically, the wearable device 100 may determine a quantity of nocturnal awakenings, NREM sleep event duration, a REM sleep event latency, and the like based on the PPG signal and the personal data of the user. When the quantity of awakenings of the user is greater than the preset quantity of times, the NREM sleep event duration is less than a first value, and duration of the REM sleep event latency is less than a second value, the wearable device 100 may determine that the elevated central sympathetic nerve activity event occurs.

In some embodiments, after determining that the user is in the sleep state, the wearable device 100 may measure the user blood pressure after detecting an abnormal event that causes dropped blood pressure. The abnormal event that causes dropped blood pressure may include but is not limited to an NREM sleep event.

Optionally, for the NREM sleep event, when the wearable device 100 determines, based on one or more of a PPG signal collected by the PPG module, personal data of the user, and the like and based on the PPG signal and the personal data of the user, that the PPG signal meets a preset condition, the wearable device 100 may determine that the REM sleep event occurs. The PPG signal may include but is not limited to a heart rate, a respiratory rate, and the like. The personal data of the user includes but is not limited to a height, a weight, an age, and a gender. Optionally, the NREM sleep event may cause dropped blood pressure of a hypertensive user, and the dropped blood pressure may cause myocardial ischemia. When myocardial ischemia occurs, the heart rate of the user is usually lower than a preset heart rate (for example, 83 times/minute). Therefore, after determining that the user is in the sleep state, the wearable device 100 may measure the user blood pressure when the wearable device 100 detects the NREM sleep event and the heart rate is lower than the preset heart rate (for example, 83 times/minute). When the heart rate is higher than the preset heart rate, the wearable device 100 may not measure the user blood pressure, to avoid affecting rest of the user.

In some embodiments, after determining that the user is in the sleep state, the wearable device 100 may measure the user blood pressure after detecting both an abnormal event that causes risen blood pressure and an abnormal event that causes dropped blood pressure. The abnormal event that causes risen blood pressure may include but is not limited to an OSA event, a REM sleep event, and an elevated central sympathetic nerve activity event. The abnormal event that causes dropped blood pressure may include but is not limited to an NREM sleep event.

In addition to a PPG signal collected by the PPG module, the OSA event, the REM event, the NREM sleep event, and the elevated central sympathetic nerve activity event may be determined based on a signal collected by another component, for example, based on information such as an EMG electromyography signal, an EEG electroencephalography signal, and a GSR galvanic skin response signal collected by an electrode. This is not limited in this application.

In some embodiments, that the wearable device 100 measures the blood pressure is a complete inflation-deflation process, and may mean that the wearable device 100 inflates an airbag, so that a barometric pressure value in the airbag reaches a first barometric pressure value, and then slowly deflates the airbag, so that the pressure value in the airbag gradually decreases from the first barometric pressure value to 0; and measures the user blood pressure in a deflation process; or may mean that the wearable device 100 inflates the airbag, so that a barometric pressure value in the airbag gradually reaches a first barometric pressure value from 0; and measures the user blood pressure in an inflation process. However, in the inflation-deflation process of the wearable device 100, the user clearly feels pressure on a wrist, which may affect sleep of the user. Based on the foregoing analysis, the wearable device 100 may obtain the correspondence between airbag pressure and blood pressure. When the wearable device 100 measures the user blood pressure through airbag inflation and deflation, the wearable device 100 may not need to perform inflation until the barometric pressure value in the airbag reaches the first barometric pressure value, and the wearable device 100 only needs to perform inflation until the barometric pressure value in the airbag reaches a second barometric pressure value. The second barometric pressure value is less than the first barometric pressure value, or the second barometric pressure value is far less than the first barometric pressure value. The wearable device 100 may obtain a real-time blood pressure value based on the second barometric pressure value and the correspondence between airbag pressure and blood pressure. Compared with a case in which the barometric pressure value in the airbag reaches the first barometric pressure value through inflation, when the barometric pressure value in the airbag reaches the second barometric pressure value through inflation, interference to the user in the airbag inflation-deflation process can be reduced, thereby reducing user sensing and improving user experience.

In another possible implementation, the wearable device 100 may alternatively determine the user blood pressure by measuring the PPG signal. Based on the foregoing analysis, the wearable device 100 may obtain the correspondence between a PPG signal and blood pressure. The wearable device 100 may obtain a real-time PPG signal through measurement by using the PPG module, and the wearable device 100 may obtain a real-time blood pressure value based on the real-time PPG signal and the correspondence between a PPG signal and blood pressure. In this way, the user blood pressure is obtained by measuring the PPG signal, and the user senses nothing throughout the process, thereby improving user experience.

### 3. After detecting abnormal blood pressure, the wearable device 100 may prompt, in an alarm manner, the user to pay attention to the abnormal blood pressure.

Usually, diastolic pressure and systolic pressure of the user are within a normal range. For example, a normal diastolic pressure range is between 60 mmHg and 90 mmHg, and a normal systolic pressure range is between 90 mmHg and 140 mmHg.

The abnormal blood pressure may include abnormal diastolic pressure and abnormal systolic pressure.

The abnormal diastolic pressure may mean that real-time diastolic pressure obtained by the wearable device 100 through measurement is greater than a maximum value of normal diastolic pressure and exceeds a first threshold, or real-time diastolic pressure obtained by the wearable device 100 through measurement is less than a minimum value of normal diastolic pressure and is less than a first threshold.

The abnormal systolic pressure may mean that real-time systolic pressure obtained by the wearable device 100 through measurement is less than a minimum value of normal diastolic pressure and is less than a second threshold, or real-time systolic pressure obtained by the wearable device 100 through measurement is greater than a maximum value of normal systolic pressure and exceeds a second threshold.

In a possible implementation, after the wearable device 100 detects the abnormal blood pressure, or continuously detects the abnormal blood pressure first duration, or detects the abnormal blood pressure M consecutive times, the wearable device 100 may wake up, in one or more manners such as vibration, voice prompt, and light blinking, the user to prompt the user to pay attention to the abnormal blood pressure.

In another possible implementation, after the wearable device 100 detects the abnormal blood pressure, or continuously detects the abnormal blood pressure first duration, or detects the abnormal blood pressure M consecutive times, the wearable device 100 may wake up, in one or more manners such as vibration, voice prompt, and light blinking by using an electronic device that establishes a communication connection to the wearable device 100, the user to prompt the user to pay attention to the abnormal blood pressure.

In another possible implementation, after the wearable device 100 detects the abnormal blood pressure, or continuously detects the abnormal blood pressure first duration, or detects the abnormal blood pressure M consecutive times, the wearable device 100 may send a first message to a device (for example, an electronic device 200) of a previously bound relative. The first message is used by the electronic device 200 to notify the user that current blood pressure of a user who wears the wearable device 100 is abnormal, and notify the user to pay attention to blood pressure of the user who wears the wearable device 100.

### 4. The wearable device 100 stops measuring the user blood pressure.

The wearable device 100 may receive a user operation and stop measuring the user blood pressure, or the wearable device 100 may automatically stop measuring the user blood pressure.

In some embodiments, after detecting the sleep event, the wearable device 100 may continuously monitor the user blood pressure until the sleep event disappears, and the wearable device 100 stops measuring the user blood pressure.

In another embodiment, after the sleep event is detected, each time the sleep event occurs, the wearable device 100 measures the user blood pressure once, until a quantity of occurrence times of the sleep event reaches a maximum value, and the wearable device 100 stops measuring the user blood pressure.

In another embodiment, after detecting the sleep event, the wearable device 100 may continuously monitor the user blood pressure within the first duration. After the first duration passes, the wearable device 100 stops measuring the user blood pressure regardless of whether the sleep event disappears.

In some embodiments, after detecting the sleep event, the wearable device 100 may continuously monitor the user blood pressure. Until the user blood pressure returns to normal, the wearable device 100 stops measuring the user blood pressure.

Optionally, after the user blood pressure returns to normal, the sleep event may continue occurring or may stop occurring. This is not limited in this application.

Alternatively, the wearable device 100 may automatically stop measuring the user blood pressure based on another condition. This is not limited in this application.

### 5. The wearable device 100 displays a blood pressure measurement result.

In some embodiments, after the wearable device 100 detects that the user switches from a sleep state to an awake state, the wearable device 100 may display a nocturnal blood pressure measurement result, to help the user view the nocturnal blood pressure.

Optionally, after determining that the user switches from the sleep state to the awake state, the wearable device 100 automatically displays a nocturnal blood pressure measurement result; or the wearable device 100 may receive a user operation and display a nocturnal blood pressure measurement result. This is not limited in this application.

Optionally, the wearable device 100 may determine, based on motion data collected by a preset motion sensor, whether the user is in the awake state.

The motion sensor may include but is not limited to an acceleration sensor, a gyro sensor, and the like. The motion sensor may collect the motion data or a motion trajectory. The wearable device 100 may determine, based on the motion data or the motion trajectory, whether the user is in the awake state.

Usually, the user is in the awake state, and the user has a large activity amplitude, for example, in a motion such as walking. The motion sensor collects a large amount of motion data, or a motion trajectory that is of the wearable device 100 and that is collected by the motion sensor is consistent with a motion trajectory of a wrist in the awake state. The wearable device 100 may determine, based on this, whether the user is in the awake state.

Optionally, the wearable device 100 may alternatively determine, based on information such as a heart rate collected by a PPG sensor, whether the user is in the awake state.

Usually, the user is in the awake state, and the user has a large activity amplitude. A heart rate of the user in the sleep state is higher than a heart rate of the user in the sleep state, and a fluctuation is large. The wearable device 100 may determine, based on this, whether the user is in the awake state.

Optionally, the wearable device 100 may alternatively determine, based on a time, whether the user is in the awake state.

Usually, the user is in the awake state at about 6:00 a.m., and the wearable device 100 may determine, based on this time period, whether the user is in the awake state.

The wearable device 100 may alternatively determine, based on one or more other conditions, whether the user is in the awake state. This is not limited in this application either.

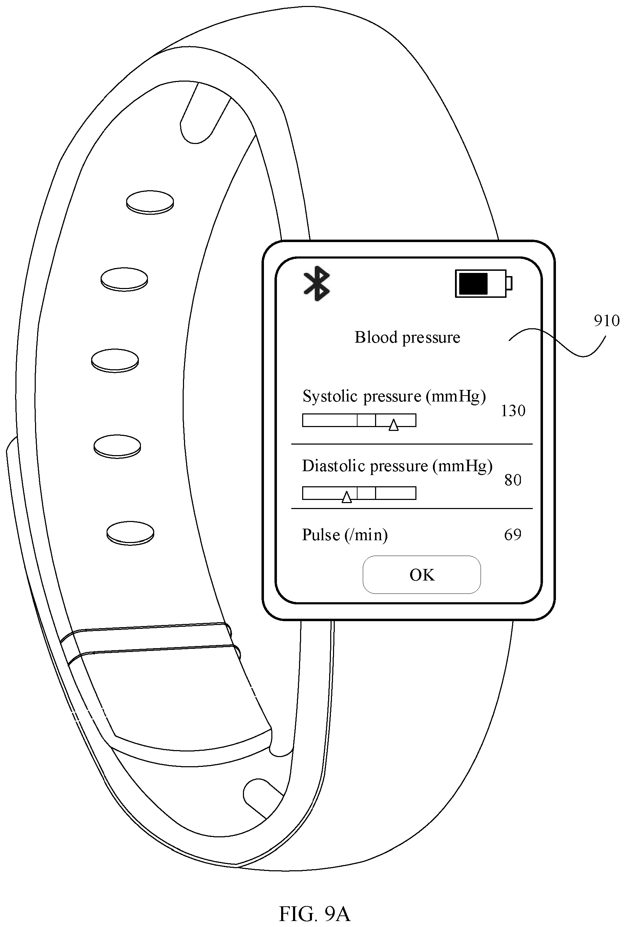

FIG. 9A to FIG. 9I are diagrams in which a wearable device 100 displays a blood pressure measurement result.

In some embodiments, after the wearable device 100 obtains the nocturnal blood pressure and the wearable device 100 obtains the blood pressure monitoring value, the wearable device 100 may display a user interface 910 shown in FIG. 9A. The user interface 910 includes the blood pressure measurement value. The blood pressure measurement value may include systolic pressure and diastolic pressure. For example, the systolic pressure may be 130 mmHg, and the diastolic pressure may be 80 mmHg. In some embodiments, the user interface 910 may further include a pulse. For example, the pulse may be 69 times per minute. The systolic pressure displayed in the user interface 910 may be nocturnal average systolic pressure, and the diastolic pressure displayed in the user interface 910 may be nocturnal average diastolic pressure.

In another embodiment, after the wearable device 100 obtains the nocturnal blood pressure, the wearable device 100 may send the nocturnal blood pressure to another electronic device that establishes a communication connection to the wearable device 100, and the another electronic device displays the nocturnal blood pressure.

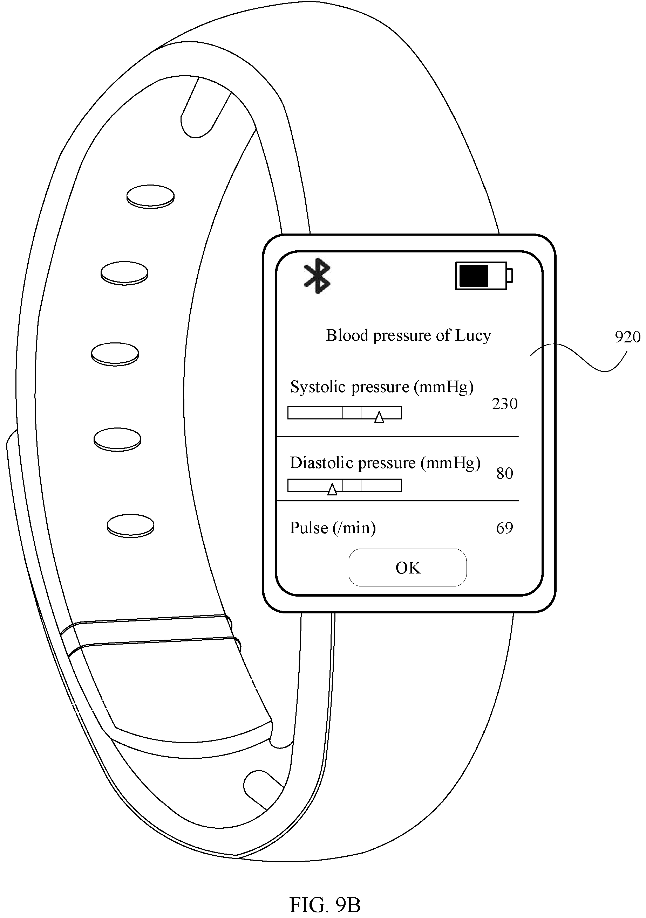

In some embodiments, after the wearable device 100 obtains the nocturnal blood pressure, the wearable device 100 may display a user interface 920 shown in FIG. 9B. The user interface 920 is similar to the user interface 910, and a difference lies in that the user interface 920 includes a nickname of a detected person, for example, "Nocturnal blood pressure of Lucy", to notify the user of a user associated with a current nocturnal blood pressure monitoring value. The wearable device 100 may further store blood pressure monitoring values of different users separately, to view nocturnal blood pressure of a same user in a specific time period.

In some embodiments, the wearable device 100 may display nocturnal blood pressure corresponding to the sleep event, so that the user can view nocturnal blood pressure corresponding to different sleep events.

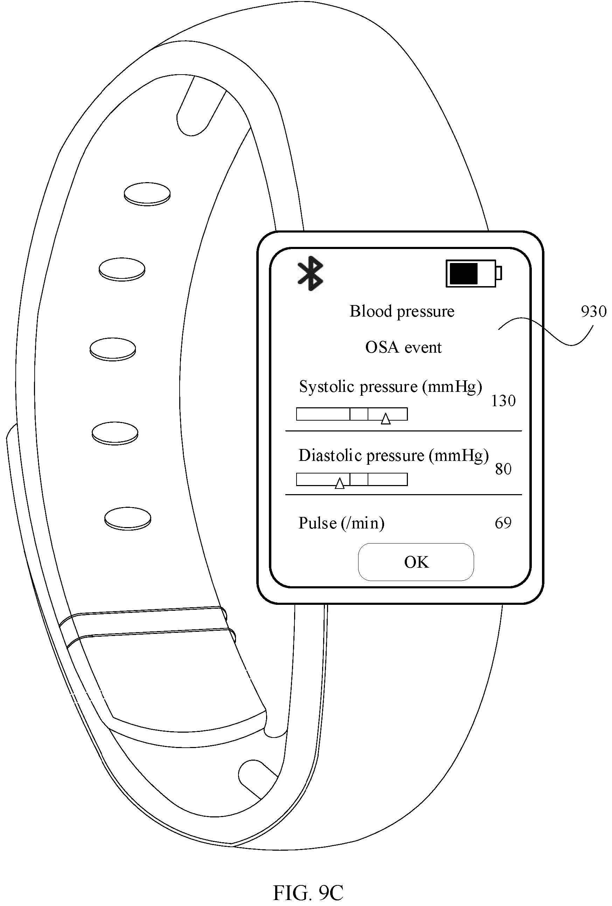

For example, as shown in FIG. 9C, the wearable device 100 may display a user interface 930. The user interface 930 is similar to the user interface 910, and a difference lies in that the user interface 930 includes a sleep event identifier, for example, an "OSA event" identifier, to notify the user of a sleep event associated with current nocturnal blood pressure. When the OSA event shown in the user interface 930 occurs, an average systolic pressure measured by the wearable device 100 is 130 mmHg, an average diastolic pressure measured by the wearable device 100 is 80 mmHg, and a pulse measured by the wearable device 100 is 69 times per minute.

Optionally, after the wearable device 100 determines that the user switches from the sleep state to the awake state, if the OSA event is detected at a previous night, the OSA event may cause risen daytime blood pressure. The wearable device 100 may continuously monitor daytime user blood pressure and user nocturnal blood pressure at a next night that is adjacent. Optionally, the wearable device 100 may alternatively notify the user of an OSA event that is detected at a previous night, and continuously monitor daytime user blood pressure and user nocturnal blood pressure at a next night that is adjacent.

Optionally, the wearable device 100 may alternatively receive a user and view nocturnal blood pressure corresponding to another sleep event.

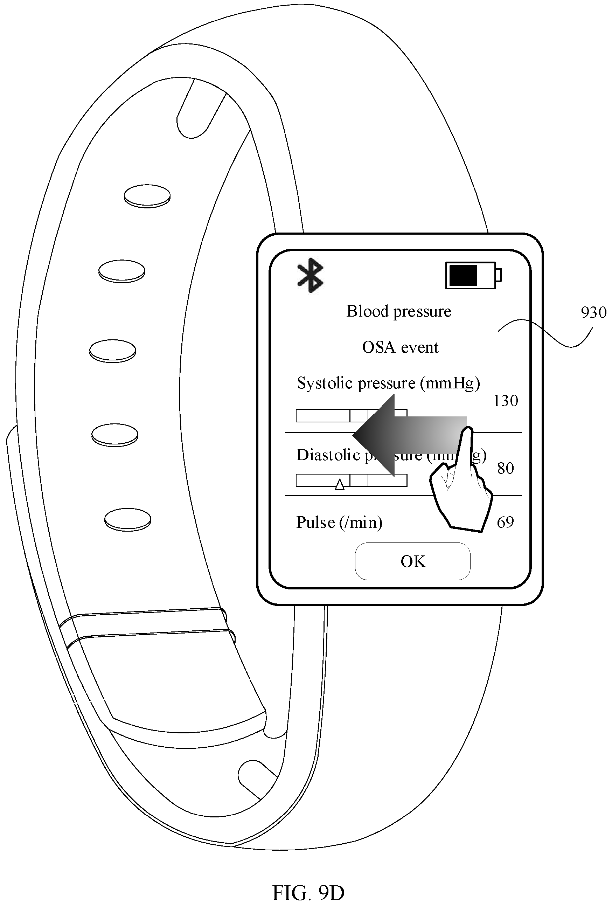

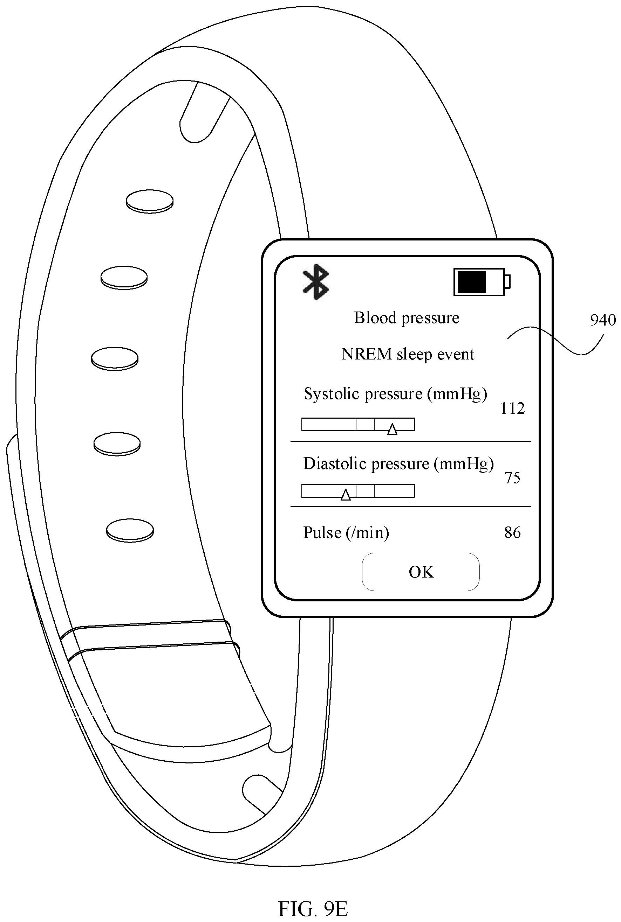

For example, as shown in FIG. 9D, the wearable device 100 may receive a slide operation (for example, a rightward slide operation) performed by the user in the user interface 930. In response to the slide operation performed by the user, the wearable device 100 may display a user interface 940 shown in FIG. 9E. The user interface 940 is similar to the user interface 910, and a difference lies in that the user interface 940 includes a sleep event identifier, for example, an "NREM sleep event" identifier, to notify the user of a sleep event associated with current nocturnal blood pressure. When the NREM sleep event shown in the user interface 940 occurs, an average systolic pressure measured by the wearable device 100 is 112 mmHg, an average diastolic pressure measured by the wearable device 100 is 75 mmHg, and a pulse measured by the wearable device 100 is 86 times per minute.

In addition to the nocturnal blood pressure corresponding to the OSA event and the nocturnal blood pressure corresponding to the NREM sleep event, the user may switch to view nocturnal blood pressure corresponding to another sleep event, for example, nocturnal blood pressure corresponding to the REM sleep event and nocturnal blood pressure corresponding to the elevated central sympathetic nerve activity event. This is not limited in this application either.

In some embodiments, the wearable device 100 may store nocturnal blood pressure within a first time period (for example, seven days). The nocturnal blood pressure at a time point that is the first time period ago is deleted, to save storage space of the wearable device 100.

Optionally, the wearable device 100 may alternatively receive a user operation and view nocturnal blood pressure of the user in the first time period.

For example, the first time period may be seven days.

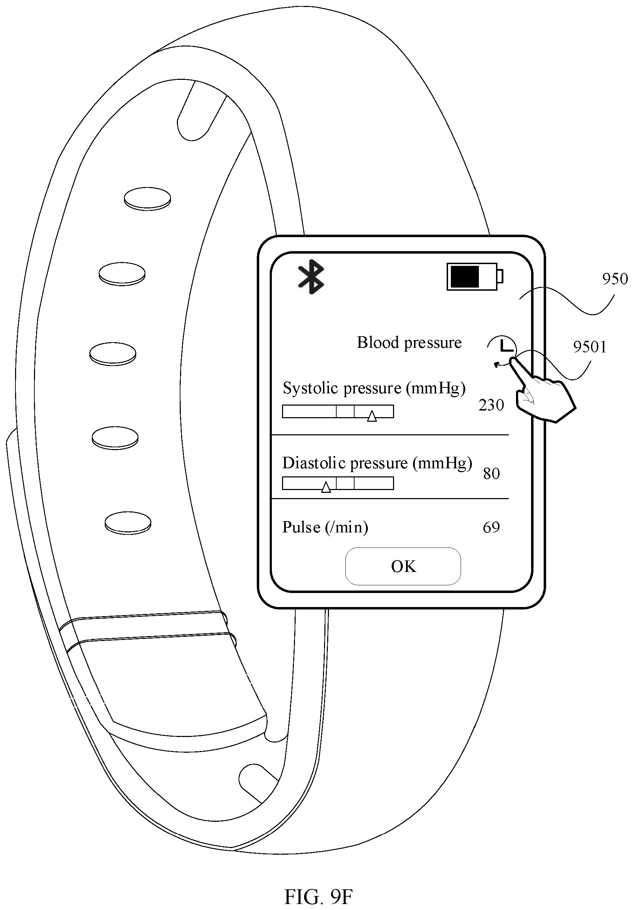

As shown in FIG. 9F, after the wearable device 100 obtains the blood pressure monitoring value, the wearable device 100 may display a user interface 950 shown in FIG. 9F. The user interface 950 is similar to the user interface 910, and A difference lies in that the user interface 950 further includes an icon 9501, and the icon 9501 is configured to display nocturnal blood pressure within seven days.

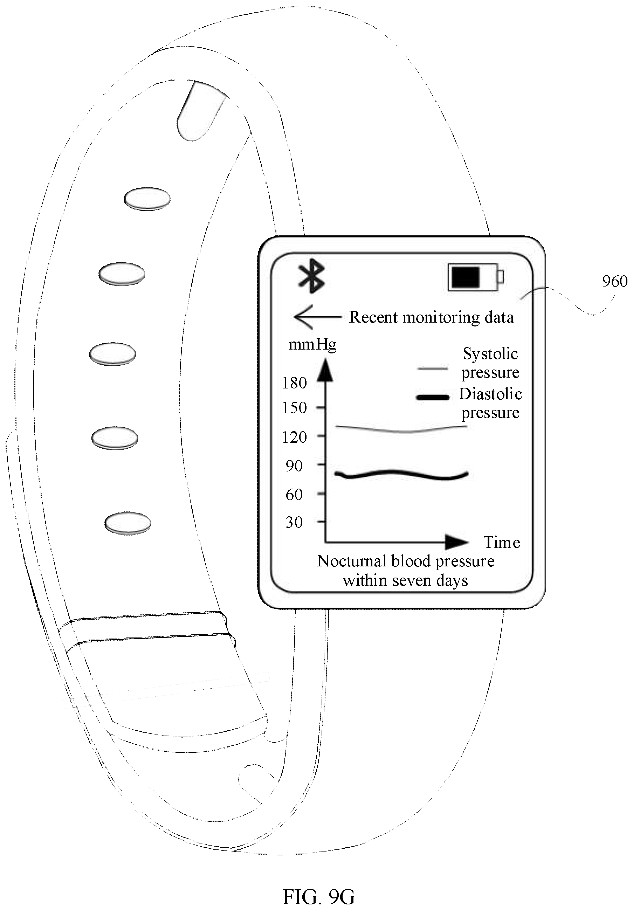

The wearable device 100 may receive an input operation (for example, a tap) performed by the user on the icon 9501 in the user interface 950, and in response to the input operation performed by the user, the wearable device 100 may obtain a blood pressure monitoring value of the user in a specific time period, and display a user interface 960 shown in FIG. 9G.

The user interface 960 includes a chart display area of the nocturnal blood pressure within seven days. The chart display area includes a systolic pressure monitoring value curve within seven days and a diastolic pressure monitoring value curve within seven days. A nocturnal blood pressure change trend of the user within seven days can be intuitively viewed in the chart display area.

In addition to seven days, the wearable device 100 may further display nocturnal blood pressure in another longer or shorter time period. This is not limited in this application either.

In some embodiments, the wearable device 100 may alternatively receive a user operation and view nocturnal blood pressure of different users in the first time period.

With reference to the descriptions in FIG. 9F, after the wearable device 100 obtains the blood pressure monitoring value, the wearable device 100 may display the user interface 950 shown in FIG. 9F.

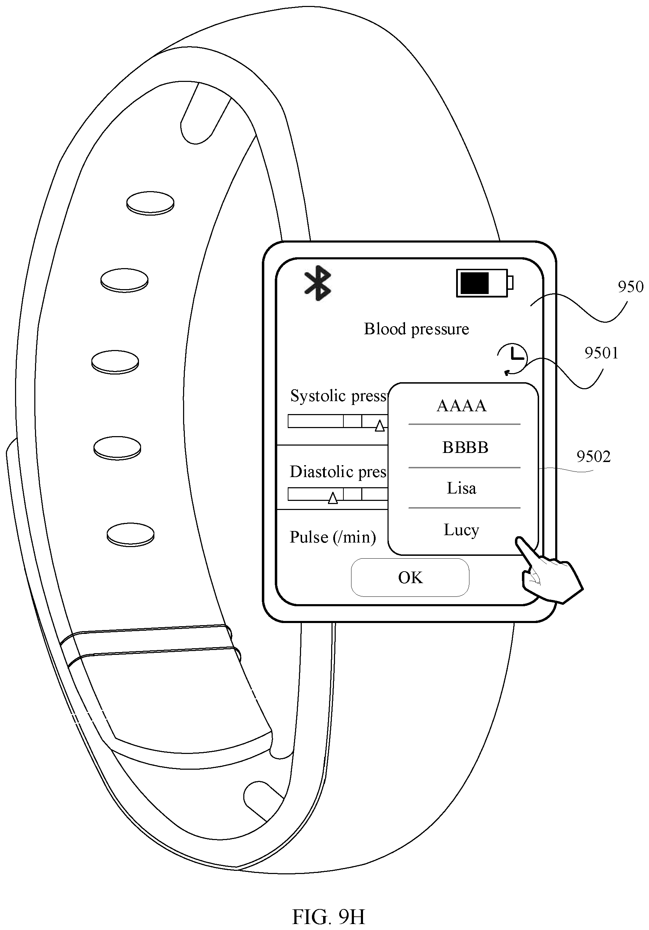

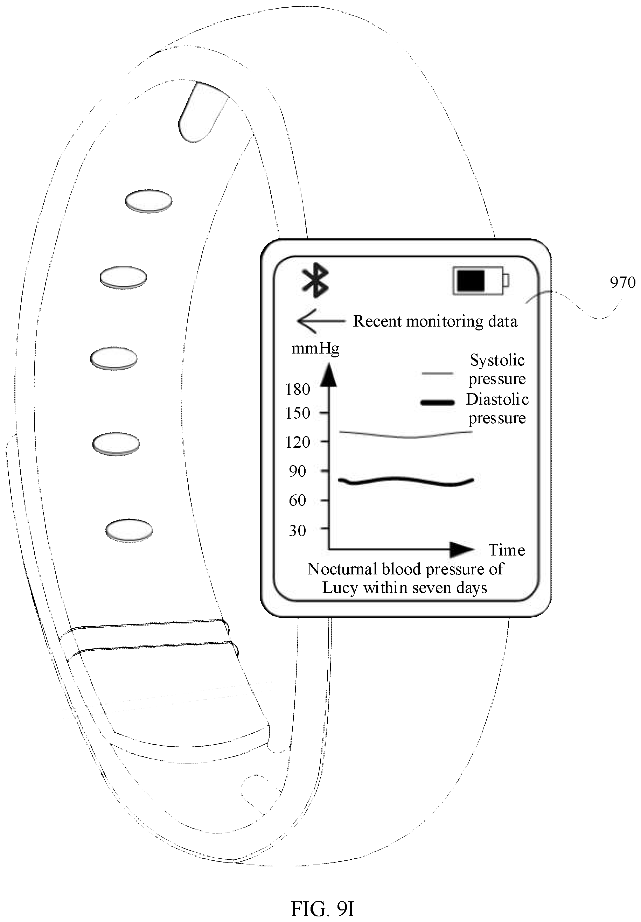

The wearable device 100 may receive an input operation performed by the user on the icon 9501 in the user interface 950, and in response to the input operation performed by the user, the wearable device 100 may display a selection bar 9502 shown in FIG. 9H. Options of a plurality of different monitoring objects are shown in the selection bar 9502. For example, the plurality of monitoring objects include but are not limited to a monitoring object "AAAA", a monitoring object "BBBB", a monitoring object "Lisa", and a monitoring object "Lucy". The user may select any monitoring object and start to view nocturnal blood pressure of the monitoring object in the first time period. As shown in FIG. 9H, the wearable device 100 may receive an input operation (for example, a tap) performed by the user on the monitoring object "Lucy" option in the selection bar 9502, and in response to the input operation performed by the user, the wearable device 100 may obtain a blood pressure monitoring value of the monitoring object "Lucy" in the first time period, and display a user interface 970 shown in FIG. 9I. The user interface 970 is similar to the user interface 960, and a difference lies in that the user interface 970 includes prompt information "Nocturnal blood pressure of Lucy within seven days", to prompt that the systolic pressure monitoring value curve and the diastolic pressure monitoring value curve that are shown in the user interface 970 are the nocturnal blood pressure of the monitoring object "Lucy" within seven days.

In the method, the wearable device 100 may not only display nocturnal blood pressure of a local user in the first time period, but also display nocturnal blood pressure of another user in the first time period.

In some embodiments, nocturnal blood pressure that is of different users in the first time period and that is stored in the wearable device 100 may be nocturnal blood pressure that is of different users in the first time period and that is collected by the wearable device 100, sent to the wearable device 100, and stored in the wearable device 100.

In another embodiment, the nocturnal blood pressure that is of different users in the first time period and that is stored in the wearable device 100 may be sent by another electronic device to the wearable device 100 periodically/aperiodically/at a specific time interval. After a user of another electronic device obtains authorization of the user, the another electronic device may send stored nocturnal blood pressure of the user to the wearable device 100 periodically/aperiodically/at a specific time interval, so that the wearable device 100 may store nocturnal blood pressure of different users in the first time period, to help a user using the wearable device 100 view nocturnal blood pressure of different users of another authorized user in the first time period. For example, the another authorized user may be a family member of the user using the wearable device 100. For example, the another authorized user may be a parent, a child, or the like of the user using the wearable device 100. In this case, after the wearable device 100 obtains nocturnal blood pressure of the parent or the child in the first time period, the user using the wearable device 100 may view the nocturnal blood pressure of the another authorized user in the first time period, to facilitate monitoring of the nocturnal blood pressure of the family member.

In some embodiments, the user may alternatively record a dosing time and a medicine type in the wearable device 100. The wearable device 100 may prompt the user to be dosed in a timely manner based on the dosing time. Optionally, if the wearable device 100 detects that the user is dosed before bed, for example, is dosed with an antihypertensive, the wearable device 100 may automatically start to measure nocturnal blood pressure of the user, to observe impact of the antihypertensive on the nocturnal blood pressure of the user. The wearable device 100 may alternatively prompt the user to start to measure the nocturnal blood pressure of the user. After the user agrees, the wearable device 100 may monitor the nocturnal blood pressure of the user, to observe impact of the antihypertensive on the nocturnal blood pressure of the user.

In some embodiments, the wearable device 100 may alternatively measure daytime blood pressure of the user. After it is detected that the daytime blood pressure of the user is abnormal, the user is prompted to record a recent activity event of the user.

The abnormal daytime blood pressure may include abnormally high daytime blood pressure and abnormally low daytime blood pressure.

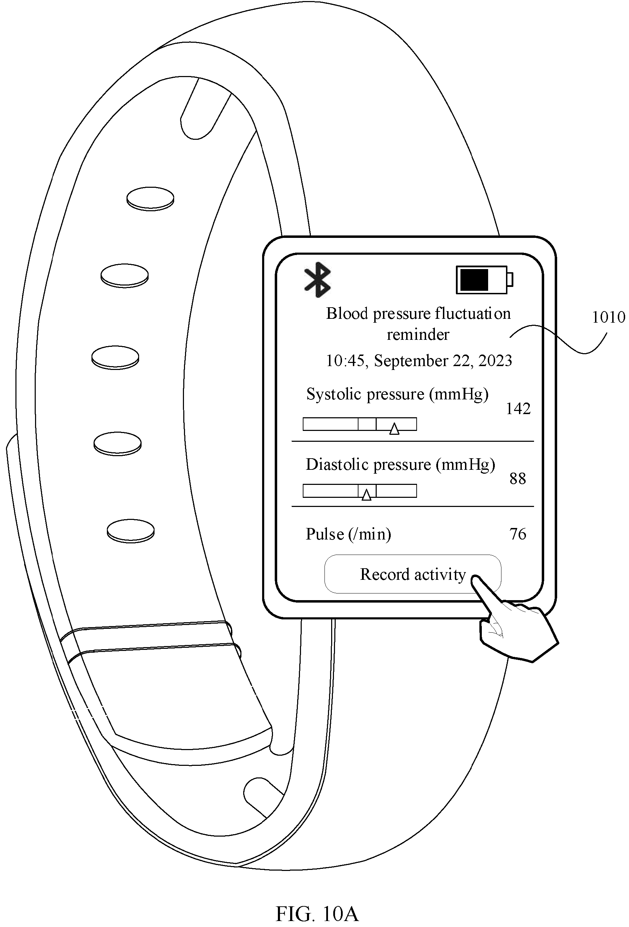

FIG. 10A to FIG. 10E are diagrams in which after detecting abnormally high daytime blood pressure, a wearable device 100 prompts a user to record a recently executed activity event.

In some embodiments, the wearable device 100 may determine, based on first M blood pressure monitoring values, that the daytime blood pressure is abnormally high.

In another embodiment, the wearable device 100 may determine, based on an average value of blood pressure monitoring values on a current day, that the daytime blood pressure is abnormally high.

In another embodiment, the wearable device 100 may determine, based on an average value of blood pressure monitoring values in first N days, that the daytime blood pressure is abnormally high.

For example, as shown in FIG. 10A, the wearable device 100 displays a user interface 1010. The user interface 1010 shows a blood pressure measurement value, and the blood pressure measurement value may include systolic pressure and diastolic pressure. For example, the systolic pressure may be 142 mmHg, the diastolic pressure may be 88 mmHg, and the pulse may be 76 times per minute. The user interface 1010 further includes a "Record activity" option, and the "Record activity" option is used by the user to record a recently executed activity event.

Optionally, the wearable device 100 may display the "Record activity" option in the user interface 1010 when identifying that the systolic pressure in the blood pressure monitoring value is abnormally high, and may not display the "Record activity" option in the user interface 1010 when identifying that the systolic pressure in the blood pressure monitoring value is within a normal range. Alternatively, the wearable device 100 may continuously display the "Record activity" option in the user interface 1010. This is not limited in this application.

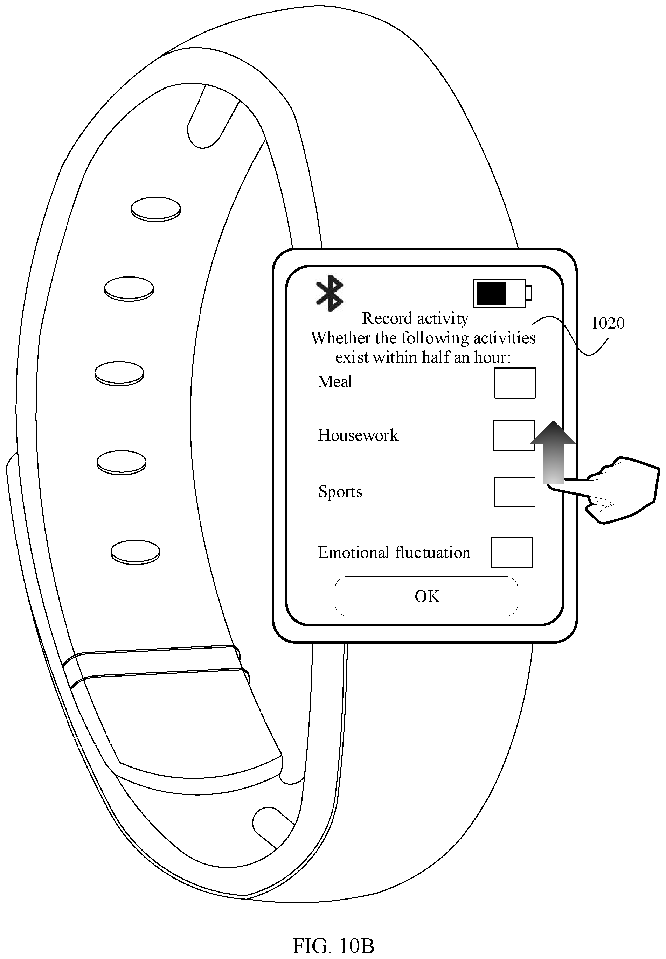

For example, as shown in FIG. 10A, the wearable device 100 may receive an input operation (for example, a tap) performed by the user on the "Record activity" option in the user interface 1010, and in response to the input operation performed by the user, the wearable device 100 may display a user interface 1020 shown in FIG. 10B. The user interface 1020 shows a plurality of different activity events such as a meal event, a housework event, a sports event, and an emotional fluctuation event. The user may alternatively slide the user interface 1020 to view more other activity events that are not displayed.

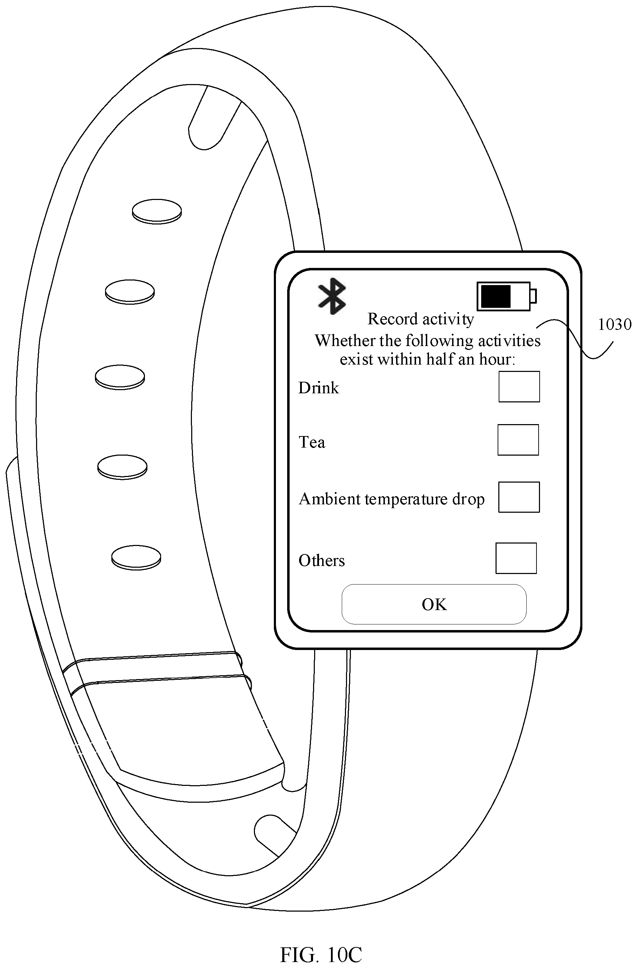

For example, as shown in FIG. 10B, the wearable device 100 may receive a slide operation (for example, an upward slide operation) performed by the user in the user interface 1020, and in response to the slide operation performed by the user, the wearable device 100 may display a user interface 1030 shown in FIG. 10C. The user interface 1030 shows other activity event such as a drink event, a tea event, a dropped ambient temperature event, and others event.

The user may select, in one or both of the user interface 1020 and the user interface 1030, one or more activity events that cause abnormally high user blood pressure.

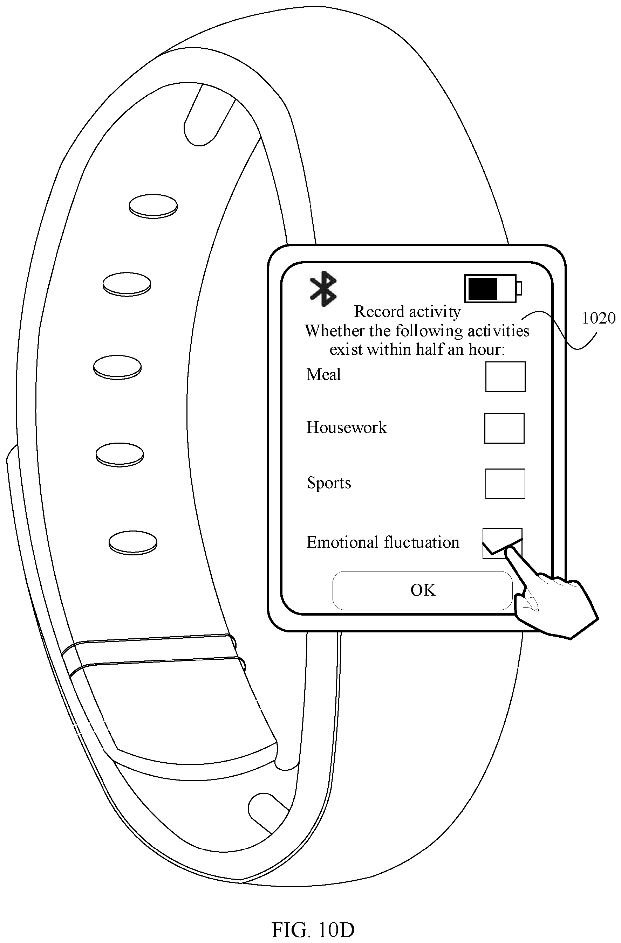

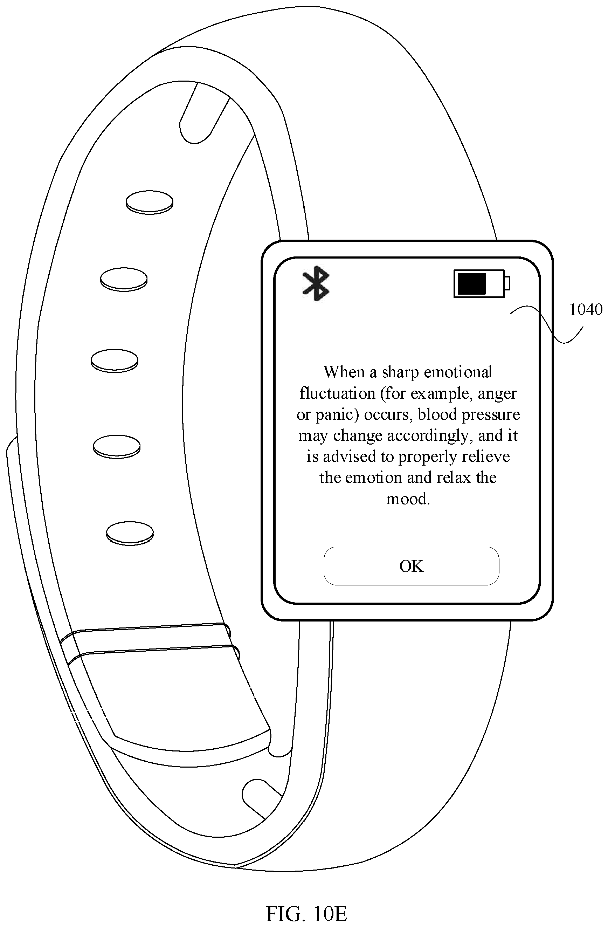

For example, as shown in FIG. 10D, the wearable device 100 may receive an input operation (for example, a tap) performed by the user on an "Emotional fluctuation event" option in the user interface 1020, and in response to the input operation performed by the user, the wearable device 100 may display a user interface 1040 shown in FIG. 10E. The user interface 1040 includes prompt information "When a sharp emotional fluctuation (for example, anger or panic) occurs, blood pressure may change accordingly, and it is advised to properly relieve the emotion and relax the mood.". The prompt information is used to prompt the user that a sharp emotional fluctuation causes risen blood pressure. The emotional fluctuation is avoided, to keep the blood pressure within a normal range.

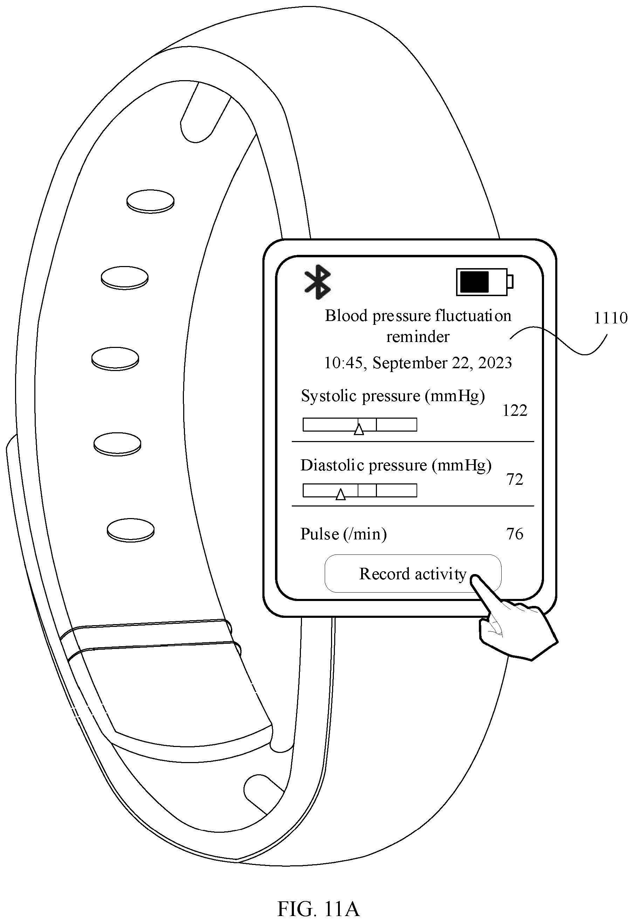

FIG. 11A to FIG. 11E are diagrams in which after detecting abnormally low daytime blood pressure, a wearable device 100 prompts a user to record a recently executed activity event.

In some embodiments, the wearable device 100 may determine, based on first M blood pressure monitoring values, that the daytime blood pressure is abnormally low.

In another embodiment, the wearable device 100 may determine, based on an average value of blood pressure monitoring values on a current day, that the daytime blood pressure is abnormally low.

In another embodiment, the wearable device 100 may determine, based on an average value of blood pressure monitoring values in first N days, that the daytime blood pressure is abnormally low.

For example, as shown in FIG. 11A, the wearable device 100 displays a user interface 1110. The user interface 1110 shows a blood pressure measurement value, and the blood pressure measurement value may include systolic pressure and diastolic pressure. For example, the systolic pressure may be 122 mmHg, the diastolic pressure may be 72 mmHg, and the pulse may be 76 times per minute. The user interface 1110 further includes a "Record activity" option, and the "Record activity" option is used by the user to record a recently executed activity event. Optionally, the wearable device 100 may display the "Record activity" option in the user interface 1110 when identifying that the diastolic pressure in the blood pressure monitoring value is abnormally low, and may not display the "Record activity" option in the user interface 1110 when identifying that the diastolic pressure in the blood pressure monitoring value is within a normal range. Alternatively, the wearable device 100 may continuously display the "Record activity" option in the user interface 1110. This is not limited in this application.

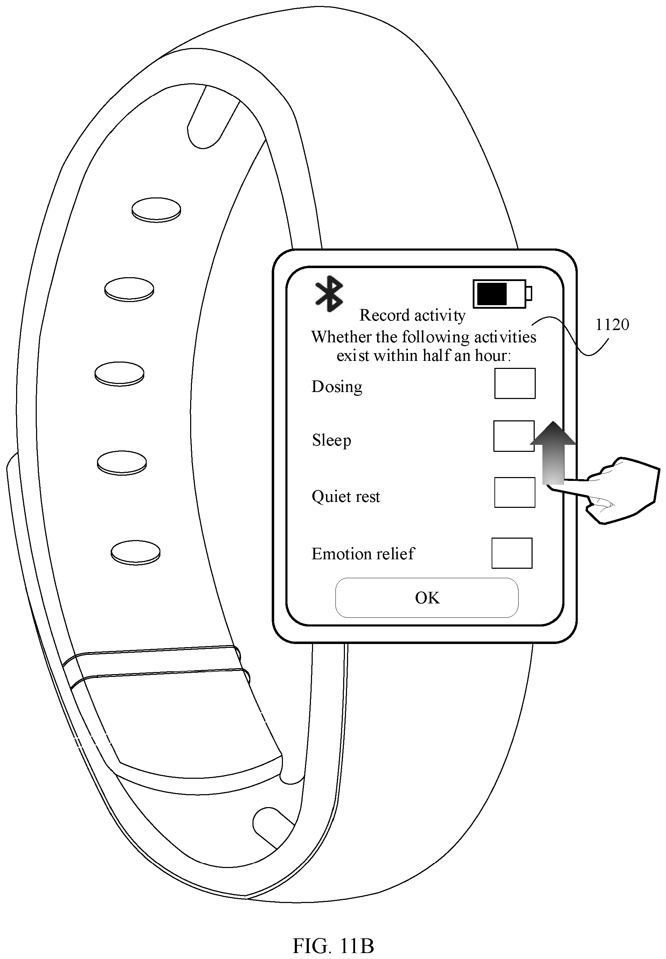

For example, as shown in FIG. 11A, the wearable device 100 may receive an input operation (for example, a tap) performed by the user on the "Record activity" option in the user interface 1110, and in response to the input operation performed by the user, the wearable device 100 may display a user interface 1120 shown in FIG. 11B. The user interface 1120 shows a plurality of different activity events such as a dosing event, a sleep event, a quiet rest event, and an emotion relief event. The user may alternatively slide the user interface 1120 to view more other activity events that are not displayed.

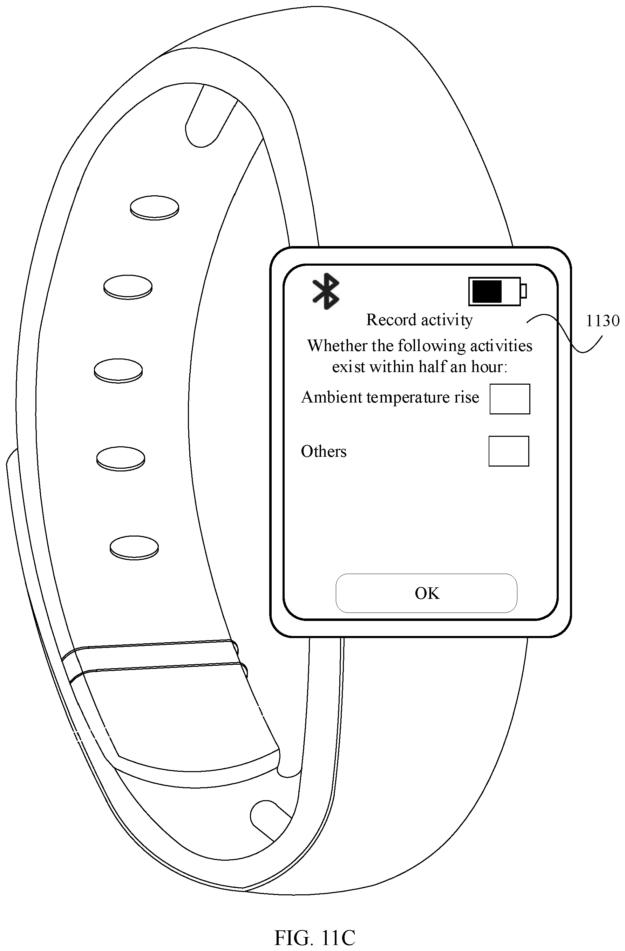

For example, as shown in FIG. 11B, the wearable device 100 may receive a slide operation (for example, an upward slide operation) performed by the user in the user interface 1120, and in response to the slide operation performed by the user, the wearable device 100 may display a user interface 1130 shown in FIG. 11C. The user interface 1130 shows other activity event such as a risen ambient temperature event and another event.

The user may select, in one or both of the user interface 1120 and the user interface 1130, one or more activity events that cause abnormally low user blood pressure.

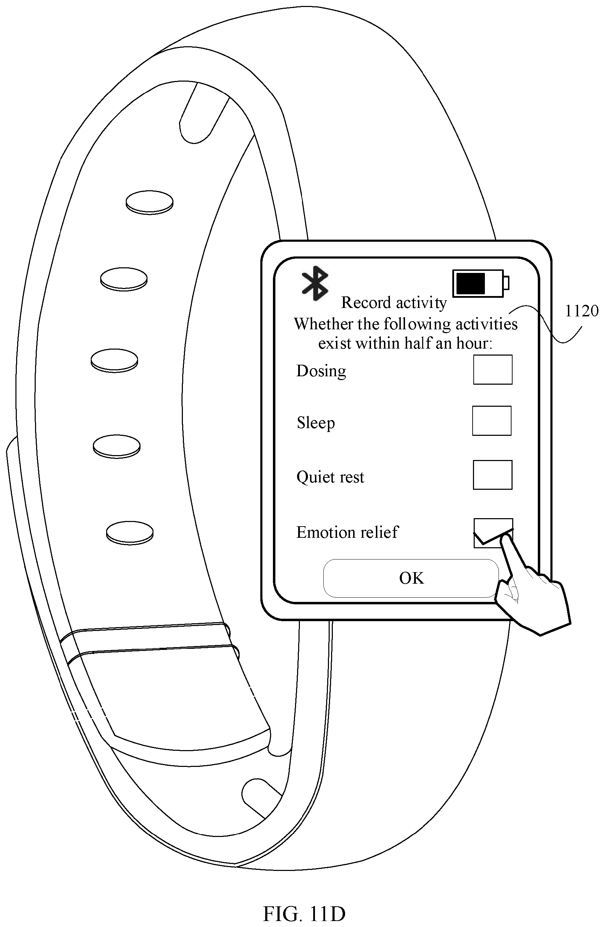

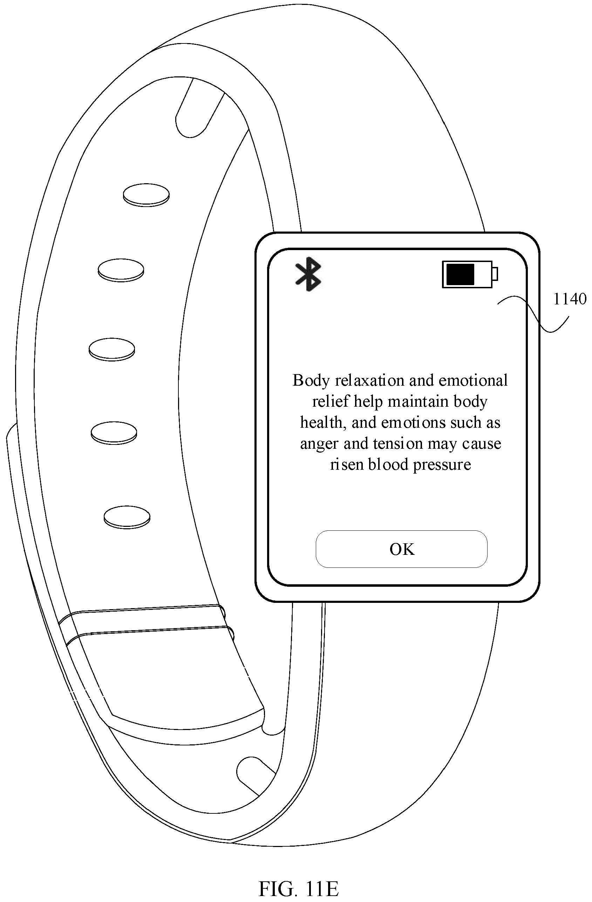

For example, as shown in FIG. 11D, the wearable device 100 may receive an input operation (for example, a tap) performed by the user on an "Emotion relief event" option in the user interface 1120, and in response to the input operation performed by the user, the wearable device 100 may display a user interface 1140 shown in FIG. 11E. The user interface 1140 includes prompt information "Body relaxation and emotional relief help maintain body health, and emotions such as anger and tension may cause risen blood pressure.". The prompt information is used to prompt the user that emotional relief can prevent a blood pressure fluctuation.

The foregoing descriptions are merely some embodiments and implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

It may be understood that each user interface described in embodiments of this application is merely an example interface, and constitutes no limitation on the solutions of this application. In another embodiment, the user interface may use different interface layouts, may include more or fewer controls, and may add or reduce other function options, and provided that the user interface is based on a same inventive idea provided in this application, all fall within the protection scope of this application.

It should be noted that, if no contradiction or conflict occurs, any feature or any part of any feature in any embodiment of this application may be combined, and a combined technical solution also falls within the scope of embodiments of this application.

In conclusion, the foregoing embodiments are merely intended for describing the technical solutions of this application, but not for limiting this application. Although this application is described in detail with reference to the foregoing embodiments, persons of ordinary skill in the art should understand that they may still make modifications to the technical solutions described in the foregoing embodiments or make equivalent replacements to some technical features thereof, without departing from the scope of the technical solutions of embodiments of this application.

## Claims

1. A blood pressure monitoring method, wherein the method is applied to a wearable device, the wearable device comprises a motion sensor and a photoplethysmography PPG module, and the method comprises:
obtaining, by the wearable device, motion data collected by the motion sensor and a first PPG signal collected by the PPG module;
when one or both of the motion data and the first PPG signal meet a first condition, determining, by the wearable device, whether a user is in a sleep state; and
when determining that the user is in a first sleep event, measuring, by the wearable device, user blood pressure, and obtaining a first blood pressure value, wherein
the first sleep event comprises any one or more of the following: an OSA event, a REM event, an NREM sleep event, and an elevated central sympathetic nerve activity event.

2. The method according to claim 1, wherein the OSA event, the REM event, and the elevated central sympathetic nerve activity event are sleep events that cause risen user blood pressure; and when the first sleep event comprises any one or more of the OSA event, the REM event, and the elevated central sympathetic nerve activity event, before obtaining, by the wearable device, the motion data collected by the motion sensor and the first PPG signal collected by the PPG module, the method further comprises:
receiving, by the wearable device, a first user operation, wherein the first user operation indicates the wearable device to measure the user blood pressure when detecting the first sleep event; and
when the user is in the sleep state and it is determined, based on the first PPG signal, that the user is in the first sleep event, measuring, by the wearable device, the user blood pressure, and obtaining the first blood pressure value specifically comprises:
in response to the first operation, when the user is in the sleep state and it is determined, based on the first PPG signal, that the user is in the first sleep event, measuring, by the wearable device, the user blood pressure, and obtaining the first blood pressure value.

3. The method according to claim 1, wherein the NREM sleep event is a sleep event that causes dropped user blood pressure; and when the first sleep event comprises the NREM sleep event, before obtaining, by the wearable device, the motion data collected by the motion sensor and the first PPG signal collected by the PPG module, the method further comprises:
receiving, by the wearable device, a second user operation, wherein the second user operation indicates the wearable device to measure the user blood pressure when detecting the first sleep event; and
when the user is in the sleep state and it is determined, based on the first PPG signal, that the user is in the first sleep event, measuring, by the wearable device, the user blood pressure, and obtaining the first blood pressure value specifically comprises:
in response to the second operation, when the user is in the sleep state and it is determined, based on the first PPG signal, that the user is in the first sleep event, measuring, by the wearable device, the user blood pressure, and obtaining the first blood pressure value.

4. The method according to claim 1 or 2, wherein the OSA event, the REM event, and the elevated central sympathetic nerve activity event are sleep events that cause risen user blood pressure; and when the first sleep event comprises any one or more of the OSA event, the REM event, and the elevated central sympathetic nerve activity event, before obtaining, by the wearable device, the motion data collected by the motion sensor and the PPG signal collected by the PPG module, the method further comprises:
obtaining, by the wearable device, user blood pressure measurement data within first duration; and
determining, by the wearable device based on the user blood pressure measurement data within the first duration, that the user is a hypertensive user; and
when the user is in the sleep state and it is determined, based on the first PPG signal, that the user is in the first sleep event, measuring, by the wearable device, the user blood pressure, and obtaining the first blood pressure value specifically comprises:
in response to determining that the user is a hypertensive user, when detecting the first sleep event, measuring, by the wearable device, the user blood pressure, and obtaining the first blood pressure value.

5. The method according to claim 1 or 3, wherein the NREM sleep event is a sleep event that causes dropped user blood pressure; and when the first sleep event comprises the NREM sleep event, before obtaining, by the wearable device, the motion data collected by the motion sensor and the PPG signal collected by the PPG module, the method further comprises:
obtaining, by the wearable device, user blood pressure measurement data within first duration; and
determining, by the wearable device based on the user blood pressure measurement data within the first duration, that the user is a hypotensive user; and
when the user is in the sleep state and it is determined, based on the first PPG signal, that the user is in the first sleep event, measuring, by the wearable device, the user blood pressure, and obtaining the first blood pressure value specifically comprises:
in response to determining that the user is a hypotensive user, when detecting the first sleep event, measuring, by the wearable device, the user blood pressure, and obtaining the first blood pressure value.

6. The method according to any one of claims 1 to 5, wherein the wearable device further comprises a blood pressure measurement device, the blood pressure measurement device comprises an inflation component, an airbag, and a barometric pressure sensor, the airbag is connected to the inflation component and the barometric pressure sensor, and the PPG module comprises a light source and a PPG sensor;
before obtaining, by the wearable device, the motion data collected by the motion sensor and the PPG signal collected by the PPG module, the method further comprises:
controlling, by the wearable device, the blood pressure measurement device to collect N groups of blood pressure values, and controlling the PPG module to collect N groups of PPG signals corresponding to the N groups of blood pressure values; and
generating, by the wearable device, a first target model based on the N groups of blood pressure values and the N groups of PPG signals, wherein an input into the first target model is a PPG signal, an output of the first target model is a blood pressure value, and N is a positive integer greater than or equal to 2; and
measuring, by the wearable device, the user blood pressure, and obtaining the first blood pressure value specifically comprises:
controlling, by the wearable device, the PPG module to collect a second PPG signal; and
determining, by the wearable device, the first blood pressure value based on the second PPG signal and the first target model.

7. The method according to any one of claims 1 to 5, wherein the wearable device further comprises a blood pressure measurement device, the blood pressure measurement device comprises an inflation component, an airbag, and a barometric pressure sensor, and the airbag is connected to the inflation component and the barometric pressure sensor;
before obtaining, by the wearable device, the motion data collected by the motion sensor and the PPG signal collected by the PPG module, the method further comprises:
collecting, by the wearable device, N groups of airbag pressure and N groups of blood pressure values corresponding to the N groups of airbag pressure, wherein the N groups of airbag pressure comprise airbag pressure corresponding to a moment at which oscillation waves of barometric pressure in N groups of airbags reach a maximum value and airbag pressure corresponding to a moment at which an oscillation wave of barometric pressure in the airbag reaches a × The maximum value; and
generating, by the wearable device, a second target model based on the N groups of airbag pressure and the N groups of blood pressure values corresponding to the N groups of airbag pressure, wherein an input into the second target model is a maximum value of the airbag pressure, an output of the first target model is a blood pressure value, and N is a positive integer greater than or equal to 2; and
measuring, by the wearable device, the user blood pressure, and obtaining the first blood pressure value specifically comprises:
controlling, by the wearable device, the inflation component to input barometric pressure of first airbag pressure into the airbag, wherein the first airbag pressure is less than the airbag pressure corresponding to the moment at which the oscillation wave of the barometric pressure in the airbag reaches a × The maximum value; and
determining, by the wearable device, the first blood pressure value based on the first airbag pressure and the second target model.

8. The method according to any one of claims 1 to 7, wherein before obtaining, by the wearable device, the motion data collected by the motion sensor and the PPG signal collected by the PPG module, the method further comprises:
displaying, by the wearable device, first prompt information when detecting that the wearable device switches from a non-worn state to a worn state, wherein the first prompt information is used to prompt the user to confirm whether the wearable device is worn by a local user; and
receiving, by the wearable device, a first operation performed by the user on a first option in the first prompt information, and in response to the first operation, confirming that the wearable device is worn by the local user; and
after obtaining, by the wearable device, the first blood pressure value, the method further comprises:
storing, by the wearable device, the first blood pressure value in a first storage area, wherein the first storage area stores blood pressure measurement data of the local user.

9. The method according to claim 8, wherein the method further comprises:
receiving, by the wearable device, a second operation performed by the user on a second option in the first prompt information, and in response to the second operation, confirming that the wearable device is worn by a non-local user; and
after obtaining, by the wearable device, the first blood pressure value, the method further comprises:
storing, by the wearable device, the first blood pressure value in a second storage area, wherein the second storage area stores blood pressure measurement data of a non-local user, and the first storage area is different from the second storage area.

10. A wearable device, wherein the wearable device comprises a motion sensor, a PPG module, a memory, and a processor, the motion sensor, the PPG module, the memory, and the processor are coupled, the memory is configured to store a computer program, and when the processor invokes the computer program, the wearable device is enabled to perform the method according to any one of claims 1 to 9.

11. A computer-readable storage medium, comprising instructions, wherein when the instructions are run on a wearable device, the wearable device is enabled to perform the method according to any one of claims 1 to 9.
